# EUROPÄISCHE PATENTSCHRIFT

(11) **EP 0 637 298 B1**
(45) Veröffentlichungstag und Bekanntmachung des Hinweises auf die Patenterteilung: **31.03.1999**
(21) Anmeldenummer: 93909375.3
(22) Anmeldetag: 20.04.1993
(51) Int. Cl.: C07C 233/61, A01N 37/32, C07C 255/46, A01N 37/34, A01N 43/08, A01N 43/78, C07C 233/63, C07D 417/10

(54) **SUBSTITUIERTE CYCLOHEXEN-1,2-DICARBONSÄUREDERIVATE UND ZWISCHENPRODUKTE ZU DEREN HERSTELLUNG**
SUBSTITUTED CYCLOHEXENE-1,2-BICARBOXYLIC ACID DERIVATIVES AND RAW MATERIALS FOR PRODUCING THEM
DERIVES D'ACIDE CYCLOHEXENE-1,2-DICARBOXYLIQUE SUBSTITUES ET PRODUITS INTERMEDIAIRES POUR LEUR FABRICATION

(30) Priorität: 25.04.1992 DE 4213715; 11.11.1992 DE 4238001
(43) Veröffentlichungstag der Anmeldung: 08.02.1995
(60) Teilanmeldung: 97118857.8
(73) Patentinhaber: BASF Aktiengesellschaft, 67063 Ludwigshafen (DE)
(72) Erfinder: KLINTZ, Ralf, D-4630 Bochum (DE); HEISTRACHER, Elisabeth, D-6700 Ludwigshafen (DE); SCHAEFER, Peter, D-6702 Bad Duerkheim (DE); HAMBRECHT, Gerhard, D-6940 Weinheim (DE); PLATH, Peter, D-6710 Frankenthal (DE); KARDORFF, Uwe, D-6800 Mannheim 1 (DE); GERBER, Matthias, D-6703 Limburgerhof (DE); WESTPHALEN, Karl-Otto, D-6720 Speyer (DE); WALTER, Helmut, D-6719 Obrigheim (DE)
(86) Internationale Anmeldenummer: EP9300953
(87) Internationale Veröffentlichungsnummer: WO9322280

(56) Entgegenhaltungen:
- EP-A- 240 659
- EP-A- 320 677
- EP-A- 385 231
- DE-A- 2 921 002
- DE-A- 3 019 758
- FR-A- 2 359 134
- PATENT ABSTRACTS OF JAPAN vol. 8, no. 52 (C-213)9. März 1984 & JP,A,58 210 056 (YAKEDA YAKUHIN KOGYO KK)
- PATENT ABSTRACTS OF JAPAN vol. 8, no. 62 (C-215)23. März 1984 & JP,A,58 216 181 (YAKEDA YAKUHIN KOGYO KK)

## Beschreibung

Substituierte Cyclohexen-1,2-dicarbonsäurederivate der allgemeinen Formeln Ia und Ib 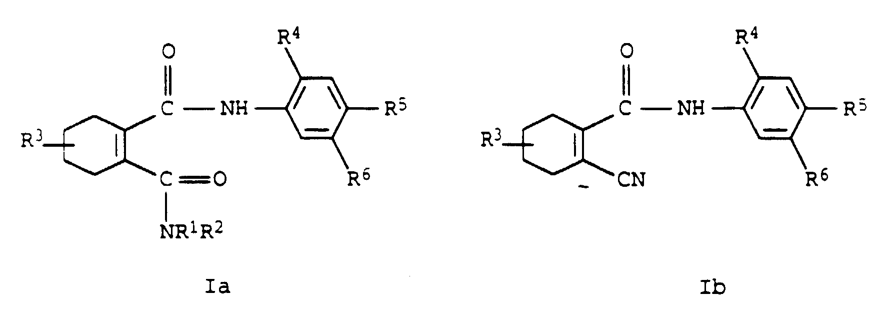 in der die Variablen folgende Bedeutung haben:
R¹, R²
- Wasserstoff,
- eine C₁-C₆-Alkyl-, C₃-C₆-Alkenyl- oder C₃-C₆-Alkinylgruppe, wobei jede dieser drei Gruppen noch einen bis drei Reste tragen kann, ausgewählt aus einer Gruppe bestehend aus Halogen, Cyano, Amino, Thio, Hydroxy, C₁-C₆-Alkoxy, C₁-C₆-Alkylsulfinyl, C₁-C₆-Alkylsulfonyl, C₁-C₆-Alkylcarbonyl, Carboxy, C₁-C₆-Alkoxycarbonyl, C₁-C₆-Alkylamino, C₁-C₆-Dialkylamino, C₁-C₆-Alkylaminocarbonyl, C₁-C₆-Dialkylaminocarbonyl, C₁-C₆-Alkylphosphono, C₁-C₆-Dialkylphosphono, Phenyl, 3- bis 8-gliedriges Heterocyclyl, das gesättigt oder partiell oder vollständig ungesättigt sein kann, wobei die Heterocyclen ein bis vier Heteroatome tragen können, ausgewählt aus einer Gruppe bestehend aus ein bis vier Stickstoffatomen, einem Sauerstoff- und einem Schwefelatom,
   und wobei die Phenyl- und heterocyclischen Reste ihrerseits an jedem substituierbaren Atom einen der folgenden Substituenten tragen können: Hydroxy, Halogen, Cyano, Nitro, Trifluormethyl, C₁-C₆-Alkyl oder C₁-C₆-Alkoxy,

- eine C₃-C₈-Cycloalkylgruppe,
- die Phenylgruppe oder eine gesättigte oder partiell oder vollständig ungesättigte 3- bis 8-gliedrige Heterocyclylgruppe die jeweils ein bis vier Heteroatome tragen kann, ausgewählt aus einer Gruppe bestehend aus ein bis vier Stickstoffatomen, einem oder zwei Sauerstoff- und einem oder zwei Schwefelatomen, und wobei die Phenyl- und heterocyclischen Gruppen ihrerseits an jedem substituierbaren C-Atom einen der folgenden Reste tragen kannen: Hydroxy, Halogen, Cyano, Nitro, Trifluormethyl, Halogen oder C₁-C₆-Alkyl,
und, sofern R¹ für Wasserstoff oder eine C₁-C₆-Alkylgruppe steht, R² zusätzlich
- Hydroxy,
- eine C₁-C₆-Alkoxy-, C₃-C₆-Alkenyloxy- oder C₃-C₆-Alkinyloxygruppe,
- eine C₃-C₇-Cycloalkoxy- oder C₅-C₇-Cycloalkenyloxygruppe,
- eine C₁-C₆-Halogenalkoxy- oder C₃-C₆-Halogenalkenyloxygruppe,
- eine C₃-C₇-Cycloalkyl-C₇-C₆-alkoxygruppe,
- eine C₁-C₆-Alkylcarbonyloxygruppe,
- eine C₁-C₆-Cyanoalkoxygruppe,
- eine Hydroxy-C₁-C₆-alkoxy-, C₁-C₆-Alkoxy-C₁-C₆-alkoxy- oder C₁-C₆-Alkoxycarbonyl-C₁-C₆-alkoxygruppe,
- C₁-C₆-Alkylthio-C₁-C₆-alkoxygruppe,
- eine C₁-C₆-Alkylamino-C₁-C₆-alkoxy- oder C₁-C₆-Dialkylamino-C₁-C₆-alkoxygruppe,
- eine Phenyl-C₁-C₆-alkoxy-, Phenyl-C₃-C₆-alkenyloxy- oder Phenyl-C₃-C₆-alkinyloxygruppe, wobei jeweils eine oder zwei Methylengruppen der Alkoxy-, Alkenyloxy- und Alkinyloxy-Ketten durch Sauerstoff, Schwefel und/oder eine C₁-C₆-Alkylamino-Kette ersetzt sein können, und wobei der Phenylring jeweils unsubstituiert sein oder einen bis drei Reste tragen kann, ausgewählt aus einer Gruppe bestehend aus C₁-C₆-Alkyl, C₂-C₆-Alkenyl, Halogen, Cyano, Nitro, C₁-C₆-Halogenalkyl, C₁-C₆-Alkoxy und C₁-C₆-Alkoxycarbonyl

- eine Gruppe -NR⁷R⁸, wobei R⁷ und R⁸ unabhängig voneinander Wasserstoff, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkylcarbonyl, C₁-C₆-Alkoxycarbonyl, C₁-C₆-Alkoxy-C₁-C₆-alkyl oder Phenyl, das unsubstituiert sein oder ein drei Reste tragen kann, ausgewählt aus einer Gruppe bestehend aus C₁-C₆-Alkyl, Halogen, Cyano, Nitro, C₁-C₆-Alkoxy und C₁-C₆-Alkoxycarbonyl, bedeuten, oder wobei
   R⁷ und R⁸ zusammen mit dem gemeinsamen Stickstoffatom einen gesättigten oder partiell oder vollständig ungesattigten 4- bis 7-gliedrigen Ring bilden, der noch ein weiteres Stickstoffatom oder ein Sauerstoff oder Schwefelatom als zweites Ringglied enthalten kann;
oder
- R¹ und R²: gemeinsam mit dem Stickstoff-Atom, an das sie gebunden sind,
einen drei- bis achtgliedrigen gesättigten oder ungesättigten, nicht aromatischen Heterocyclus, der ein bis drei Stickstoffatome und/oder ein Sauerstoff- oder Schwefelatom sowie ein oder zwei Carbonylgruppen tragen kann,
und wobei der Heterocyclus unsubstituiert sein oder ein bis drei C₁-C₆-Alkylreste tragen kann;
- R³: Wasserstoff oder eine C₁-C₆-Alkylgruppe;
- R⁴: Wasserstoff oder Halogen;
- R⁵: Wasserstoff, Halogen, Nitro, Cyano oder Trifluormethyl;
- R⁶: eine Gruppe -A-CO-B, wobei
A für
   eine C₂-Alkenylenkette, die unsubstituiert sein oder ein oder zwei Reste tragen kann, ausgewählt aus der Gruppe bestehend aus Halogen, Cyano, C₁-C₆-Alkyl, C₁-C₆-Halogenalkyl, Hydroxy, C₁-C₆-Alkoxy,
   C₁-C₆- Alkylcarbonyloxy, C₁-C₆-Alkoxycarbonyl und C₁-C₆- Alkylcarbonyl;
B
   - Wasserstoff, C₁-C₆-Alkyl, C₃-C₆-Alkenyl, C₃-C₆-Alkinyl, C₁-C₆-Halogenalkyl, C₃-C₇-Cycloalkyl,
   - OR¹⁷ oder -SR¹⁷, wobei R¹⁷
      - Wasserstoff, C₁-C₆-Alkyl, C₃-C₆-Alkenyl, C₃-C₆-Alkinyl, C₃-C₆-Cycloalkyl, C₁-C₆-Halogenalkyl,
      - Phenyl, das unsubstituiert sein oder ein bis drei Reste tragen kann, ausgewählt aus einer Gruppe bestehend aus C₁-C₆-Alkyl, C₂-C₆-Alkenyl, Halogen, Cyano, Nitro, C₁-C₆-Halogenalkyl, C₁-C₆-Alkoxy und C₁-C₆-Alkoxycarbonyl,
      - C₁-C₆-Cyanoalkyl, C₃-C₆-Halogenalkenyl, C₁-C₆-Alkoxycarbonyl, C₁-C₆-Alkoxy- C₁-C₆-alkyl, C₁-C₆-Alkoxy-carbonyl-C₁-C₆-alkyl oder C₁-C₆-Alkyloximino-C₁-C₆-alkyl,
      bedeutet;
   - Phenyl, das unsubstituiert sein oder ein bis drei Reste tragen kann, ausgewählt aus einer Gruppe bestehend aus C₁-C₆-Alkyl, C₂-C₆-Alkenyl, Halogen, Cyano, Nitro, C₁-C₆-Halogenalkyl, C₁-C₆-Alkoxy und C₁-C₆-Alkoxycarbonyl,
   - C₁-C₆-Alkoxy-C₁-C₆-alkyl, Di-(C₁-C₆-alkoxy) -C₁-C₆-alkyl C₁-C₆-Alkylthio-C₁-C₆- alkyl, oder
   - -NR¹⁸R¹⁹, wobei R¹⁸ und R¹⁹ unabhängig voneinander Wasserstoff, C₁-C₆-Alkyl, C₃-C₆-Alkenyl, C₃-C₆-Alkinyl, C₃-C₆-Cycloalkyl, C₁-C₆-Halogenalyl, C₁-C₆-Alkylcarbonyl, C₁-C₆-Alkoxycarbonyl, C₁-C₆-Alkoxy- C₁-C₆-alkyl oder Phenyl, das unsubstituiert sein oder ein drei Reste tragen kann, ausgewählt aus einer Gruppe bestehend aus C₁-C₆-Alkyl, C₃-C₆-Alkenyl, Halogen, Cyano, Nitro, C₁-C₆-Alkoxy und C₁-C₆-Alkoxycarbonyl, bedeuten, oder wobei
      R¹⁸ und R¹⁹ zusammen mit dem gemeinsamen Stickstoffatom einen gesättigten oder partiell oder vollständig ungesättigten 4- bis 7-gliedrigen Ring bilden, der noch ein weiteres Stickstoffatom oder ein Sauerstoff oder Schwefelatom als zweites Ringglied enthalten kann;
sowie die landwirtschaftlich brauchbaren Salze der Verbindungen Ia und Ib.

Außerdem betrifft die Erfindung neue Zwischenprodukte der Formeln IIIa und VIIIa.

Des weiteren betrifft die Erfindung herbizide und das Wachstum der Pflanzen regulierende Mittel, welche diese Verbindungen als wirksame Substanzen enthalten.

Aus der EP-A 097 056 sind herbizid wirksame Cyclohexen-1,2-dicarbonsäurederivate Ia' bekannt 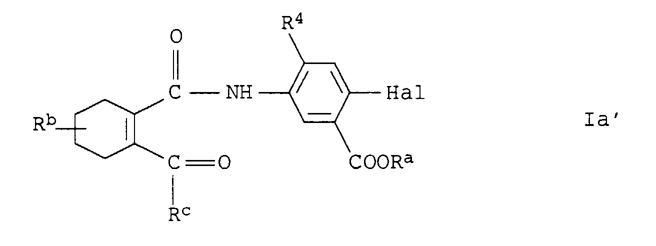 wobei R^{a} Wasserstoff oder durch Halogen oder C₁-C₄-Alkoxy substituiertes C₁-C₈-Alkyl, Hal Halogen, R^{b} Wasserstoff oder C₁-C₄-Alkyl und R^{c} einen Aminrest bedeuten.

Ferner werden in der JO 60/252 457 Cyclohexendicarbonsäurederivate der Formel Ia" 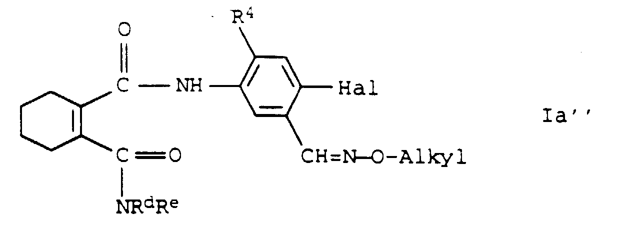 sowie deren Verwendung als Herbizide beschrieben, wobei R^{d} und R^{e} Wasserstoff, Alkyl, Alkenyl, Alkinyl, Cycloalkyl oder zusammen mit dem Stickstoffatom einen heterocyclischen Ring bedeuten.

Außerdem sind der WO-A 87/107 602 u.a. Verbindungen der Formel Ia"' 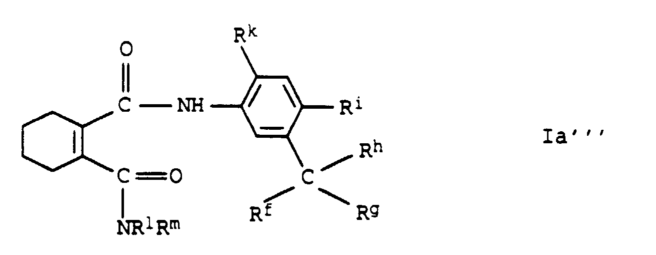 zu entnehmen, wobei R^{f} und R^{g} Alkyl, Alkenyl, Alkinyl oder Halogen und R^{h} u.a. eine Cyano-, subst. Alkylcarbonyl-, Carbonyl- oder Alkoxycarbonyl-alkylgruppe bedeuten.

Des weiteren offenbart die JO 59/051 250 herbizid wirksame N-Phenyltetrahydrophthalamid-Derivate der Formel Ia"" 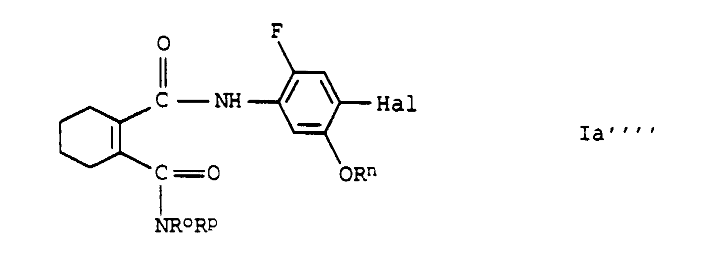 wobei Rⁿ für Alkyl, Alkenyl, Alkinyl, Carbamoylmethyl oder Alkoxycarbonylmethyl und R^{o} und R^{p} für Wasserstoff, Alkyl, Alkenyl, Phenyl oder zusammen mit dem Stickstoffatom für einen heterocyclischen Ring stehen.

Aus der U.S. Patentschrift 4,613,675 sind weitere Verbindungen vom Typ der Formel Ia"" bekannt, die anstelle des Fluoratoms einen Rest R⁴ und anstelle von Rⁿ einen Rest -CHR^{q}P(=O)(OR^{r})R^{s} tragen, wobei R^{q} Wasserstoff oder Alkyl, R^{r} Alkyl, Alkoxyalkyl oder Alkoxycarbonyl und R^{s} Wasserstoff, Alkyl, Cycloalkyl oder Alkoxyalkyl bedeuten.

Aus JO 55/157 545, DE-A 30 19 758 und JO 55/154 949 sind ebenfalls Verbindungen vom Typ der Formel Ia"" bekannt, die anstelle des Fluoratoms einen Rest R⁴ und anstelle von Rⁿ u.a. Alkyl oder Alkenyl tragen.

Weitere Cyclohexen-1,2-dicarbonsäurederivate vom Typ der Verbindungen Ia sind beispielsweise den folgenden Druckschriften zu entnehmen:
DE-A 28 51 379, DE-A 29 21 002, JO 48/096 722, JO 55/157 547, JO 55/157 552, JO 55 162 756, JO 58/188 848, JO 58/210 056, JO 58/216 181 und JO 61/043 160.

In JO 58/210 056 werden Cyclohexen-1,2-dicarbonsäurederivate der allgemeinen Formel Ib' 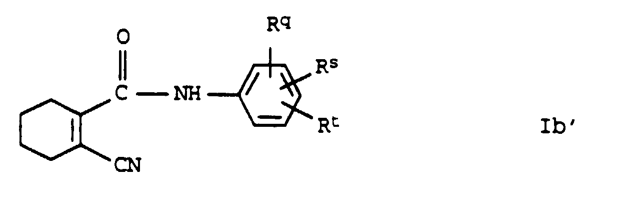 offenbart, in der R^{q}, R^{s}, R^{t} für H, Halogen, Cyano, Alkoxy, Alkenyloxy, ggf. subst. Aralkoxy, ggf. verestertes Carboxy und Haloalkyl stehen.

Die Selektivität dieser bekannten Herbizide bezüglich der Schadpflanzen vermag jedoch nur bedingt zu befriedigen, so daß der Erfindung neue herbizid wirksame Verbindungen als Aufgabe zugrunde lagen, mit denen sich (bei guter Verträglichkeit für die Nutzpflanzen) die Schadpflanzen besser als bisher gezielt bekämpfen lassen.

Demgemäß wurden die eingangs definierten substituierten Cyclohexen-1,2-dicarbonsäurederivate Ia und Ib gefunden.

Ferner wurden herbizide Mittel gefunden, die diese Substanzen enthalten und eine gute herbizide Wirkung besitzen.

Außerdem wurde gefunden, daß sich die erfindungsgemäßen Verbindungen Ia und Ib als Defoliations- und Desikkationsmittel eignen, z. B. in den Kulturen Baumwolle, Kartoffel, Raps, Sonnenblume, Sojabohne oder Ackerbohnen.

Die vorstehend für die Substituenten R¹ bis R⁸ und R¹⁷ bis R¹⁹ genannten Bedeutungen stellen Sammelbegriffe für individuelle Aufzählungen der einzelnen Gruppenmitglieder dar. Sämtliche Alkyl-, Alkenyl-, Alkinyl-, Halogenalkyl- und Halogenalkoxyteile können geradkettig oder verzweigt sein, sofern nichts anderes angegeben ist. Die Halogenalkyl- und Halogenalkoxyreste können gleiche oder verschiedene Halogenatome tragen.

Im einzelnen bedeuten beispielsweise:
- Halogen: Fluor, Chlor, Brom und Jod, vorzugsweise Fluor und Chlor;
- C₁-C₆-Alkyl und die C₁-C₆-Alkylteile in den Resten Di-(C₁-C₆-alkoxy)-C₁-C₆-alkyl, C₃-C₆-Alkenyloxy-C₁-C₆-alkyl, C₃-C₆-Alkinyloxy-C₁-C₆-alkyl, C₁-C₆-alkoxy-C₁-C₆-alkoxycarbonyl-C₁-C₆-alkyl und Heterocyclyl-C₁-C₆-alkyl: Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, Isopropyl und tert.-Butyl;
- C₂-C₆-Alkenyl: Ethenyl, und C₃-C₆-Alkenyl wie Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1;1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethylprop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl, vorzugsweise Ethenyl und Prop-2-en-1-yl;
- C₂-C₆-Alkinyl: Ethinyl und C₃-C₆-Alkinyl wie Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pentin-5-yl, Pent-2-in-1-yl, Pent-2-in-4-yl, Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in 6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise Prop-2-inyl;
- C₃-C₈-Cycloalkyl: Cyclopropyl, Cyclobutyl, Cyclopentyl, Cyclohexyl, Cycloheptyl und Cyclooctyl, vorzugsweise C₃-C₆-Cycloalkyl wie Cyclopropyl, Cylopentyl und Cyclohexyl;
- C₁-C₆-Halogenalkyl: Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor- 2,2-difluorethyl, 2,2-Dichlor-2-fluorethyl, 2,2,2-Trichlorethyl, Pentafluorethyl und 3-Chlorpropyl, vorzugsweise Trifluormethyl,
- C₁-C₆-Hydroxyalkyl: Hydroxymethyl, 1-Hydroxyeth-1-yl, 2-Hydroxyeth-1-yl, 1-Hydroxy-prop-1-yl, 2-Hydroxy-prop-1-yl, 3-Hydroxy-prop-1-yl, 1-Hydroxy-prop-2-yl, 2-Hydroxy-prop-2-yl, 1-Hydroxy-but-1-yl, 2-Hydroxy-but-1-yl, 3-Hydroxy-but-1-yl, 4-Hydroxy-but-1-yl, 1-Hydroxy-but-2-yl, 2-Hydroxy-but-2-yl, 1-Hydroxy-but-3-yl, 2-Hydroxy-but-3-yl, 1-Hydroxy-2-methyl-prop-3-yl, 2-Hydroxy-2-methyl-prop-3 -yl, 3-Hydroxy-2-methyl-prop-3-yl und 2-Hydroxymethyl-prop-2-yl, vorzugsweise Hydroxymethyl;
- C₁-C₆-Thiolalkyl: Thiolmethyl, 1-Thioleth-1-yl, 2-Thioleth-1-yl, 1-Thiol-prop-1-yl, 2-Thiol-prop-1-yl, 3-Thiol-prop-1-yl, 1-Thiol-prop-2-yl, 2-Thiol-prop-2-yl, 1-Thiol-but-1-yl, 2-Thiol-but-1-yl, 3-Thiol-but-1-yl, 4-Thiol-but-1-yl, 1-Thiol-but-2-yl, 2-Thiol-but-2-yl, 1-Thiol-but-3-yl, 2-Thiol-but-3-yl, 1-Thiol-2-methyl-prop-3-yl, 2-Thiol-2-methyl-prop-3-yl, 3-Thiol-2-methyl-prop-3-yl und 2-Thiolmethyl-prop-2-yl, vorzugsweise Thiolmethyl;
- C₁-C₆-Cyanoalkyl: Cyanomethyl, 1-Cyanoeth-1-yl, 2-Cyanoeth-1-yl, 1-Cyano-prop-1-yl, 2-Cyano-prop-1-yl, 3-Cyano-prop-1-yl, 1-Cyano-prop-2-yl, 2-Cyano-prop-2-yl, 1-Cyano-but-1-yl, 2-Cyano-but-1-yl, 3-Cyano-but-1-yl, 4-Cyano-but-1-yl, 1-Cyano-but-2-yl, 2-Cyano-but-2-yl, 1-Cyano-but-3-yl, 2-Cyano-but-3-yl, 1-Cyano-2-methyl-prop-3-yl, 2-Cyano-2-methyl-prop-3-yl, 3-Cyano-2-methyl-prop-3-yl, und 2-Cyanomethyl-prop-2-yl, vorzugsweise Cyanomethyl, 1-Cyano-eth-1-yl;
- C₁-C₆-Cyanoalkoxy: Cyanomethoxy, 1-Cyanoeth-1-oxy, 2-Cyanoeth-1-oxy, 1-Cyano-prop-1-oxy, 2-Cyano-prop-1-oxy, 3-Cyano-prop-1-oxy, 1-Cyano-prop-2-oxy, 2-Cyano-prop-2-oxy, 1-Cyano-but-1-oxy, 2-Cyano-but-1-oxy, 3-Cyano-but-1-oxy, 4-Cyano-but-1-oxy, 1-Cyano-but-2-oxy, 2-Cyano-but-2-oxy, 1-Cyano-but-3-oxy, 2-Cyano-but-3-oxy, 1-Cyano-2-methyl-prop-3-oxy, 2-Cyano-2-methyl-prop-3-oxy, 3-Cyano-2-methyl-prop-3-oxy, und 2-Cyanomethyl-prop-2-oxy, vorzugsweise Cyanomethoxy, 1-Cyano-eth-1-oxy;
- Phenyl-C₁-C₆-alkyl: Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 3-Phenylbut-2-yl, 4-Phenylbut-2-yl, 1- (Phenylmethyl)-eth-1-yl, 1-(Phenylmethyl)-1-(methyl)-eth-1-yl, und 1-(Phenylmethyl)-prop- 1-yl, vorzugsweise Benzyl;
- C₁-C₆-Alkoxy und die C₁-C₆-Alkoxyteile in den Resten C₃-C₇-Cycloalkyl-C₁-C₆-alkoxy, Di-(C₁-C₆-alkoxy)-C₁-C₆- alkyl, C₁-C₆-Alkoxycarbonyl-C₁-C₆-alkoxy und C₁-C₆-alkoxy-C₁-C₆-alkoky-carbonyl-C₁-C₆-alkyl: Methoxy, Ethoxy, n-Propoxy, 1-Methylethoxy, n-Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, l-Ethyl-l-methylpropoxy und l-Ethyl-2-methylpropoxy, vorzugsweise C₁-C₄-Alkoxy wie Methoxy und Ethoxy;
- C₁-C₆-Halogenalkoxy: Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise C1-C2-Halogenalkoxy wie Trifluormethoxy;
- C₁-C₆-Alkylthio: Methylthio, Ethylthio, n-Propylthio, 1-Methylethylthio, n-Butylthio, 1-Methyl-propylthio, 2-Methylpropylthio, 1,1-Dimethylethylthio, n-Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, n-Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und l-Ethyl-2-methylpropylthio, vorzugsweise C1-C4-Alkylthio wie Methylthio und Ethylthio;
- Phenyl-C₁-C₆-alkoxy: Benzyloxy, 1-Phenylethoxy, 2-Phenylethoxy, 1-Phenylprop-1-oxy, 2-Phenylprop-1-oxy, 3-Phenylprop-1-oxy, 1-Phenylbut-1-oxy, 2-Phenylbut-1-oxy, 3-Phenylbut-1-oxy, 4-Phenylbut-1-oxy, 1-Phenylbut-2-oxy, 2-Phenyl-but-2-oxy, 3-Phenylbut-2-oxy, 3-Phenylbut-2-oxy, 4-Phenyl-but-2-oxy, 1-(Phenylmethyl)-eth-1-oxy, 1-(Phenoxymethyl)-1-(methyl)-eth-1-oxy, und 1-(Phenylmethyl)-prop-1-oxy, vorzugsweise Benzyloxy;
- C₃-C₆-Alkenyloxy und der C₃-C₆-Alkenyloxyteil in dem Rest C₃-C₆-Alkenyloxy-C₁-C₆-alkyl: Prop-2-en-1-yloxy, n-Buten-4-yl-oxy, n-Buten-3-yloxy, 1-Methyl-prop-2-en-1-yloxy, 2-Methyl-prop-2-en-1-yloxy, n-Penten-3-yloxy, n-Penten-4-yloxy, n-Penten-5-yloxy, 1-Methyl-but-2-en-1- yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Di-methyl-prop-2-en-1-yloxy, 1-Ethyl-prop-2-en-1-yloxy, n-Hex-2-en-1-yloxy, n-Hex-3-en-1-yloxy, n-Hex-4-en-1-yloxy, n-Hex-5-en-1-yloxy, 1-Methyl-pent-2-en-1-yloxy, 2-Methyl-pent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methyl-pent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-2-en-1-yloxy, 1,2-Dimethyl-but-3-en-1- yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-2-en-1-yloxy, 2,3-Dimethyl-but-3-en-1-yloxy, 3,3-Dimethyl-but-2-en-1-yloxy, 1-Ethyl-but-2-en-1-yloxy, 1-Ethyl-but-3-en-1-yloxy, 2-Ethyl-but-2-en-1-yloxy, 2-Ethyl-but-3-en-1-yloxy, 1,1,2-Trimethyl-prop-2-en- 1-yloxy, 1-Ethyl-1-methyl-prop-2-en-1-yloxy und 1-Ethyl-2-methyl-prop-2-en-1-yloxy, vorzugsweise Prop-2-en-1-yloxy;
- C₃-C₆-Alkenylthio: Prop-2-en-1-ylthio, n-Buten-4-ylthio, n-Buten-3-ylthio, 1-Methyl-prop-2-en-1-ylthio, 2-Methyl-prop-2-en-1-ylthio, n-Penten-3-ylthio, n-Penten-4-ylthio, n-Penten-5-ylthio, 1-Methyl-but-2-en-1-ylthio, 2-Methyl-but-2-en-1-ylthio, 3-Methyl-but-2-en-1-ylthio, 1-Methyl-but-3-en-1-ylthio, 2-Methyl-but-3-en-1-ylthio, 3-Methyl-but-3-en-1-ylthio, 1,1-Dimethyl-prop-2-en-1-ylthio, 1,2-Dimethyl-prop-2-en-1-ylthio, 1-Ethyl-prop-2- en-1-ylthio, n-Hex-2-en-1-ylthio, n-Hex-3-en-1-ylthio, n-Hex-4-en-1-ylthio, n-Hex-5-en-1-ylthio, 1-Methyl-pent-2-en-1-ylthio, 2-Methyl-pent-2-en-1-ylthio, 3-Methyl-pent-2-en-1-ylthio, 4-Methyl-pent-2-en-1-ylthio, 1-Methyl-pent-3-en-1-ylthio, 2-Methyl-pent-3-en-1-ylthio, 3-Methyl-pent-3-en-1-ylthio, 4-Methyl-pent-3-en-1-ylthio, 1-Methyl-pent-4-en-1-ylthio, 2-Methyl-pent-4-en- 1-ylthio, 3-Methyl-pent-4-en-1-ylthio, 4-Methyl-pent-4-en-1-ylthio, 1,1-Dimethyl-but-2-en-1-ylthio, 1,1-Dimethyl-but-3-en-1-ylthio, 1,2-Dimethyl-but-2-en-1-ylthio, 1,2-Dimethyl-but-3-en-1-ylthio, 1,3-Dimethyl-but-2-en-1-ylthio, 1,3-Dimethyl-but-3-en-1-ylthio, 2,2-Dimethyl-but-3-en-1-ylthio, 2,3-Dimethyl-but-2-en-1-ylthio, 2,3-Dimethyl-but-3-en-1-ylthio, 3,3-Dimethyl-but-2-en- 1-ylthio, 1-Ethyl-but-2-en-1-ylthio, 1-Ethyl-but-3-en-1- ylthio, 2-Ethyl-but-2-en-1-ylthio, 2-Ethyl-but-3-en-1- ylthio, 1,1,2-Trimethylprop-2-en-1-ylthio, 1-Ethyl-1-methyl-prop-2-en-1-ylthio und 1-Ethyl-2-methyl-prop-2-en- 1-ylthio, vorzugsweise Prop-2-en-1-ylthio;
- C₃-C₆-Alkinyloxy und der C₃-C₆-Alkinyloxyteil in dem Rest C₃-C₆-Alkinyloxy-C₁-C₆-alkyl: Prop-2-in-3-yloxy, n-But-1-in-4-yloxy, n-But-2-in-1-yloxy, n-Pent-1-in-3-yloxy, n-Pent-1-in-4-yloxy, n-Pent-1-in-5-yloxy, Pent-2- in-1-yloxy, Pent-2-in-4-yloxy, Pent-2-in-5-yloxy, 3-Methyl-but-1-in-3-yloxy, 3-Methyl-but-1-in-4-yloxy, n-Hex-1-in-3-yloxy, n-Hex-1-in-4-yloxy, n-Hex-1-in-5-yloxy, n-Hex-1-in 6-yloxy, n-Hex-2-in-1-yloxy, n-Hex-2-in-4- yloxy, n-Hex-2-in-5-yloxy, n-Hex-2-in-6-yloxy, n-Hex-3- in-1-yloxy, n-Hex-3-in-2-yloxy, 3-Methyl-pent-1-in-3- yloxy, 3-Methyl-pent-1-in-4-yloxy, 3-Methylpent-1-in- 5-yloxy, 4-Methyl-pent-2-in-4-yloxy und 4-Methyl-pent-2-in- 5-yloxy, vorzugsweise Prop-2-inyloxy;
- C₃-C₆-Alkinylthio: Prop-2-in-3-ylthio, n-But-1-in-4-ylthio, n-But-2-in-1-ylthio, n-Pent-1-in-3-ylthio, n-Pent-1-in-4-ylthio, n-Pent-1-in-5-ylthio, Pent-2-in-1-ylthio, Pent-2-in-4-ylthio, Pent-2-in-5-ylthio, 3-Methyl-but-1-in-3-ylthio, 3-Methyl-but-1-in-4-ylthio, n-Hex-1-in-3- ylthio, n-Hex-1-in-4-ylthio, n-Hex-1-in-5-ylthio, n-HeX-1-in-6-ylthio, n-Hex-2-in-1-ylthio, n-Hex-2-in-4- ylthio, n-Hex-2-in-5-ylthio, n-Hex-2-in-6-ylthio, n-Hex- 3-in-1-ylthio, n-Hex-3-in-2-ylthio, 3-Methyl-pent-1-in- 3-ylthio, 3-Methyl-pent-1-in-4-ylthio, 3-Methyl-pent-1- in-5-ylthio, 4-Methyl-pent-2-in-4-ylthio und 4-Methyl-pent-2-in-5-ylthio, vorzugsweise Prop-2-inylthio;
- C₃-C₆-Halogenalkenyloxy: 2-Chlorprop-2-enyloxy, 3-Chlor-prop-2-enyloxy, 2,3-Dichlorprop-2-enyloxy, 3,3-Dichlor-prop-2-enyloxy, 2,3,3-Trichlorprop-2-enyloxy, 2,3-Dichlor-but-2-enyloxy, 2-Bromprop-2-enyloxy, 3-Bromprop-2-enyloxy, 2,3-Dibromprop-2-enyloxy, 3,3-Dibromprop-2-enyloxy, 2,3,3-Tribromprop-2-enyloxy und 2,3-Dibrombut-2-enyloxy;
- Phenyl-C₃-C₆-alkenyloxy: 2-Phenylprop-2-enyloxy, 3-Phenyl-prop-2-enyloxy und 4-Phenylbut-2-en-1-yloxy;
- Phenyl-C₃-C₆-alkinyloxy: 2-Phenylprop-2-inyloxy, 3-Phenyl-prop-2-inyloxy und 4-Phenylbut-2-in-1-yloxy;
- C₃-C₇-Cycloalkoxy: Cyclopropyloxy, Cyclobutyloxy, Cyclopentyloxy, Cyclohexyloxy und Cycloheptyloxy;
- C₅-C₇-Cycloalkenyloxy wie Cyclopent-1-enyloxy, Cyclo-pent-2-enyloxy, Cyclopent-3-enyloxy, Cyclohex-1-enyloxy, Cyclohex-2-enyloxy, Cyclohex-3-enyloxy, Cyclohept-1- enyloxy, Cyclohept-2-enyloxy, Cyclohept-3-enyloxy und Cyclohept-4-enyloxy;
- C₁-C₄-Alkylamino: Methylamino, Ethylamino, n-Propylamino, 1-Methylethylamino, n-Butylamino, 1-Methylpropylamino, 2-Methylpropylamino und 1,1-Dimethylethylamino;
- C₁-C₆-Alkylamino: Methylamino, Ethylamino, n-Propylamino, 1-Methylethylamino, n-Butylamino, 1-Methylpropylamino, 2-Methylpropylamino, 1,1-Dimethylethylamino, n-Pentylamino, 1-Methylbutylamino, 2-Methylbutylamino, 3-Methylbutylamino, 2,2-Dimethylpropylamino, 1-Ethylpropylamino, n-Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino, vorzugsweise C₁-C₄-Alkylamino wie Methylamino und Ethylamino;
- Di-(C₁-C₆)-alkylamino: N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl- N-(2-methylpropyl)amino, N-(1,1-Dimethylethyl)--N-methylamino, N-Ethyl-N-propylamino, N-Ethyl-N-(1-methyl-ethyl)amino, N-Butyl-N-ethylamino, N-Ethyl-N-(1-methyl-propyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl) -N-propylamino, N-Butyl-N-(1-methyl-ethyl)-amino, N-(1-Methylethyl)-N-(1-methylpropyl)amino, N-(1-Methylethyl)-N-(2-methylpropyl)amino, N-(1,1-Di-methylethyl)-N-(1-methylethyl)amino, N-Butyl-N-(1-methyl-propyl)amino, N-Butyl-N-(2-methylpropyl)amino, N-Butyl-N-(1,1-dimethylethyl)amino, N-(1-Methylpropyl)- N-(2-methyl-propyl)amino, N-(1,1-Dimethylethyl)-N-(1-methylpropyl)amino und N-(1,1-Dimethylethyl)-N-(2-methylpropyl)amino, vorzugsweise Dimethylamino und Diethylamino;
- C₁-C₆-Alkylphosphono : Methylphosphono, Ethylphosphono, n-Propylphosphono, 1-Methylethylphosphono, n-Butylphosphono, 1-Methylpropylphosphono, 2-Methylpropylphosphono, 1,1-Dimethylethylphosphono, n-Pentylphosphono, 1-Methylbutylphosphono, 2-Methylbutylphosphono, 3-Methylbutylphosphono, 2,2-Dimethylpropylphosphono, 1-Ethylpropylphosphono, n-Hexylphosphono, 1,1-Dimethylpropylphosphono, 1,2-Dimethylpropylphosphono, 1-Methylpentylphosphono, 2-Methylpentylphosphono, 3-Methylpentylphosphono, 4-Methylpentylphosphono, 1,1-Dimethylbutylphosphono, 1,2-Dimethylbutylphosphono, 1,3-Dimethylbutylphosphono, 2,2-Dimethylbutylphosphono, 2,3-Dimethylbutylphosphono, 3,3-Dimethylbutylphosphono, 1-Ethylbutylphosphono, 2-Ethylbutylphosphono, 1,1,2-Trimethylpropylphosphono, 1,2,2-Trimethylpropylphosphono, 1-Ethyl-1-methylpropylphosphono und 1-Ethyl-2-methylpropylphosphono, vorzugsweise C₁-C₄-Alkylphosphono wie Methylphosphono und Ethylphosphono;
- Di-(C₁-C₆)-alkylphosphono: N,N-Dimethylphosphono, N,N-Diethylphosphono, N,N-Dipropylphosphono, N,N-Di- (1-methylethyl)phosphono, N,N-Dibutylphosphono, N,N-Di- (1-methylpropyl)phosphono, N,N-Di-(2-methylpropyl)phosphono, N,N-Di-(1,1-dimethylethyl)phosphono, N-Ethyl-N- methylphosphono, N-Methyl-N-propylphosphono, N-Methyl- N-(1-methylethyl)phosphono, N-Butyl-N-methylphosphono, N-Methyl-N-(1-methylpropyl)phosphono, N-Methyl-N-(2-methylpropyl)phosphono, N-(1,1-Dimethylethyl)-N-methylphosphono, N-Ethyl-N-propylphosphono, N-Ethyl-N-(1-methylethyl)phosphono, N-Butyl-N-ethylphosphono, N-Ethyl- N-(1-methylpropyl)phosphono, N-Ethyl-N-(2-methylpropyl)phosphono, N-Ethyl-N-(1,1-dimethylethyl)phosphono, N-(1-Methylethyl)-N-propylphosphono, N-Butyl-N-propylphosphono, N-(1-Methylpropyl)-N-propylphosphono, N-(2-Methylpropyl)-N-propylphosphono, N-(1,1-Dimethylethyl)-N-propylphosphono, N-Butyl-N-(1-methylethyl)-phosphono, N-(1-Methylethyl)-N-(1-methylpropyl)phosphono, N-(1-Methylethyl)-N-(2-methylpropyl)phosphono, N-(1,1-Dimethylethyl)-N-(1-methylethyl)phosphono, N-Butyl-N-(1-methyl-propyl)phosphono, N-Butyl-N-(2-methylpropyl)phosphono, N-Butyl-N-(1,1-dimethylethyl)phosphono, N-(1-Methylpropyl)-N-(2-methyl-propyl)phosphono, N-(1,1-Dimethylethyl)-N-(1-methylpropyl)phosphono und N-(1,1-Dimethylethyl)-N-(2-methylpropyl)phosphono, vorzugsweise Dimethylphosphono und Diethylphosphono;
- C₁-C₆-Alkylsulfonyl: Methylsulfonyl, Ethylsulfonyl, n-Propylsulfonyl, 1-Methylethyl-sulfonyl, n-Butylsulfonyl, 1-Methylpropylsulfonyl, 2-Methylpropylsulfonyl, 1,1-Dimethylethylsulfonyl, n-Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, n-Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl, vorzugsweise C₁-C₄-Alkylsulfonyl wie Methylsulfonyl und Ethylsulfonyl;
- C₁-C₆-Alkoxy-C₁-C₆-alkyl: Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, n-Pentoxymethyl, (1-Methylbutoxy)methyl, (2-Methylbutoxy)methyl, (3-Methylbutoxy)-methyl, (2,2-Dimethylpropoxy)methyl, (1-Ethylpropoxy)methyl, n-Hexoxymethyl, (1,1-Dimethylpropoxy)methyl, (1,2-Dimethylpropoxy)methyl, (1-Methylpentoxy)methyl, (2-Methylpentoxy)methyl, (3-Methylpentoxy)methyl, (4-Methylpentoxy)methyl, (1,1-Dimethylbutoxy)methyl, (1,2-Dimethylbutoxy)methyl, (1,3-Dimethylbutoxy)methyl, (2,2-Dimethylbutoxy)methyl, (2,3-Dimethylbutoxy)methyl, (3,3-Dimethylbutoxy)methyl, (1-Ethylbutoxy) methyl, (2-Ethylbutoxy)methyl, (1,1,2-Trimethylpropoxy) methyl, (1,2,2-Trimethylpropoxy)methyl, (1-Ethyl-1-methylpropoxy)methyl, (1-Ethyl-2-methylpropoxy)methyl, Methoxyethyl, Ethoxyethyl, n-Propoxyethyl, (1-Methylethoxy)-ethyl, n-Butoxyethyl, (1-Methylpropoxylethyl, (2-Methylpropoxy)ethyl, (1,1-Dimethylethoxy)ethyl, n-Pentoxyethyl, (1-Methylbutoxy)ethyl, (2-Methylbutoxy)ethyl, (3-Methylbutoxy)ethyl, (2,2-Dimethylpropoxy)ethyl, (1-Ethylpropoxy)ethyl, n-Hexoxyethyl, (1,1-Dimethylpropoxy)ethyl, (1,2-Dimethylpropoxy)ethyl, (1-Methylpent- oxy)ethyl, (2-Methylpentoxy)ethyl, (3-Methylpentoxy) ethyl, (4-Methylpentoxy)ethyl, (1,1-Dimethylbutoxy)-ethyl, (1,2-Dimethylbutoxy)ethyl, (1,3-Dimethylbutoxy)-ethyl, (2,2-Dimethylbutoxy)ethyl, (2,3-Dimethylbutoxy)-ethyl, (3,3-Dimethylbutoxy)ethyl, (1-Ethylbutoxy)ethyl, (2-Ethylbutoxy)ethyl, (1,1,2-Trimethylpropoxy)ethyl, (1,2,2-Trimethylpropoxy)ethyl, (1-Ethyl-1-methylpropoxy)ethyl, (1-Ethyl-2-methylpropoxy)ethyl, 2-(Methoxy)propyl, 3-(Methoxy)propyl und 2-(Ethoxy)propyl, vorzugsweise C₁-C₆-Alkoxy-C₁-C₂-alkyl wie Methoxymethyl, Ethoxymethyl, 2-Methoxyethyl und 2-Ethoxyethyl;
- C₁-C₆-Alkoximino-C₁-C₆-alkyl: Methoximino-methyl, Ethoximinomethyl, n-Propoximino-methyl, (1-Methylethoximino)methyl, n-Butoximino-methyl, (1-Methylpropoximino)methyl, (2-Methylpropoximino)methyl, (1,1-Dimethylethoximino)methyl, n-Pentoximino-methyl, (1-Methylbutoximino)methyl, (2-Methylbutoximino)methyl, (3-Methylbutoximino)methyl, (2,2-Dimethylpropoximino)methyl, (1-Ethylpropoximino)methyl, n-Hexoximinomethyl, (1,1-Dimethylpropoximino)methyl, (1,2-Dimethylpropoximino)methyl, (1-Methylpentoximino)methyl, (2-Methylpentoximino)methyl, (3-Methylpentoximino)methyl, (4-Methylpentoximino)methyl, (1,1-Dimethylbutoximino)methyl, (1,2-Dimethylbutoximino)methyl, (1,3-Dimethylbutoximino)methyl, (2,2-Dimethylbutoximino)methyl, (2,3-Dimethylbutoximino)methyl, (3,3-Dimethylbutoximino)methyl, (1-Ethylbutoximino)methyl, (2-Ethylbutoximino)methyl, (1,1,2-Trimethylpropoximino)methyl, (1,2,2-Trimethylpropoximino)methyl, (1-Ethyl-1-methylpropoximino)methyl, (1-Ethyl-2-methylpropoximino)methyl, Methoximino-ethyl, Ethoximino-ethyl, n-Propoximinoethyl, (1-Methylethoximino)ethyl, n-Butoximino-ethyl, (1-Methylpropoximino)ethyl, (2-Methylpropoximino)ethyl, (1,1-Dimethylethoximino)ethyl, n-Pentoximinoethyl, (1-Methylbutoximino)ethyl, (2-Methylbutoximino)ethyl, (3-Methylbutoximino)ethyl, (2,2-Dimethylpropoximino)ethyl, (1-Ethylpropoximino)- ethyl, n-Hexoximino-ethyl, (1,1-Dimethylpropoximino)-ethyl, (1,2-Dimethylpropoximino)ethyl, (1-Methylpentoximino)ethyl, (2-Methylpentoximino)ethyl, (3-Methylpentoximino)ethyl, (4-Methylpentoximino)ethyl, (1,1-Dimethylbutoximino)ethyl, (1,2-Dimethylbutoximino)ethyl, (1,3-Dimethylbutoximino)ethyl, (2,2-Dimethylbutoximino)ethyl, (2,3-Dimethylbutoximino)ethyl, (3,3-Dimethylbutoximino)ethyl, (1-Ethylbutoximino)- ethyl, (2-Ethylbutoximino)ethyl, (1,1,2-Trimethylpropoximino)ethyl, (1,2,2-Trimethylpropoximino)ethyl, (1-Ethyl-1-methylpropoximino)ethyl, (1-Ethyl-2-methylpropoximino)ethyl, 2-(Methoximino)propyl, 3-(Methoximino)propyl und 2-(Ethoximino)propyl, vorzugsweise C₁-C₆-Alkoximino-C₁-C₂-alkyl wie Methoximino-methyl, Ethoximino-methyl, 2-Methoximino-ethyl und 2-Ethoximino-ethyl;
- C₁-C₆-Alkoxy-C₁-C₆-alkoxy: Methoxymethoxy, Ethoxymethoxy, n-Propoxymethoxy, (1-Methylethoxy)methoxy, n-Butoxymethoxy, (1-Methylpropoxy)methoxy, (2-Methylpropoxy)methoxy, (1,1-Dimethylethoxy)methoxy, n-Pentoxymethoxy, (1-Methylbutoxylmethoxy, (2-Methylbutoxy)methoxy, (3-Methylbutoxy)methoxy, (2,2-Dimethylpropoxy)methoxy, (1-Ethylpropoxy)methoxy, n-Hexoxymethoxy, (1,1-Dimethylpropoxy)methoxy, (1,2-Dimethylpropoxy)methoxy, (1-Methylpentoxy)methoxy, (2-Methylpentoxy)methoxy, (3-Methylpentoxy)methoxy, (4-Methylpentoxy)methoxy, (1,1-Dimethylbutoxy)methoxy, (1,2-Dimethylbutoxy)methoxy, (1,3-Dimethylbutoxy)methoxy, (2,2-Dimethylbutoxy)- methoxy, (2,3-Dimethylbutoxy)methoxy, (3,3-Dimethylbutoxy)methoxy, (1-Ethylbutoxy)methoxy, (2-Ethylbutoxy)- methoxy, (1,1,2-Trimethylpropoxy)methoxy, (1,2,2-Trimethylpropoxy)methoxy, (1-Ethyl-1-methylpropoxy)methoxy, (1-Ethyl-2-methylpropoxy)methoxy, Methoxyethoxy, Ethoxyethoxy, n-Propoxyethoxy, (1-Methylethoxy)ethoxy, n-Butoxyethoxy, (1-Methylpropoxy)ethoxy, (2-Methylpropoxy)ethoxy, (1,1-Dimethylethoxy)ethoxy, n-Pentoxyethoxy, (1-Methylbutoxy)ethoxy, (2-Methylbutoxy)ethoxy, (3-Methylbutoxy)ethoxy, (2,2-Dimethylpropoxy)ethoxy, (1-Ethylpropoxy)ethoxy, n-Hexoxyethoxy, (1,1-Dimethylpropoxy)ethoxy, (1,2-Dimethylpropoxy)ethoxy, (1-Methylpentoxy)ethoxy, (2-Methylpentoxy)ethoxy, (3-Methylpentoxy)ethoxy, (4-Methylpentoxy)ethoxy, (1,1-Dimethylbutoxy)ethoxy, (1,2-Dimethylbutoxy)ethoxy, (1,3-Dimethylbutoxy)ethoxy, (2,2-Dimethylbutoxy)ethoxy, (2,3-Dimethylbutoxy)ethoxy, (3,3-Dimethylbutoxy)ethoxy, (1-Ethylbutoxy)ethoxy, (2-Ethylbutoxy)ethoxy, (1,1,2-Trimethylpropoxy)ethoxy, (1,2,2-Trimethylpropoxy)ethoxy, (1-Ethyl-1-methylpropoxy)ethoxy, (1-Ethyl-2-methylpropoxy)ethoxy, 2-(Methoxy)propoxy, 3-(Methoxy)propoxy und 2-(Ethoxy)propoxy, vorzugsweise C₁-C₆-Alkoxy-C₁-C₂-alkoxy wie Methoxymethoxy, Ethoxymethoxy, 2-Methoxyethyl und 2-Ethoxyethyl;
- C₁-C₆-Alkylthio-C₁-C₆-alkoxy: Methylthiomethoxy, Ethylthiomethoxy, n-Propylthiomethoxy, (1-Methylethylthio)methoxy, n-Butylthiomethoxy, (1-Methylpropylthio)methoxy, (2-Methylpropylthiolmethoxy; (1,1-Dimethylethylthio)methoxy, n-Pentylthiomethoxy, (1-Methylbutylthio)methoxy, (2-Methylbutylthiolmethoxy, (3-Methylbutylthio)methoxy, (2,2-Dimethylpropylthioimethoxy, (1-Ethylpropylthio)methoxy, n-Hexylthiomethoxy, (1,1-Dimethylpropylthio)methoxy, (1,2-Dimethylpropylthio)methoxy, (1-Methylpentylthio)methoxy, (2-Methylpentylthio)methoxy, (3-Methylpentylthio)methoxy, (4-Methylpentylthio)methoxy, (1,1-Dimethylbutylthio)methoxy, (1,2-Dimethylbutylthio)methoxy, (1,3-Dimethylbutylthio)methoxy, (2,2-Dimethylbutylthio)methoxy, (2,3-Dimethylbutylthio)methoxy, (3,3-Dimethylbutylthiol methoxy, (1-Ethylbutylthio)methoxy, (2-Ethylbutylthio)methoxy, (1,1,2-Trimethylpropylthio)methoxy, (1,2,2-Trimethylpropylthio)methoxy, (1-Ethyl-1-methylpropylthio)methoxy, (1-Ethyl-2-methylpropylthio)methoxy, Methylthioethoxy, Ethylthioethoxy, n-Propylthioethoxy, (1-Methylethylthio)ethoxy, n-Butylthioethoxy, (1-Methylpropylthio)ethoxy, (2-Methylpropylthio)ethoxy, (1,1-Dimethylethylthio)ethoxy, n-Pentylthioethoxy, (1-Methylbutylthio)ethoxy, (2-Methylbutylthio)ethoxy, (3-Methylbutylthio)ethoxy, (2,2-Dimethylpropylthio)ethoxy, (1-Ethylpropylthio)ethoxy, n-Hexylthioethoxy, (1,1-Dimethylpropylthio)ethoxy, (1,2-Dimethylpropylthio)ethoxy, (1-Methylpentylthio)ethoxy, (2-Methylpentylthio)ethoxy, (3-Methylpentylthio)ethoxy, (4-Methylpentylthio)ethoxy, (1,1-Dimethylbutylthio)ethoxy, (1,2-Dimethylbutylthio)ethoxy, (1,3-Dimethylbutylthio)ethoxy, (2,2-Dimethylbutylthio)ethoxy, (2,3-Dimethylbutylthio)ethoxy, (3,3-Dimethylbutylthio)ethoxy, (1-Ethylbutylthio)ethoxy, (2-Ethylbutylthio)ethoxy, (1,1,2-Trimethylpropylthio)ethoxy, (1,2,2-Trimethylpropylthio)ethoxy, (1-Ethyl-1-methylpropylthio)ethoxy, (1-Ethyl-2-methylpropylthio)ethoxy, 2-(Methylthio)propoxy, 3-(Methylthio)propoxy und 2-(Ethylthio)propoxy, vorzugsweise C₁-C₆-Alkylthio-C₁-C₂-alkoxy wie Methylthiomethoxy, Ethylthiomethoxy, 2-Methylthioethyl und 2-Ethylthioethyl;
- C₁-C₆-Alkylamino-C₁-C₆-alkoxy: Methylaminomethoxy, Ethylaminomethoxy, n-Propylaminomethoxy, (1-Methylethylamino)methoxy, n-Butylaminomethoxy, (1-Methylpropylamino)methoxy, (2-Methylpropylamino)methoxy, (1,1-Dimethylethylamino)methoxy, n-Pentylaminomethoxy, (1-Methylbutylamino)methoxy, (2-Methylbutylamino)methoxy, (3-Methylbutylamino)methoxy, (2,2-Dimethylpropylamino)methoxy, (1-Ethylpropylamino)methoxy, n-Hexylaminomethoxy, (1,1-Dimethylpropylamino)methoxy, (1,2-Dimethylpropylamino)methoxy, (1-Methylpentylamino)methoxy, (2-Methylpentylamino)methoxy, (3-Methylpentylamino)methoxy, (4-Methylpentylamino)methoxy, (1,1-Dimethylbutylamino)methoxy, (1,2-Dimethylbutylamino)methoxy, (1,3-Dimethylbutylamino)methoxy, (2,2-Dimethylbutylamino)methoxy, (2,3-Dimethylbutylamino)methoxy, (3,3-Dimethylbutylaminolmethoxy, (1-Ethylbutylamino)methoxy, (2-Ethylbutylamino)methoxy, (1,1,2-Trimethylpropylamino)methoxy, (1,2,2-Trimethylpropylamino)methoxy, (1-Ethyl-1-methylpropylamino)methoxy, (1-Ethyl-2-methylpropylamino)methoxy, Methylaminoethoxy, Ethylaminoethoxy, n-Propylaminoethoxy, (1-Methylethylamino)ethoxy, n-Butylaminoethoxy, (1-Methylpropylamino)ethoxy, (2-Methylpropylamino)ethoxy, (1,1-Dimethylethylamino)ethoxy, n-Pentylaminoethoxy, (1-Methylbutylaminolethoxy, (2-Methylbutylamino)ethoxy, (3-Methylbutylamino)ethoxy, (2,2-Dimethylpropylamino)ethoxy, (1-Ethylpropylamino)ethoxy, n-Hexylaminoethoxy, (1,1-Dimethylpropylamino)ethoxy, (1,2-Dimethylpropylamino)ethoxy, (1-Methylpentylamino)ethoxy, (2-Methylpentylamino)ethoxy, (3-Methylpentylamino)ethoxy, (4-Methylpentylamino)ethoxy, (1,1-Dimethylbutylamino)ethoxy, (1,2-Dimethylbutylamino)ethoxy, (1,3-Dimethylbutylamino)ethoxy, (2,2-Dimethylbutylamino)ethoxy, (2,3-Dimethylbutylamino)ethoxy, (3,3-Dimethylbutylamino)ethoxy, (1-Ethylbutylamino)ethoxy, (2-Ethylbutylamino)ethoxy, (1,1,2-Trimethylpropylamino)ethoxy, (1,2,2-Trimethylpropylamino)ethoxy, (1-Ethyl-1-methylpropylamino)ethoxy, (1-Ethyl-2-methylpropylamino)ethoxy, 2-(Methylamino)propoxy, 3-(Methylamino)propoxy und 2-(Ethylamino)propoxy, vorzugsweise C₁-C₆-Alkylamino-C₁-C₂-alkoxy wie Methylaminomethoxy, Ethylaminomethoxy, 2-Methylaminoethoxy und 2-Ethylaminoethoxy;
- Di-(C₁-C₆)-alkylamino-(C₁-C₆)-alkoxy: N,N-Dimethylaminomethoxy, N,N-Diethylaminomethoxy, N,N-Dipropylaminomethoxy, N,N-Di-(1-methylethyl)aminomethoxy, N,N-Dibutylaminomethoxy, N,N-Di-(1-methylpropyl)aminomethoxy, N,N-Di-(2-methylpropyl)aminomethoxy, N,N-Di-(1,1-dimethylethyl)aminomethoxy, N-Ethyl-N-methylaminomethoxy, N-Methyl-N-propylaminomethoxy, N-Methyl-N-(1-methylethyl)aminomethoxy, N-Butyl-N-methylaminomethoxy, N-Methyl-N- (1-methylpropyl) aminomethoxy, N-Methyl-N-(2-methylpropyl)aminomethoxy, N-(1,1-Dimethylethyl)-N-methylaminomethoxy, N-Ethyl-N-propylaminomethoxy, N-Ethyl-N-(1-methylethyl)aminomethoxy, N-Butyl-N-ethylaminomethoxy, N-Ethyl-N-(1-methylpropyl)aminomethoxy, N-Ethyl-N-(2-methylpropyl)aminomethoxy, N-Ethyl-N-(1,1-dimethylethyl)aminomethoxy, N-(1-Methylethyl)--N-propylaminomethoxy, N-Butyl-N-propylaminomethoxy, N-(1-Methylpropyl)-N-propylaminomethoxy, N-(2-Methylpropyl)-N-propylaminomethoxy, N-(1,1-Dimethylethyl)-N-propylaminomethoxy, N-Butyl-N-(1-methylethyl)aminomethoxy, N-(1-Methylethyl)-N-(1-methylpropyl)aminomethoxy, N-(1-Methylethyl)-N-(2-methylpropyl)aminomethoxy, N-(1,1-Di-methylethyl)-N-(1-methylethyl)aminoethoxy, N-Butyl-N-(1-methyl-propyl)aminomethoxy, N-Butyl-N-(2-methylpropyl)aminomethoxy, N-Butyl-N-(1,1-dimethylethyl)aminomethoxy, N-(1-Methylpropyl)-N-(2-methylpropyl)aminomethoxy, N-(1,1-Dimethylethyl)-N-(1-methylpropyl)aminomethoxy, N-(1,1-Dimethylethyl)-N-(2-methylpropyl)aminomethoxy, N,N-Dimethylaminoethoxy, N,N-Diethylaminoethoxy, N,N-Di(n-propyl)aminoethoxy, N,N-Di-(1-methylethyl)aminoethoxy, N,N-Dibutylaminoethoxy, N,N-Di-(1-methylpropyl)aminoethoxy, N,N-Di-(2-methylpropyl)aminoethoxy, N,N-Di-(1,1-dimethylethyl)aminoethoxy, N-Ethyl-N-methylaminoethoxy, N-Methyl-N-propylaminoethoxy, N-Methyl-N-(1-methylethyl)aminoethoxy, N-Butyl-N-methylaminoethoxy, N-Methyl-N-(1-methylpropyl)aminoethoxy, N-Methyl-N-(2-methylpropyl)aminoethoxy, N-(1,1-Dimethylethyl)-N-methylaminoethoxy, N-Ethyl-N-propylaminoethoxy, N-Ethyl-N-(1-methylethyl)aminoethoxy, N-Butyl-N-ethylaminoethoxy, N-Ethyl-N-(1-methyl-propyl)aminoethoxy, N-Ethyl-N-(2-methylpropyl)aminoethoxy, N-Ethyl-N- (1,1-dimethylethyl)aminoethoxy, N-(1-Methylethyl)-N-propylaminoethoxy, N-Butyl-N-propylaminoethoxy, N-(1-Methylpropyl)-N-propylaminoethoxy, N-(2-Methylpropyl)-N-propylaminoethoxy, N-(1,1-Dimethylethyl)-N-propylaminoethoxy, N-Butyl-N-(1-methyl-ethyl) aminoethoxy, N-(1-Methylethyl)-N-(1-methylpropyl)aminoethoxy, N-(1-Methylethyl)-N-(2-methylpropyl)aminoethoxy, N-(1,1-Dimethylethyl)-N-(1-methylethyl)aminoethoxy, N-Butyl-N-(1-methylpropyl)aminoethoxy, N-Butyl-N-(2-methylpropyl)aminoethoxy, N-Butyl-N-(1,1-dimethylethyl)aminoethoxy, N-(1-Methylpropyl)-N-(2-methyl-propyl)aminoethoxy, N-(1,1-Dimethylethyl)-N-(1-methylpropyl)aminoethoxy und N-(1,1-Dimethylethyl)-N-(2-methylpropyl)aminoethoxy;
- C₁-C₆-Alkylcarbonyl: Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl,2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2 -Trimethylpropylcarbonyl, 1,2,2-Trimethylpropylcarbonyl, 1-Ethyl-1-methylpropylcarbonyl und 1-Ethyl-2-methylpropoxycarbonyl, vorzugsweise C₁-C₄-Alkylcarbonyl wie Methylcarbonyl und Ethylcarbonyl;
- C₁-C₆-Alkoxycarbonyl und die C₁-C₆-Alkoxycarbonylteile in den Resten C₁-C₆-Alkoxycarbonyl-C₂-C₆-alkyl, C₁-C₆- Alkoxycarbonyl-C₁-C₆-alkoxy und C₁-C₆-Alkoxy-C₁-C₆-alkoxycarbonyl-C₁-C₆-alkyl: Methoxycarbonyl, Ethoxycarbonyl, n-Propoxycarbonyl, 1-Methylethoxy-carbonyl, n-Butoxycarbonyl, 1-Methylpropoxycarbonyl, 2-Methylpropoxycarbonyl, 1,1-Dimethylethoxycarbonyl, n-Pentoxycarbonyl, 1-Methylbutoxycarbonyl, 2-Methylbutoxycarbonyl, 3-Methylbutoxycarbonyl, 1,1-Dimethylpropoxycarbonyl, 1,2-Dimethylpropoxycarbonyl, 2,2-Dimethylpropoxycarbonyl, 1-Ethylpropoxycarbonyl, Hexoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methylpropoxycarbonyl und 1-Ethyl-2-methylpropyl-carbonyl, vorzugsweise C₁-C₄-Alkoxycarbonyl wie Methoxycarbonyl und Ethoxycarbonyl;
- C₁-C₆-Alkylaminocarbonyl: Methylaminocarbonyl, Ethylaminocarbonyl, n-Propylaminocarbonyl, 1-Methylethylaminocarbonyl, n-Butylaminocarbonyl, 1-Methylpropylaminocarbonyl, 2-Methylpropylaminocarbonyl, 1,1-Dimethylethylaminocarbonyl, n-Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, n-Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl und 1-Ethyl-2-methylpropylaminocarbonyl, vorzugsweise C₁-C₄-Alkylaminocarbonyl wie Methylaminocarbonyl und Ethylaminocarbonyl;
- Di-(C₁-C₆)-alkylaminocarbonyl: N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)--N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N- (2-methylpropyl)aminocarbonyl, N-Ethyl-N-(1,1-dimethylethyl)aminocarbonyl, N-(1-Methylethyl)--N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Di-methylethyl)-N-(1-methylethyl)aminocarbonyl, N-Butyl-N-(1-methyl-propyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl und N-(1,1-Dimethylethyl)- N-(2-methylpropyl)aminocarbonyl, vorzugsweise Dimethylaminocarbonyl und Diethylaminocarbonyl;
- C₁-C₆-Alkylcarbonyloxy: Acetyloxy, Propionyloxy, Butyryloxy, alpha-Methylpropionyloxy, n-Butylcarbonyloxy, 1-Methylpropylcarbonyloxy, 2-Methylpropylcarbonyloxy, 1,1-Dimethylethylcarbonyloxy, n-Pentylcarbonyloxy, 1-Methylbutylcarbonyloxy, 2-Methylbutylcarbonyloxy, 3-Methylbutylcarbonyloxy, 1,1-Dimethylpropylcarbonyloxy, 1,2-Dimethylpropylcarbonyloxy, 2,2-Dimethylpropylcarbonyloxy, 1-Ethylpropylcarbonyloxy, n-Hexylcarbonyloxy, 1-Methylpentylcarbonyloxy, 2-Methylpentylcarbonyloxy, 3-Methylpentylcarbonyloxy, 4-Methylpentylcarbonyloxy, 1,1-Dimethylbutylcarbonyloxy, 1,2-Dimethylbutylcarbonyloxy, 1,3-Dimethylbutylcarbonyloxy, 2,2-Dimethylbutylcarbonyloxy, 2,3-Dimethylbutylcarbonyloxy, 3,3-Dimethylbutylcarbonyloxy, 1-Ethylbutylcarbonyloxy, 2-Ethylbutylcarbonyloxy, 1,1,2-Trimethylpropylcarbonyloxy, 1,2,2-Trimethylpropylcarbonyloxy, 1-Ethyl-1-methylpropylcarbonyloxy und 1-Ethyl-2-methylpropylcarbonyloxy, vorzugsweise C₁-C₄-Alkylcarbonyloxy wie Methylcarbonyloxy und Ethylcarbonyloxy;
- C₁-C₆-Alkoxycarbonyl-C₁-C₆-alkyl: Methoxycarbonylmethyl, Ethoxycarbonylmethyl, n-Propoxycarbonylmethyl, (1-Methylethoxycarbonyl)methyl, n-Butoxycarbonylmethyl, (1-Methylpropoxycarbonyl)methyl, (2-Methylpropoxycarbonyl)methyl, (1,1-Dimethylethoxycarbonyl)methyl, n-Pentoxycarbonylmethyl, (1-Methylbutoxycarbonyl)methyl, (2-Methylbutoxycarbonyl)methyl, (3-Methylbutoxycarbonyl)methyl, (1,1-Dimethylpropoxycarbonyl)methyl, (1,2-Dimethylpropoxycarbonyl)methyl, (2,2-Dimethylpropoxycarbonyl)methyl, (1-Ethylpropoxycarbonyl)methyl, n-Hexoxycarbonylmethyl, (1-Methylpentoxycarbonyl)methyl, (2-Methylpentoxycarbonyl)methyl, (3-Methylpentoxycarbonyl)methyl, (4-Methylpentoxycarbonyl)methyl, (1,1-Dimethylbutoxycarbonyl)methyl, (1,2-Dimethylbutoxycarbonyl)methyl, (1,3-Dimethylbutoxycarbonyl)methyl, (2,2-Dimethylbutoxycarbonyl)methyl, (2,3-Dimethylbutoxycarbonyl)methyl, (3,3-Dimethylbutoxycarbonyl)methyl, (1-Ethylbutoxycarbonyl)methyl, (2-Ethylbutoxycarbonyl)methyl, (1,1,2-Trimethylpropoxycarbonyl)methyl, (1,2,2-Trimethylpropoxycarbonyl)methyl, (1-Ethyl-1-methylpropoxycarbonyl)methyl, (1-Ethyl-2-methylpropyl-carbonyl)methyl, Methoxycarbonylethyl, Ethoxycarbonylethyl, n-Propoxycarbonylethyl, (1-Methylethoxycarbonyl)ethyl, n-Butoxycarbonylethyl, (1-Methylpropoxycarbonyl)ethyl, (2-Methylpropoxycarbonyl)ethyl, (1,1-Dimethylethoxycarbonyl)ethyl, n-Pentoxycarbonylethyl, (1-Methylbutoxycarbonyl)ethyl, (2-Methylbutoxycarbonyl)ethyl, (3-Methylbutoxycarbonyl)ethyl, (1,1-Dimethylpropoxycarbonyl)ethyl, (1,2-Dimethylpropoxycarbonyl)ethyl, (2,2-Dimethylpropoxycarbonyl)ethyl, (1-Ethylpropoxycarbonyl)ethyl, n-Hexoxycarbonylethyl, (1-Methylpentoxycarbonyl)ethyl, (2-Methylpentoxycarbonyl)ethyl, (3-Methylpentoxycarbonyl)ethyl, (4-Methylpentoxycarbonyl)ethyl, (1,1-Dimethylbutoxycarbonyl)ethyl, (1,2-Dimethylbutoxycarbonyl)ethyl, (1,3-Dimethylbutoxycarbonyl)ethyl, (2,2-Dimethylbutoxycarbonyl)ethyl, (2,3-Dimethylbutoxycarbonyl)ethyl, (3,3-Dimethylbutoxycarbonyl)ethyl, (1-Ethylbutoxycarbonyl)ethyl, (2-Ethylbutoxycarbonyl)ethyl, (1,1,2-Trimethylpropoxycarbonyl)ethyl, (1,2,2-Trimethylpropoxycarbonyl)ethyl, (1-Ethyl-1-methylpropoxycarbonyl)ethyl, (1-Ethyl-2-methylpropyl-carbonyl)ethyl, 3-(Methoxycarbonyl)propyl, 2-(Methoxycarbonyl)propyl und 2-(Ethoxycarbonyl)propyl, vorzugsweise C1-C4-Alkoxycarbonyl-C1-C2-alkyl wie Methoxycarbonylmethyl, Ethoxycarbonylmethyl, 2-Methoxycarbonylethyl und 2-Ethoxycarbonylethyl;
- eine Heterocyclylgruppe sowie der Heterocyclylrest in der Heterocyclyl-C₁-C₆-alkylgruppe, wobei der Heterocyclus jeweils gesättigt oder partiell oder vollständig ungesättigt sein und ein bis vier Heteroatome tragen kann: Tetrahydrofuranyl, Tetrahydrothienyl, Pyrrolidinyl, Isoxazolidinyl, Isothiazolidinyl, Pyrazolidinyl, Oxazolidinyl, Thiazolidinyl, Imidazolidinyl, 1,2,4-oxadiazolidinyl, 1,3,4-Oxadiazolidinyl, 1,2,4-Thiadiazolidinyl, 1,3,4-Thiadiazolidinyl, 1,2,4-Triazolidinyl, 1,3,4-Triazolidin-2-yl, 2,3-Dihydrofuryl, 2,4-Dihydrofuryl, 2,3-Dihydrothienyl, 2,4-Dihydrothienyl, 2,3-Pyrrolin-2-yl, 2,4-Pyrrolinyl, 2,3-Isoxazolinyl, 3,4-Isoxazolinyl, 4,5-Isoxazolinyl, 2,3-Isothiazolinyl, 3,4-Isothiazolinyl, 4,5-Isothiazolinyl, 2,3-Dihydropyrazolyl, 3,4-Dihydropyrazolyl, 4,5-Dihydropyrazol-1-yl, 2,3-Dihydrooxazolyl, 3,4-Dihydrooxazolyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro- triazin-2-yl und 1,2,4-Tetrahydrotriazin-3-yl; Furanyl, Thienyl, Pyrrolyl, Isoxazolyl, isothiazolyl, Pyrazolyl, Oxazolyl, Thiazolyl, Imidazolyl, 1,2,4-Oxadiazolyl, 1,3,4-Oxadiazolyl, 1,2,4-Thiadiazolyl, 1,3,4-Thiadiazolyl, 1,2,4-Triazolyl, 1,3,4-Triazolyl, Pyridinyl, Pyridazinyl, Pyrimidinyl, Pyrazinyl, 1,2,4-Triazinyl, 1,3,5-Triazinyl, Dihydropyran-2-yl, Dihydropyran-3-yl, Dihydropyran-4-yl, Dihydrothiopyran-2-yl, Dihydrothiopyran-3-yl, Dihydrothipyran-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl,

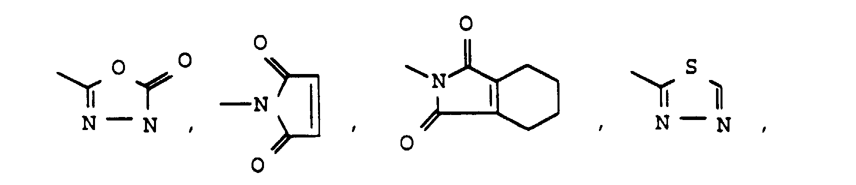

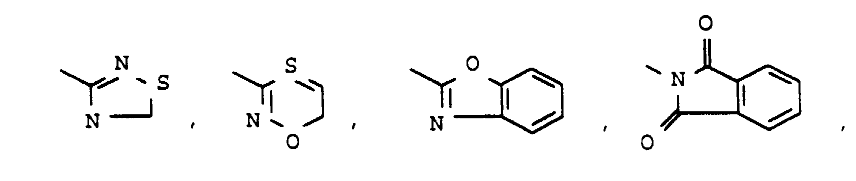

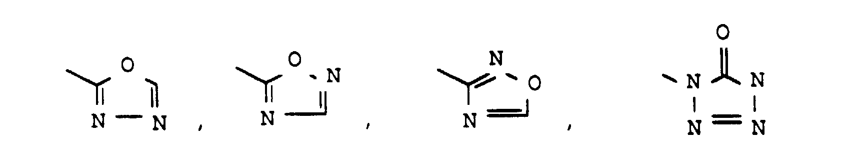

Die substituierten Cyclohexen-1,2-dicarbonsaurederivate Ia und Ib können in Form ihrer landwirtschaftlich brauchbaren Salze vorliegen, wobei im allgemeinen die Salze von solchen Basen in Betracht kommen, welche die herbizide Wirkung von Ia und Ib nicht beeinträchtigen.

Als basische Salze eignen sich besonders diejenigen der Alkalimetalle, vorzugsweise die Natrium- und Kaliumsalze, die der Erdalkalimetalle, vorzugsweise Calcium-, Magnesium-, und Bariumsalze und die der Übergangsmetalle, vorzugsweise Mangan-, Kupfer, Zink- und Eisensalze sowie die Ammoniumsalze, die ein bis drei C₁-C₄-Alkyl-, Hydroxy-C₁-C₄-alkylsubstituenten und/oder einen Phenyl- oder Benzylsubstituenten tragen können, vorzugsweise Diisopropylammonium-, Tetramethylammonium-, Tetrabutylammonium-, Trimethylbenzylammonium-, und Trimethyl-(2-hydroxyethyl)ammoniumsalze, die Phosphoniumsalze, die Sulfoniumsalze, vorzugsweise Tri-(C₁-C₄-)alkylsufoniumsalze, und die Sulfoxoniumsalze, vorzugsweise Tri-(C₁-C₄-)alkylsulfoxoniumsalze.

Im Hinblick auf die Verwendung der erfindungsgemäßen Cyclohexen-1,2-dicarbonsäurederivate Ia und Ib, Tetrahydrophthalisoimide IIIa und Tetrahydrophthalamidsäureester VIIIa als herbizide und defoliant/desikkant wirksame Verbindungen haben die Variablen vorzugsweise folgende Bedeutung:
- R¹ und R²: unabhängig voneinander einen Rest aus der Gruppe 1.01 - 1.179 oder
- R¹ und R²: zusammen einen Rest aus der Gruppe 2.01 - 2.07,
- R³: einen Rest aus der Gruppe 3.01 - 3.09,
- R⁴: einen Rest aus der Gruppe 4.01 - 4.08,
- R⁵: einen Rest aus der Gruppe 5.01 - 5.08,
- R⁶: eine Gruppe A-CO-B, wobei
A einen Rest aus der Gruppe A.01 - A.17,
B einen Rest aus der Gruppe B.01 - B.138,
wobei die Substituenten R¹, R², R³, R⁴, R⁵ und R⁶ beliebig miteinander kombiniert werden können, mit der Maßgabe, daß R¹ nur 1.01 bis 1.08 sein darf, wenn R² für 1.50 bis 1.156 steht.

**Tabelle 1**

| Nr. | R¹, R² |
|---|---|
| 1.01 | H |
| 1.02 | CH₃ |
| 1.03 | C₂H₅ |
| 1.04 | n-C₃H₇ |
| 1.05 | i-C₃H₇ |
| 1.06 | n-C₄H₉ |
| 1.07 | n-C₅H₁₁ |
| 1.08 | n-C₆H₁₃ |
| 1.09 | CH₂CH=CH₂ |
| 1.10 | CH(CH₃)-CH=CH₂ |
| 1.11 | CH₂-CH=CH-CH₂ |
| 1.12 | CH₂-C≡CH |
| 1.13 | CH(CH₃)-C≡CH |
| 1.14 | CH₂-C≡C-CH₃ |
| 1.15 | Cyclopropyl |
| 1.16 | Cyclobutyl |
| 1.17 | Cyclopentyl |
| 1.18 | Cyclohexyl |
| 1.19 | Cycloheptyl |
| 1.20 | (CH₂)₂Cl |
| 1.21 | CH₂Cl |
| 1.22 | Phenyl |
| 1.23 | 2-F-phenyl |
| 1.24 | 3-F-phenyl |
| 1.25 | 4-F-phenyl |
| 1.26 | 2-Cl-phenyl |
| 1.27 | 3-Cl-phenyl |
| 1.28 | 4-Cl-phenyl |
| 1.29 | 2-Br-phenyl |
| 1.30 | 3-Br-phenyl |
| 1.31 | 4-Br-phenyl |
| 1.32 | 2-CH₃-phenyl |
| 1.33 | 3-CH₃-phenyl |
| 1.34 | 4-CH₃-phenyl |
| 1.35 | 2-CF₃-phenyl |
| 1.36 | 3-CF₃-phenyl |
| 1.37 | 4-CF₃-phenyl |
| 1.38 | 2-OCH₃-phenyl |
| 1.39 | 3-OCH₃-phenyl |
| 1.40 | 4-OCH₃-phenyl |
| 1.41 | 4-NO₂-phenyl |
| 1.42 | 4-CN-phenyl |
| 1.43 | 2,4-Cl₂-phenyl |
| 1.44 | 2,4-(CH₃)₂-phenyl |
| 1.45 | CH₂-OCH₃ |
| 1.46 | (CH₂)₂-OC₂H₅ |
| 1.47 | OH |
| 1.48 | OCH₃ |
| 1.49 | OC₂H₅ |
| 1.50 | O-n-C₃H₇ |
| 1.51 | O-i-C₃H₇ |
| 1.52 | O-n-C₄H₉ |
| 1.53 | O-i-C₄H₉ |
| 1.54 | O-s-C₄H₉ |
| 1.55 | O-tert.-C₄H₉ |
| 1.56 | O-CH₂CH=CH₂ |
| 1.57 | O-CH(CH₃)CH=CH₂ |
| 1.58 | O-CH₂C≡CH |
| 1.59 | O-CH(CH₃)-C≡CH |
| 1.60 | O-CH₂-C≡C-CH₃ |
| 1.61 | O-CH₂-CH=CH-CH₃ |
| 1.62 | O-cyclopentyl |
| 1.63 | O-cyclohexyl |
| 1.64 | O-cyclopent-3-enyl |
| 1.65 | O-cyclohex-3-enyl |
| 1.66 | O-(CH₂)₂-Cl |
| 1.67 | O-(CH₂)₂-Cl |
| 1.68 | O-(CH₂)-F |
| 1.69 | O-CH₂-CF₃ |
| 1.70 | O-(CH₂)₂-Br |
| 1.71 | O-CH₂-CH=CHCl |
| 1.72 | O-CH₂-C(Cl)=CH₂ |
| 1.73 | O-CH₂-C(Br)=CH₂ |
| 1.74 | O-CH₂-CH=C(Cl)-CH₃ |
| 1.75 | O-CH₂-C(Cl)=CCl₂ |
| 1.76 | O-CH₂-cyclopropyl |
| 1.77 | O-CH₂-cyclobutyl |
| 1.78 | O-CH₂-cyclopentyl |
| 1.79 | O-CH₂-cyclohexyl |
| 1.80 | O-CH₂-cycloheptyl |
| 1.81 | O-CO-CH₃ |
| 1.82 | O-CO-C₂H₅ |
| 1.83 | O-CH₂-CN |
| 1.84 | O-(CH₂)₃-CN |
| 1.85 | O-CH₂-OCH₃ |
| 1.86 | O-CH₂-OC₂H₅ |
| 1.87 | O-(CH₂)₂-OCH₃ |
| 1.88 | O-(CH₂)₂-OC₂H₅ |
| 1.89 | O-(CH₂)₃-OC₂H₅ |
| 1.90 | O-(CH₂)₂-CO-OCH₃ |
| 1.91 | O-(CH₂)₂-CO-OC₂H₅ |
| 1.92 | O-C(CH₃)-CO-OCH₃ |
| 1.93 | O-C(CH₃)-CO-OC₂H₅ |
| 1.94 | O-(CH₂)₂-OH |
| 1.95 | O-CH₂-SCH₃ |
| 1.96 | O-(CH₂)₂-N(CH₃)₂ |
| 1.97 | O-(CH₂)₂-N(C₂H₅)₂ |
| 1.98 | O-CH₂-phenyl |
| 1.99 | O-(CH₂)₂-phenyl |
| 1.100 | O-(CH₂)₃-phenyl |
| 1.101 | O-(CH₂)₄-phenyl |
| 1.102 | O-(CH₂)₄-(4-C₁-phenyl) |
| 1.103 | O-(CH₂)₄-(4-CH₃-phenyl) |
| 1.104 | O-(CH₂)₄-(4-CH₃-phenyl) |
| 1.105 | O-(CH₂)₄-(4-F-phenyl) |
| 1.106 | O-CH₂CH=CH-phenyl |
| 1.107 | O-CH₂CH=CH-(4-F-phenyl) |
| 1.108 | O-CH₂CH=CH-(4-Cl-phenyl) |
| 1.109 | O-CH₂CH=CH-(3-OCH₃-phenyl) |
| 1.110 | O-(CH₂)₂-CH=CH-(4-F-phenyl) |
| 1.111 | O-(CH₂)₂-CH=CH-(4-Cl-phenyl) |
| 1.112 | O-(CH₂)-CH=CH-(3,4-Cl₂-phenyl) |
| 1.113 | O-CH₂-CH=C(CH₃)-(4-F-phenyl) |
| 1.114 | O-CH₂-C≡C-CH₂-phenyl |
| 1.115 | O-(CH₂)₂-O-phenyl |
| 1.116 | O-(CH₂)₂-OCH₂-phenyl |
| 1.117 | O-(CH₂)₂-OCH₂-(4-F-phenyl) |
| 1.118 | O-CH₂CH=CH-CH₂-O-phenyl |
| 1.119 | O-CH₂-C≡C-CH₂-O-phenyl |
| 1.120 | O-CH₂-C≡C-CH₂-O-(4-F-phenyl) |
| 1.121 | O-(CH₂)₂-SCH₂-phenyl |
| 1.122 | O-(CH₂)₂-SCH₂-(4-Cl-phenyl) |
| 1.123 | O-(CH₂)₂-N(CH₃)-CH₂-phenyl |
| 1.124 | NH₂ |
| 1.125 | NHCH₃ |
| 1.126 | NH-C₂H₅ |
| 1.127 | NH-n-C₃H₇ |
| 1.128 | NH-i-C₃H₇ |
| 1.129 | NH-n-C₄H₉ |
| 1.130 | NH-i-C₄H₉ |
| 1.131 | NH-s-C₄H₉ |
| 1.132 | NH-tert.-C₄H₉ |
| 1.133 | NH-cyclopropyl |
| 1.134 | NH-cyclobutyl |
| 1.135 | NH-cyclopentyl |
| 1.136 | NH-cyclohexyl |
| 1.137 | NH-cycloheptyl |
| 1.138 | N(CH₃)₂ |
| 1.139 | N(C₂H₅)₂ |
| 1.140 | NH-CH₂CH=CH₂ |
| 1.141 | NH-CH₂C≡CH |
| 1.142 | NH-CH₂-CF₃ |
| 1.143 | NH-CO-CH₃ |
| 1.144 | NH-COC₂H₅ |
| 1.145 | NH-CO-OCH₃ |
| 1.146 | NH-CO-OC₂H₅ |
| 1.147 | NH-COO-tert.-C₄H₉ |
| 1.148 | NH-phenyl |
| 1.149 | NH-(4-Cl-phenyl) |
| 1.150 | NH-(4-F-phenyl) |
| 1.151 | NH-(4-OCH₃-phenyl) |
| 1.152 | NH-(2,4-Cl₂-phenyl) |
| 1.153 | CH₂-OCH₃ |
| 1.154 | (CH₂)₂-OCH₃ |
| 1.155 | CH₂COOCH₃ |
| 1.156 | CH₂COOC₂H₅ |
| 1.157 | CH(CH₃)-COOCH |
| 1.158 | CH(CH₃)-COOC₂H₃ |
| 1.159 | (CH₂)₂-COOCH₃ |
| 1.160 | (CH₂)₃-COOCH₃ |
| 1.161 | CH(COOH)CH₃ |
| 1.162 | CH(COOH)CH₂OH |
| 1.163 | CH(COOH)CH(CH₃)-OH |
| 1.164 | CH(COOH)CH₂-Phenyl |
| 1.165 | CH(COOH)CH₂-4-OH-Phenyl |
| 1.166 | CH(COOH)(CH₂)₄-NH₂ |
| 1.167 | CH(COOH)(CH₂)₂COOH |
| 1.168 | CH(COOH)(CH₂)₂COOH |
| 1.169 | CH(COOH)CH₂-CONH₂ |
| 1.170 | CH(COOH)(CH₂)₂-CONH₂ |
| 1.171 | CH(COOH)CH₂SH |
| 1.172 | CH(COOH)CH₂SCH₃ |
| 1.173 | CH(COOH)CH₂-CH(COOH)₂ |
| 1.174 | CH(COOH)CH₂-imidazol-3-yl |
| 1.175 | CH(COOH)CH₂-inden-3-yl |

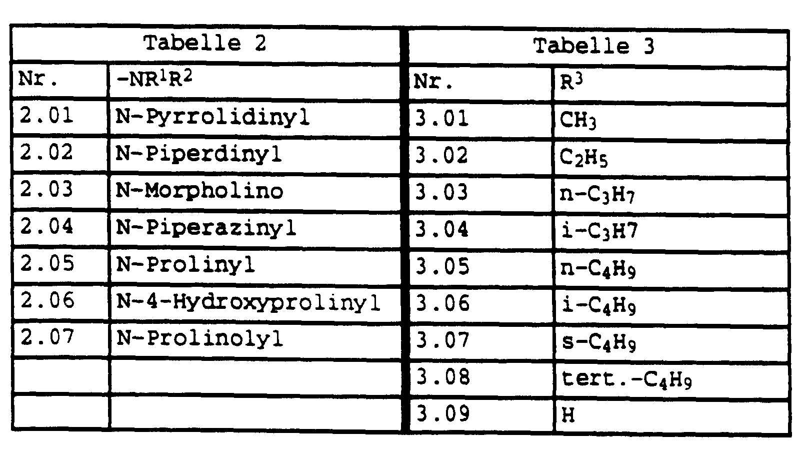

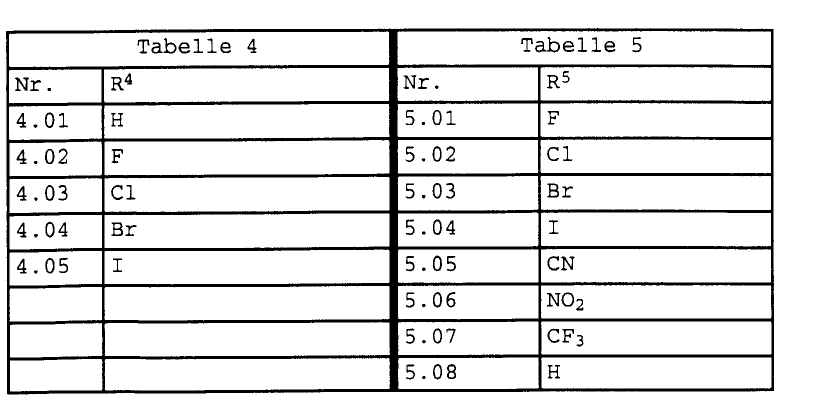

**Tabelle 6**

| Nr. | A | Nr. | A |
|---|---|---|---|
| A.1 | CH=CH- | A.11 | -CH=C(COCH₃)- |
| A.2 | -CH=CCl- | | |
| A.3 | -CH=CBr- | | |
| A.4 | -CH=CI- | | |
| A.5 | -CH=C(CH₃)- | A.12 | -C(CH₃)=CH- |
| A.6 | -CH=C(C₂H₅)- | A.13 | -C(CH₃)=CCl |
| A.7 | -CH=C(CN)- | A.14 | -C(CH₃)=CBr- |
| A.8 | -CH=C(COOCH₃)- | A.15 | -C(CH₃)=C(CH₃)- |
| A.9 | -CH=C(COOC₂H₅)- | A.16 | -C(CH₃)=C(CN)- |
| A.10 | -CH=C(OCOCH₃) - | A.17 | -CH=CF- |

**Tabelle 7**

| Nr. | B | Nr. | B |
|---|---|---|---|
| B.01 | H | B.34 | O-(4-Cl-phenyl) |
| B.02 | OH | B.35 | O-(4-Br-phenyl) |
| B.03 | OCH₃ | B.36 | O-(4-OCH₃-phenyl) |
| B.04 | OC₂H₅ | B.37 | O-(4-CN-phenyl) |
| B.05 | O-n-C₃H₇ | B.38 | O-(4-COOCH₃-phenyl) |
| B.06 | O-i-C₃H₇ | B.39 | O-(CH₃-phenyl) |
| B.07 | O-n-C₄H₉ | B.40 | O-(2,4-Cl₂-phenyl) |
| B.08 | O-i-C₄H₉ | B.41 | O-(2,4-(CH₃)₂-phenyl) |
| B.09 | O-s-C₄H₉ | B.42 | O-CH₂CN |
| B.10 | O-tert.-C₄H₉ | B.43 | O-CH₂CH=CCl₂ |
| B.11 | O-n-C₅H₁₁ | B.44 | O-CH₂CH=CHCl |
| B.12 | O-n-C₆H₁₃ | B.45 | O-CH₂OCH₃ |
| B.13 | O-CH₂CH=CH₂ | B.46 | O-CH₂OC₂H₅ |
| B.14 | O-CH(CH₃)CH=CH₂ | B.47 | O-C₂H₄OCH₃ |
| B.15 | O-CH-CH=CH-CH₂ | B.48 | O-C₂H₄OC₂H₅ |
| B.16 | O-CH₂-C≡CH | B.49 | O-CH(CH₃)-OCH₃ |
| B.17 | O-CH(CH₃)-C≡CH | B.50 | O-CH(CH₃)-OC₂H₅ |
| B.18 | O-CH₂-C≡C-CH₃ | B.51 | OCH₂C=NOCH₃ |
| B.19 | O-cyclopropyl | B.52 | O-C₂H₄C=NOCH₃ |
| B.20 | O-cyclobutyl | B.53 | O-CH₂C=NOC₂H₅ |
| B.21 | O-cyclopentyl | B.54 | O-C(O)CH₃ |
| B.22 | O-cyclohexyl | B.55 | O-C(O)C₂H₅ |
| B.23 | O-CH₂-CF₃ | B.56 | O-C₂H₄C=NOC₂H₅ |
| B.24 | O-CH₂-CCl₃ | B.57 | SCH₃ |
| B.25 | O-(CH₂)₃-Br | B.58 | SC₂H₅ |
| B.26 | O-phenyl | B.59 | S-n-C₃H₇ |
| B.27 | O-(2-F-phenyl) | B.60 | S-i-C₃H₇ |
| B.28 | O-(2-Cl-phenyl) | B.61 | S-CH₂CH=CH₂ |
| B.29 | O-(2-Br-phenyl) | B.62 | S-CH₂C≡CH |
| B.30 | O-(3-F-phenyl) | B.63 | S-phenyl |
| B.31 | O-(3-Cl-phenyl) | B.64 | S-CH₂CN |
| B.32 | O-(3-Br-phenyl) | B.65 | S-CH₂OCH₃ |
| B.33 | O-(4-F-phenyl) | B.66 | CH₃ |
| B.67 | C₂H₇ | B.103 | N(CH₃)₂ |
| B.68 | n-C₃ H₇ | B.104 | N(C₂H₅)₂ |
| B.69 | i-C₃H₇ | B.105 | N(CH₃)C₂H₅ |
| B.70 | n-C₄H₉ | B.106 | N(n-C₃H₇)₂ |
| B.71 | i-C₄H₉ | B.107 | NH-CH₂CH=CH₂ |
| B.72 | s-C₄H₉ | B.108 | NH-CH(CH₃)-CH=CH₂ |
| B.73 | tert.-C₄H₉ | B.109 | NH-CH₂-C≡CH |
| B.74 | n-C₅H₁₁ | B.110 | NH-CH(CH₃)-C≡CH |
| B.75 | n-C₆H₁₃ | B.111 | N(CH₃)-CH₂CH=CH |
| B.76 | CH₂CH=CH₂ | B.112 | N(CH₃)-CH₂C≡CH |
| B.77 | CH₂C-CH | B.113 | NH-cyclopropyl |
| B.78 | CH(CH₃)CH=CH₂ | B.114 | NH-cyclobutyl |
| B.79 | CH(CH₃C≡CH | B.115 | NH-cyclopentyl |
| B.80 | CH₂Cl | B.116 | NH-cyclohexyl |
| B.81 | CH₂Br | B.117 | N(CH₃)-cyclohexyl |
| B.82 | CHCl₂ | B.118 | N(C₂H₅)-cyclohexyl |
| B.83 | CF₃ | B.119 | NH-COCH₃ |
| B.84 | Cyclopropyl | B.120 | NH-COC₂H₅ |
| B.85 | Cyclobutyl | B.121 | NH-COOCH₃ |
| B.86 | Cyclopentyl | B.122 | NH-CH₂OCH₃ |
| B.87 | Cyclohexyl | B.123 | NH-(CH₂)₂CCH₃ |
| B.88 | Phenyl | B.124 | N-piperindinyl |
| B.89 | 2-F-phenyl | B.125 | N-pyrrolidinyl |
| B.90 | 3-F-phenyl | B.126 | N-morpholino |
| B.91 | 4-F-phenyl | B.127 | N-Piperazinyl |
| B.92 | 2-Cl-phenyl | B.128 | NH-phenyl |
| B.93 | 4-Cl-phenyl | B.129 | NH-(2-CH₃-phenyl) |
| B.94 | 2,4-Cl₂-Phenyl | B.130 | NH-(2-F-phenyl) |
| B.95 | CH₂-OCH₃ | B.131 | NH-(4-F-phenyl) |
| B.96 | CH(OCH₃)₂ | B.132 | NH-(2-Cl-phenyl) |
| B.97 | CH₂-SCH₃ | B.133 | NH-(4-Cl-phenyl) |
| B.98 | NH₂ | B.134 | NH-(2,4-Cl₂-Phenyl) |
| B.99 | NHCH₃ | B.135 | O-CO-OCH₃ |
| B.100 | NH-n-C₃H7 | B.136 | O-CO-OC₂H₅ |
| B.101 | NH-i-C₃H₇ | B.137 | O-CH₂-COOCH₃ |
| B.102 | NH-n-C₄H₉ | B.138 | O-CH(CH₃)-COOCH₃ |

Im Hinblick auf die herbizide Verwendung besonders bevorzugte Verbindungen sind nachfolgend in den Tabellen 17-21 aufgeführt:

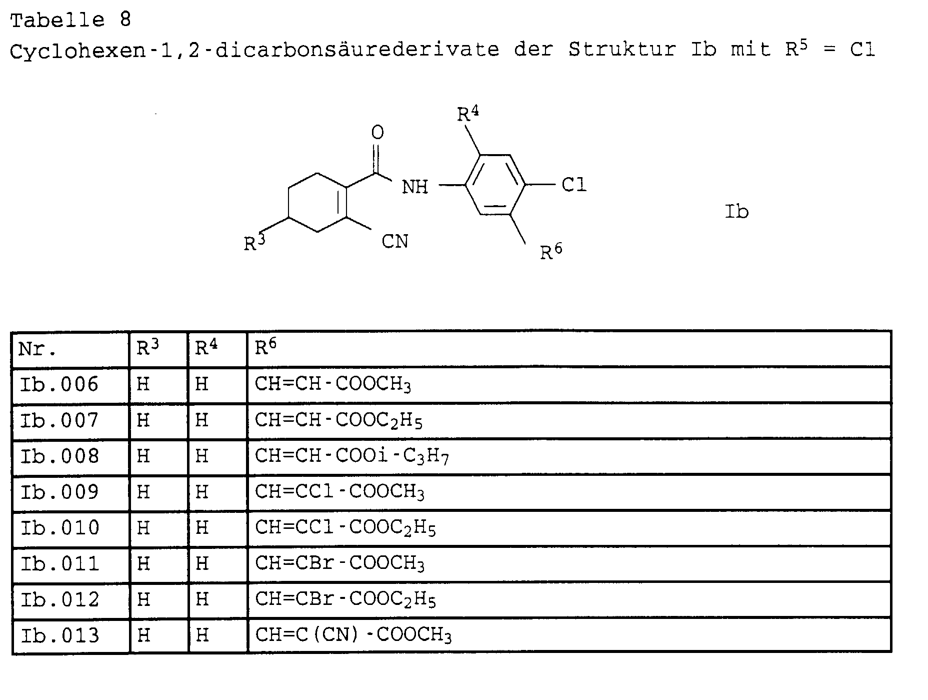

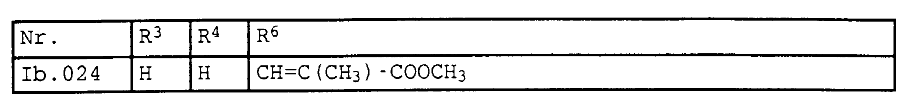

Des weiteren sind die folgenden Cyclohexen-1,2-dicarbonsäure-derivate Ib (R⁵ = C1) besonders bevorzugt.
- die Verbindungen Nr. Ib.106 bis Ib.113 und Ib.124, die sich von den entsprechenden Verbindungen Nr. Ib.006 bis Ib.013 und Ib.024 dadurch unterscheiden, daß R⁴ jeweils Fluor bedeutet;
- die Verbindungen Nr. Ib.206 bis Ib.213 und Ib.224, die sich von den entsprechenden Verbindungen Nr. Ib.006 bis Ib013 und Ib.024 dadurch unterscheiden, daß R³ jeweils für Methyl steht;
- die Verbindungen Nr. Ib.306 bis Ib.313 und Ib.324, die sich von den entsprechenden Verbindungen Nr. Ib.006 bis Ib.013 und Ib.024 dadurch unterscheiden, daß jeweils R³ für Methyl und R⁴ für Fluor stehen.

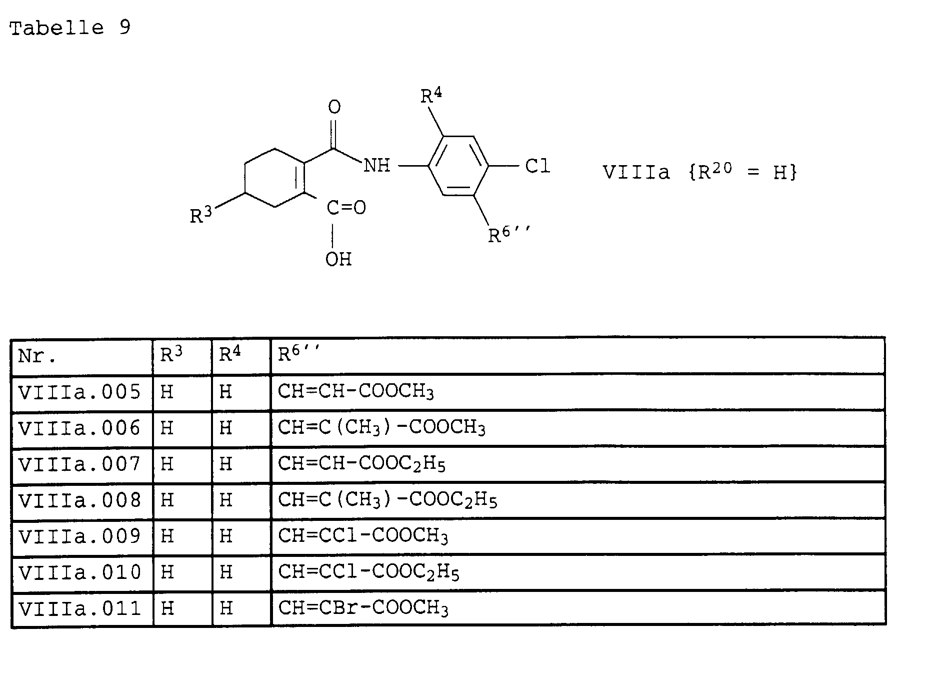

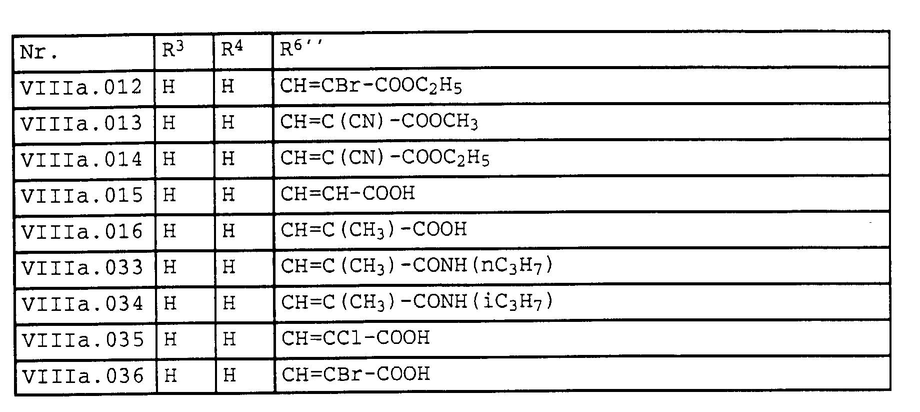

Des weiteren sind die folgenden Tetrahydrophthalamidsäureester VIIIa (R²⁰ = H) besonders bevorzugt:
- die Verbindungen Nr. VIIIa.105 bis VIIIa.116 und VIIIa.133 bis VIIIa.136, die sich von den entsprechenden Verbindungen Nr. VIIIa.005 bis VIIIa.016 und VIIIa.033 bis VIIIa.036 dadurch unterscheiden, daß R⁴ jeweils für Fluor steht;
- die Verbindungen Nr. VIIIa.205 bis VIIIa.216 und VIIIa.233 bis VIIIa.236, die sich von den entsprechenden Verbindungen Nr. VIIIa.005 bis VIIIa.016 und VIIIa.033 bis VIIIa.036 dadurch unterscheiden, daß R³ jeweils für Methyl steht;
- die Verbindungen Nr. VIIIa.305 bis VIIIa.316 und VIIIa.333 bis VIIIa.336, die sich von den entsprechenden Verbindungen Nr. VIIIa.006 bis VIIIa.016 und VIIIa.033 bis VIIIa.036 dadurch unterscheiden, daß jeweils R³ für Methyl und R⁴ für Fluor stehen;

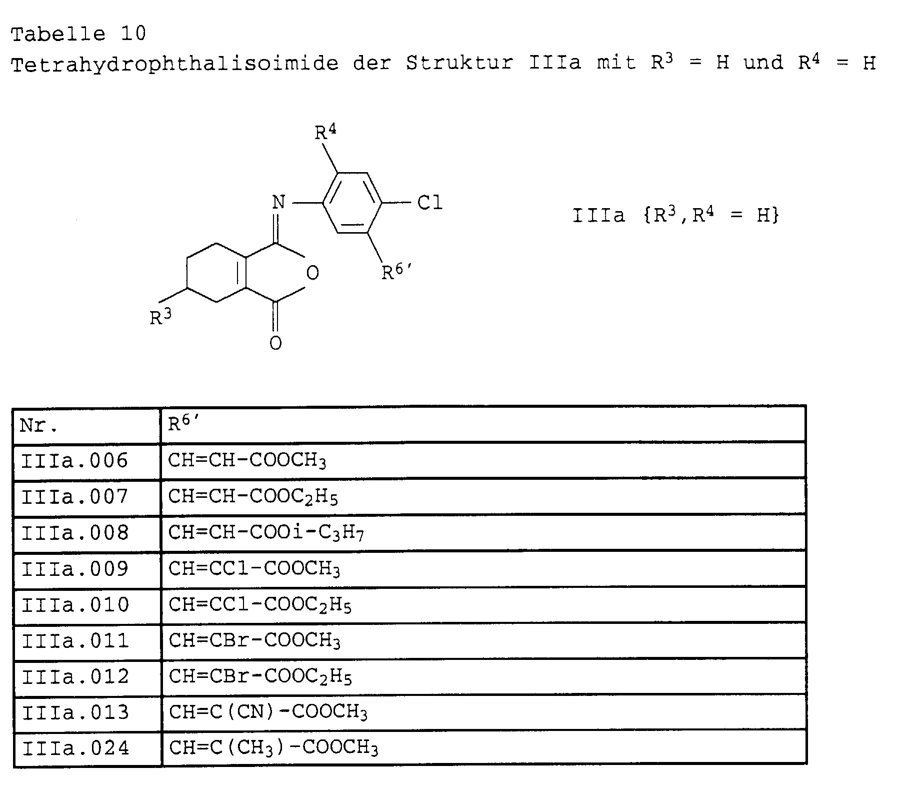

Des weiteren sind die den Verbindungen Nr. IIIa.006 bis IIIa.013 und IIIa.024 entsprechenden Tetrahydrophthalisoimide Nr. IIIa.106 bis IIIa.113 und IIIa.124, bei denen R⁴ jeweils für Fluor steht, besonders bevorzugt.

Die substituierten Cyclohexen-1,2-dicarbonsäurederivate Ia sind auf verschiedene Weise erhältlich, und zwar vorzugsweise nach einem der folgenden Verfahren:
a) Ringöffnung eines substituierten 3,4,5,6-Tetrahydrophthalimids der Formel II oder eines 3,4,5,6-Tetrahydrophtalisoimids der Formel III mit einem Amin, Hydroxylamin oder Hydrazin IV: 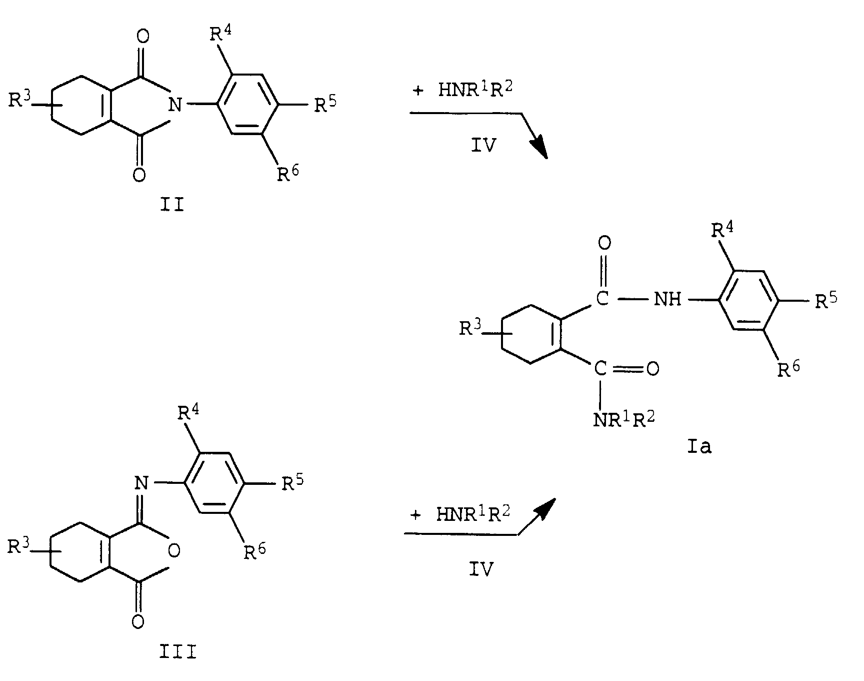
   In der Regel arbeitet man in einem inerten Lösungs- oder Verdünnungsmittel, beispielsweise in einem aliphatischen oder aromatischen Kohlenwasserstoff wie n-Hexan, Cyclohexan, Toluol und o-, m-, p-Xylol, in einem halogenierten Kohlenwasserstoff wie Dichlormethan und Chlorbenzol, in einem aliphatischen oder cyclischen Ethern wie Diethylether, 1,2-Dimethoxyethan, Tetrahydrofuran und Dioxan, in einem inerten polaren, organischen Lösungsmitteln wie Dimethylformamid, Dimethylsulfoxid und Acetonitril, in einem Alkohol wie Methanol, Ethanol und Diethylenglykol, in Aceton, Ethylacetat, Methylethylketon, Nitrobenzol, Wasser oder in einer Mischung der genannten Losungsmittel.
   Es besteht jedoch auch die Möglichkeit, ohne Lösungsmittel in einem Überschuß an IV zu arbeiten.
   In Abhangigkeit von den eingesetzten Edukten kann die Anwesenheit einer katalytisch wirksamen Menge eines Katalysators, beispielsweise einer Bronsted-Säure wie Chlorwasserstoff, oder einer Lewis-Säure wie Aluminiumtrichlorid und Bortrifluorid, die Reaktionsgeschwindigkeit erhöhen.
   Die Verbindungen der Formel IV können als freie Aminbasen oder in Form ihrer Ammonium-, Hydroxylammonium oder Hydrazinium-Salze eingesetzt werden, wobei Salze mit unter den Reaktionsbedingungen inerten Anionen in Betracht kommen. Beispiele für geeignete Salze sind diejenigen der Verbindungen IV mit anorganischen Mineralsäuren wie Halogenwasserstoffsäuren, insbesondere Salzsäure und Bromwasserstoffsäure, sowie Schwefelsäure und Salpetersäure, oder mit organischen Säuren wie Oxalsäure und Essigsäure.
   Die Mengenverhältnisse können normalerweise in weiten Bereichen variiert werden. Im allgemeinen liegt die Menge an IV jedoch zwischen 10 mol-% und einem 10-fachen molaren Überschuß, bezogen auf II oder III. Für einen größtmöglichen Umsatz an II oder III benötigt man mindestens äquimolare Mengen an IV. Ein größerer Überschuß an IV liegt insbesondere dann vor, wenn die Verbindung gleichzeitig als Reaktionspartner und als Lösungsmittel dient.
   Die Reaktionsführung erfolgt normalerweise zwischen (-40)°C und der Siedetemperatur des Reaktionsgemisches, vorzugsweise zwischen (-20) und 40°C.
   Die Reaktion ist vom Druck nicht erkennbar abhängig. Zweckmäßigerweise arbeitet man daher bei Atmosphärendruck oder unter dem Eigendruck des jeweiligen Losungsmittels.
b) Umsetzung eines substituierten Anilins der Formel V mit einem Tetrahydrophtalimid der Formel VI oder mit einem Tetrahydrophalisoimid der Formel VII, zu Verbindungen I (R¹ = Wasserstoff): 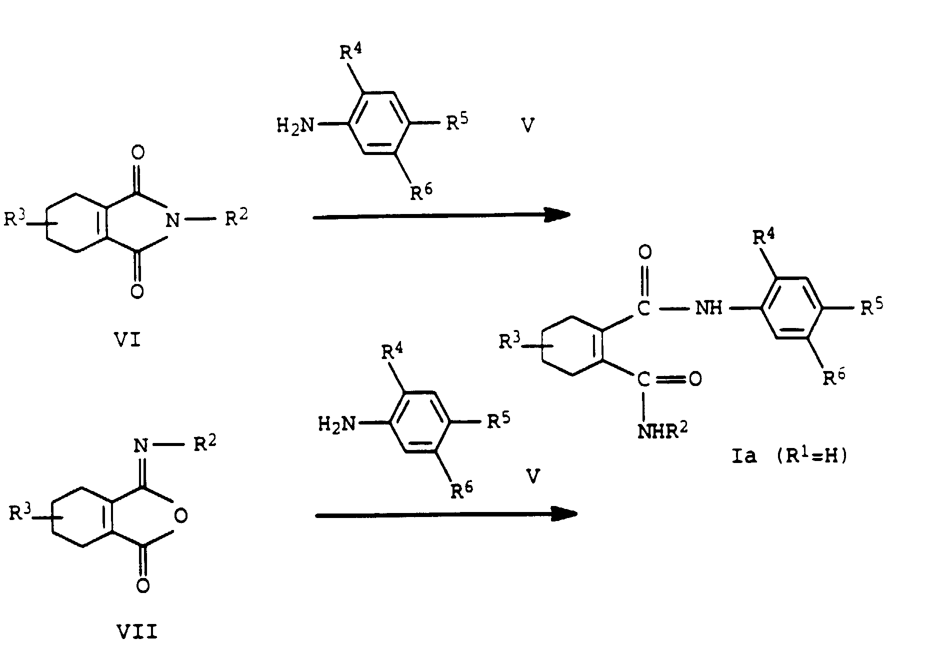
   Die Amine V können als freie Aminbasen oder in Form ihrer Ammonium-Salze eingesetzt werden, wobei Salze mit unter den Reaktionsbedingungen inerten Anionen in Betracht kommen. Beispiele für geeignete Salze sind diejenigen der Verbindungen V mit anorganischen Mineralsäuren wie Halogenwasserstoffsäuren, insbesondere Salzsäure und Bromwasserstoffsäure, sowie Schwefelsäure und Salpetersäure, oder mit organischen Säuren wie Oxalsäure und Essigsäure.
   Die Reaktionsführung erfolgt in einem inerten Lösungs- oder Verdünnungsmittel oder in einem Überschuß an V, wobei die Anwesenheit eines Katalysators vorteilhaft sein kann.
   Bezüglich des Lösungsmittels, des Katalysators, der Mengenverhältnisse, der Temperatur und des Druckes gelten die Angaben für Methode (a).
c) Kondensation einer Tetrahydrophtalamidsäure oder eines Tetrahydrophthalamidsäureesters VIII (R²⁰ = Wasserstoff oder Kohlenstoffrest) mit einem primären Amin IV (R¹=H) in an sich bekannter Weise (siehe z.B. Houben-Weyl, Methoden der Organischen Chemie, Band E5/2, 1985, Seiten 941 ff. und 983 ff.): 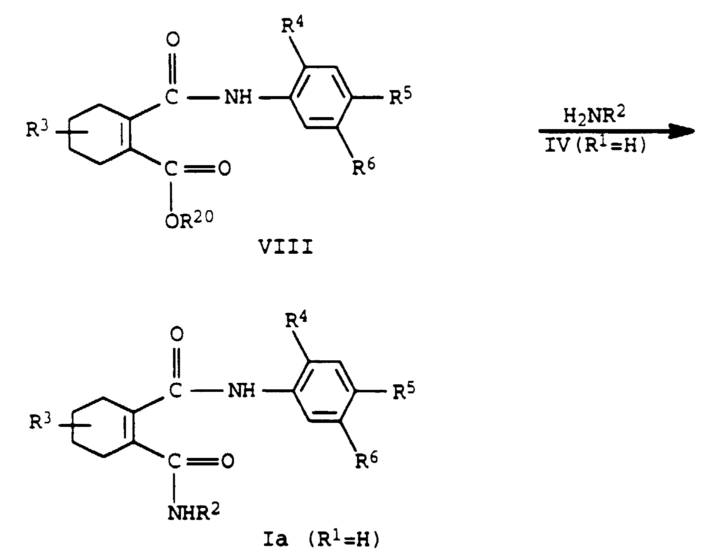
d) Umsetzung von Tetrahydrophtalamidsäurederivaten Ia (R¹ = H) mit Elektrophilen R¹-Y in Gegenwart einer Base: 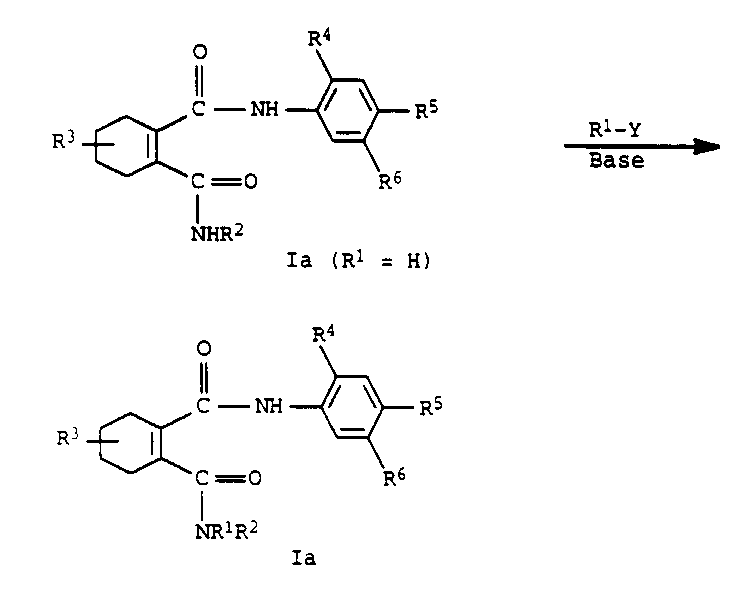
   Y steht für einen reaktionsfähigen Substituenten der bewirkt, daß R¹ auf das Stickstoffatom einer Amidgruppe als Elektrophil wirkt. Besonders geeignete Y sind Halogenatome wie Chlor und Brom, Alkyl- oder Halogenalkylsulfonyloxygruppen.
   Die Reaktionsführung erfolgt in an sich bekannter Weise (vgl. z.B. Houben-Weyl, Methoden der Organischen Chemie, Band E 5/2, 1985, Seiten 998 ff.).

Neben den genannten Synthesemethoden kann es in Abhängigkeit von den jeweiligen Substituenten vorteilhaft sein, die gewünschte Verbindung I aus einem anderen substituierten Cyclohexen-1,2-dicarbonsäurederivat Ia herzustellen:
e) Oxidation von substituierten Cyclohexen-1,2-dicarbonsäureamiden I, in denen R¹ für eine C₁-C₄-Alkylthio-C₁-C₄- alkylgruppe steht, zu substituierten Cyclohexen-1,2-dicarbonsäureamiden der Formel I, wobei R¹ C₁-C₄-Alkylsulfinyl-C₁-C₄-alkyl oder C₁-C₄-Alkylsulfonyl-C₁-C₄-alkyl bedeutet.
   Die Oxidation erfolgt auf an sich bekannte Weise (siehe z.B. Houben-Weyl, Methoden der Organischen Chemie, Band 11/1, 4. Auflage 1957, Seiten 702 ff. und Band 11/2, 4. Auflage 1958, Seiten 1194 ff.), wobei als Oxidationsmittel insbesondere m-Chlorperbenzoesäure und Wasserstoffperoxyd in Betracht kommen.
f) Acylierung eines substituierten Cyclohexen-1,2-dicarbonsäure-derivates Ia, wobei R¹ eine C₁-C₄-Hydroxyalkylgruppe bedeutet, mit einem Acylierungsmittel wie Acetylchlorid, zu einem substituierten Cyclohexen-1,2-dicarbonsäurederivate Ia, wobei R¹ für C₁-C₄-Alkylcarbonyloxy-C₁-C₄-alkyl steht.
   Die Umsetzung erfolgt auf an sich bekannte Weise (vgl. z.B. Houben-Weyl, Methoden der Organischen Chemie, Band E 5/2, 1985, Seite 1126).

Die substituierte Cyclohexen-1,2-dicarbonsäurederivate Ib sind ebenfalls auf verschiedene Art erhältlich und zwar vorzugsweise nach einem der folgenden Verfahren:
g) Dehydratisierung eines substituierten Cyclohexen-1,2-dicarbonsäureamids der Formel Ia, wobei R¹ und R² H bedeuten, mit einem geeigneten Dehydratisierungsmittel 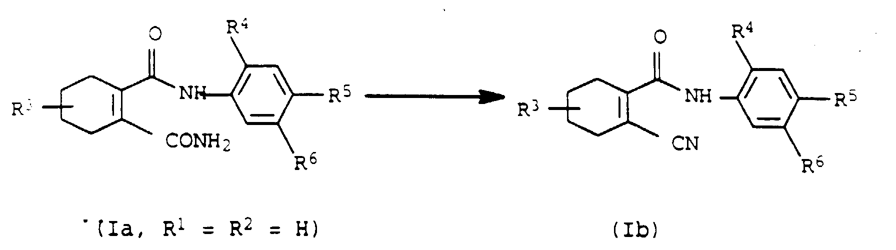
   In der Regel arbeitet man in einem inerten Lösungs- oder Verdünnungsmittel, beispielsweise in einem aliphatischen oder aromatischen Kohlenwasserstoff wie n-Hexan, Cyclohexan, Toluol und o-, m-, p-Xylol, in einem halogenierten Kohlenwasserstoff wie Dichlormethan und Chlorbenzol in einem aliphatischen oder cyclischen Ethern wie Diethylether, 1,2-Dimethoxyethan, Tetrahydrofuran und Dioxan, in einem inerten polaren, organischen Lösungsmitteln wie Dimethylformamid, Dimethylsulfoxid und Acetonitril oder in einer organischen Stickstoffbase, wie z.B. Pyridin oder Triethylamin.
   Als Dehydratisierungsmittel kommen insbesondere Cyanurchlorid, Chlorsulfonylisocyanat, p-Toluolsulfonsäurechlorid/Pyridin, Thionylchlorid oder besonders bevorzugt Trifluoracetanhydrid in Frage. Andere ebenfalls geeignete Entwässerungsmittel sind z.B. in Houben-Weyl, Methoden der organischen Synthese, Bd. E5, S. 1356 ff und Bd. VIII, S. 300 ff sowie in der dort zitierten Literatur beschrieben.
   Die Reaktionsführung erfolgt normalerweise zwischen -40°C und 120°C, bevorzugt zwischen -20°C und der Rückflußtemperatur des verwendeten Lösungsmittels.
   Die Reaktion ist von Druck nicht erkennbar abhängig. Zweckmäßigerweise arbeitet man daher bei Atmosphärendruck oder unter dem Eigendruck des jeweiligen Lösungsmittels.
h) Umsetzung von einem Säurechlorid der Formel IX mit einem Anilin der Formel V 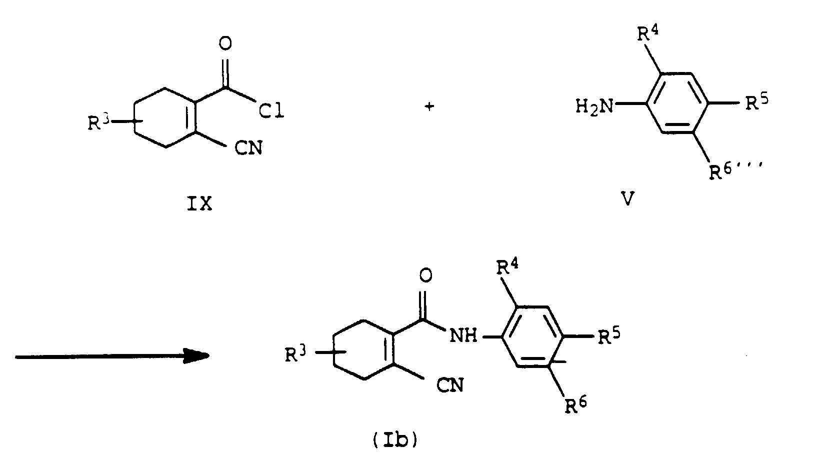
   Die Bedingungen, unter denen eine solche Reaktion durchgeführt wird, ist allgemein bekannt und z.B. beschrieben in Houben-Weyl, Methoden der org. Chemie, Bd. VIII, S. 655; Bd. XI/2 S. 10 ff; Bd. E5, 5972 ff.
   Bezüglich der Herstellung der Säurechloride IX sei auf Tetrahedron 32, 2379 f. (1976) und La Chimica e L'Industria 40, 887-995 (1958) sowie der dort zitierten Literatur verweisen.
i) Reaktionen an den Resten R⁵ und R⁶ in den Formeln Ia und Ib
   Beispielhaft genannt seien die Esterhydrolyse, Amidierung, Veresterung, Umesterung, Veretherung, Etherspaltung, Olefinierung, Reduktion, Oxidation oder der Halogen- oder Cyanaustausch.
   Die Reaktionsbedingungen sind im allgemeinen bekannt und chemischen Standardwerken wie Houben-Weyl, Methoden der Organischen Chemie oder R.C. Larock, Comprehensive Organic Transformations, VCH Publishes, New York 1989, zu entnehmen.

Die Herstellung der Verbindungen II erfolgt im allgemeinen durch Kondensation der Verbindungen XI mit Tetrahydrophthalsäureanhydriden XII. Hierbei kann die Anwesenheit einer Protonensäure wie p-Toluolsulfonsäure und Benzolsulfonsäure vorteilhaft sein:

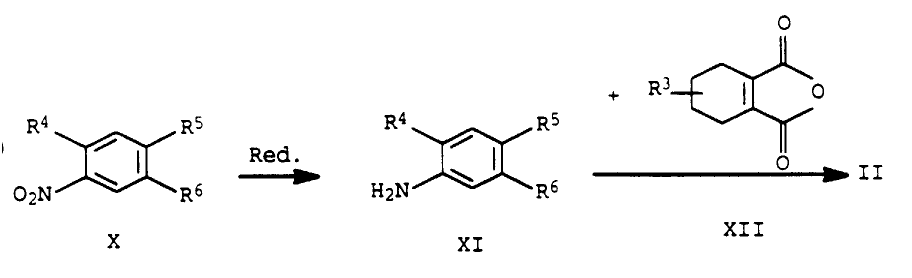

Die Kondensation erfolgt zweckmäßig in einem inerten organischen Lösungsmittel, wobei sich beispielsweise niedere Alkansäuren wie Essigsäure, Propionsäure und Isobuttersäure, Alkansäureester wie Essigsäureethylester, höhersiedende Kohlenwasserstoffe wie Toluol und Xylol, Amide wie Dimethylformamid oder auch Mischungen der genannten Solventien eignen.

Bei Verwendung eines aprotischen Lösungsmittels empfiehlt sich die kontinuierliche Entfernung das entstehenden Reaktionswassers.

Für einen größtmöglichen Umsatz benötigt man mindestens äquimolare Mengen an XI oder XII. Bevorzugt verwendet man einen geringen Überschuß einer der beiden Komponenten, bis etwa 10 mol-%.

Die Reaktionstemperatur liegt vorzugsweise zwischen 0°C und dem Siedepunkt des jeweiligen Reaktionsgemisches, insbesondere zwischen 20 und 140°C.

Normalerweise erfolgt die Reaktionsführung bei Atmosphärendruck oder unter dem Eigendruck des jeweiligen Losungsmittels. Höherer oder niedrigerer Druck ist möglich, bringt im allgemeinen aber keinen Vorteil.

Die Anilin-Derivate XI sind ihrerseits durch an sich bekannte Reduktion aus Verbindungen X zugänglich (vgl. z.B. Houben-Weyl, Methoden der Organischen Chemie, Band XI/1, 4. Auflage 1957, Seiten 341 ff.).

Als Reduktionsmittel kommen z. B.
- elementare Metalle wie Eisen,
- Wasserstoff in Gegenwart eines geeigneten Katalysators, vorzugsweise handelsübliche Edelmetall-Katalysatoren wie Platin auf Aktivkohle und Raney-Nickel, oder
- komplexe Metallhydride, z.B. Natriumborhydrid, in Gegenwart eines geeigneten Katalysators, vorzugsweise eines handelsüblichen Edelmetall-Katalysators, in Betracht (vgl. z.B. T. Neilson et al., J. Chem. Soc. 1962, 371 und dort zitierte Literatur.

Die reduzierten Produkte XI können auch ohne Isolierung der Kondensation mit XII unterworfen werden.

Weitere für die Synthese der Verbindungen Ia benötigten Zwischenprodukte II sind entweder bekannt oder nach an sich bekannten Methoden herstellbar. Beispielsweise sei auf die folgenden Druckschriften verwiesen: DE-A 36 03 789, DE-A 36 07 300, DE-A 37 41 273, EP-A 049 508, EP-A 083 055, EP-A 170 191, EP-A 177 032, EP-A 188 259, EP-A 207 894, EP-A 211 805, EP-A 218 972, EP-A 263 299, EP-A 271 170, EP-A 275 131, EP-A 288 960, EP-A 290 863, EP-A 296 416, EP-A 300 307, EP-A 300 387, EP-A 300 398, EP-A 313 963, EP-A 398 115, EP-A 400 427, U.S. 4,332,944, U.S. 4,816,065, JO 57/056 403, JO 59/082 360, JO 59/095 203, JO 59/155 358, JO 59/181 256, JO 60/152 465, JO 60/246 367, JO 61/027 962, JO 61/165 383, JO 61/174 970, JO 61/280 471, JO 62/114 961, JO 63/267 779, JO 63/275 580, JO 01/034 982, JO 01/047 784, JO 01/066 182.

Unter den 3,4,5,6-Tetrahydrophtalisoimiden III sind diejenigen der Formel IIIa 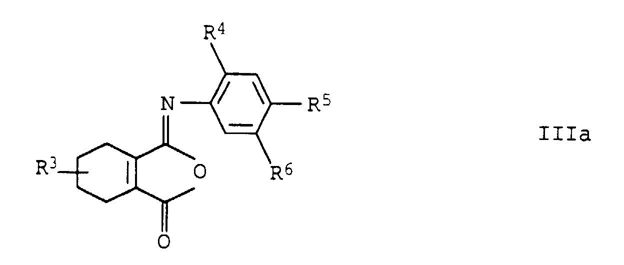 neu, wobei die Variablen folgende Bedeutung haben:
- R³: Wasserstoff oder eine C₁-C₆-Alkylgruppe;
- R⁴: Wasserstoff oder Halogen;
- R⁵: Wasserstoff, Halogen, Nitro, Cyano oder Trifluormethyl;
- R⁶: eine Gruppe -A-CO-B, wobei
A für
   eine C₂-Alkenylenkette, die unsubstituiert sein oder ein oder zwei Reste tragen kann, ausgewählt aus der Gruppe bestehend aus Halogen, Cyano, C₁-C₆-Alkyl, C₁-C₆-Halogenalkyl, Hydroxy, C₁-C₆-Alkoxy, C₁-C₆-Alkylcarbonyloxy, C₁-C₆-Alkoxycarbonyl und C₁-C₆-Alkylcarbonyl,
B
   - Wasserstoff, C₁-C₆-Alkyl, C₃-C₆-Alkenyl, C₃-C₆-Alkinyl, C₁-C₆-Halogenalkyl, C₃-C₇-Cycloalkyl,
   - -OR¹⁷ oder -SR¹⁷, wobei R¹⁷
      - Wasserstoff, C₁-C₆-Alkyl, C₃-C₆-Alkenyl, C₃-C₆-Alkinyl, C₃-C₆-Cycloalkyl, C₁-C₆-Halogenalkyl,
      - Phenyl, das unsubstituiert sein oder ein bis drei Reste tragen kann, ausgewählt aus einer Gruppe bestehend aus C₁-C₆-Alkyl, C₂-C₆-Alkenyl, Halogen, Cyano, Nitro, C₁-C₆-Halogenalkyl, C₁-C₆-Alkoxy und C₁-C₆-Alkoxycarbonyl,
      - C₁-C₆-Cyanoalkyl, C₃-C₆-Halogenalkenyl, C₁-C₆-Alkoxycarbonyl, C₁-C₆-Alkoxy-C₁-C₆-alkyl, C₁-C₆-Alkoxy-carbonyl-C₁-C₆-alkyl oder C₁-C₆-Alkyloximino-C₁-C₆-alkyl,
      bedeutet;
   - Phenyl, das unsubstituiert sein oder ein bis drei Reste tragen kann, ausgewählt aus einer Gruppe bestehend aus C₁-C₆-Alkyl, C₂-C₆-Alkenyl, Halogen, Cyano, Nitro, C₁-C₆-Halogenalkyl, C₁-C₆-Alkoxy und C₁-C₆-Alkoxycarbonyl,
   - C₁-C₆-Alkoxy-C₁-C₆-alkyl, Di- (C₁-C₆-alkoxyl-C₁-C₆-alkyl, C₁-C₆-Alkylthio- C₁-C₆-alkyl, oder
   - -NR¹⁸R¹⁹, wobei R¹⁸ und R¹⁹ unabhängig voneinander Wasserstoff, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkylcarbonyl, C₁-C₆-Alkoxycarbonyl, C₁-C₆-Alkoxy-C₁-C₆-alkyl oder Phenyl, das unsubstituiert sein oder ein drei Reste tragen kann, ausgewählt aus einer Gruppe bestehend aus C₁-C₆-Alkyl, C₃-C₆-Alkenyl, Halogen, Cyano, Nitro, C₁-C₆-Alkoxy und C₁-C₆-Alkoxycarbonyl, bedeuten, oder wobei
      R¹⁸ und R¹⁹ zusammen mit dem gemeinsamen Stickstoffatom einen gesättigten oder partiell oder vollständig ungesättigten 4- bis 7-gliedrigen Ring bilden, der noch ein weiteres Stickstoffatom oder ein Sauerstoff oder Schwefelatom als zweites Ringglied enthalten kann;

Bezüglich der bekannten 3,4,5,6-Tetrahydrophthalisoimide III vgl. insbesondere DE-A 27 33 115, EP-A 271 170, EP-A 275 131, EP-A 073 409, JO 59/070 682 und JO 59/204 181.

Die 3,4,5,6-Tetrahydrophthalisoimide der Formel III werden zweckmäßigerweise durch intramolekulare Kondensation aus Verbindungen VIII erhalten:

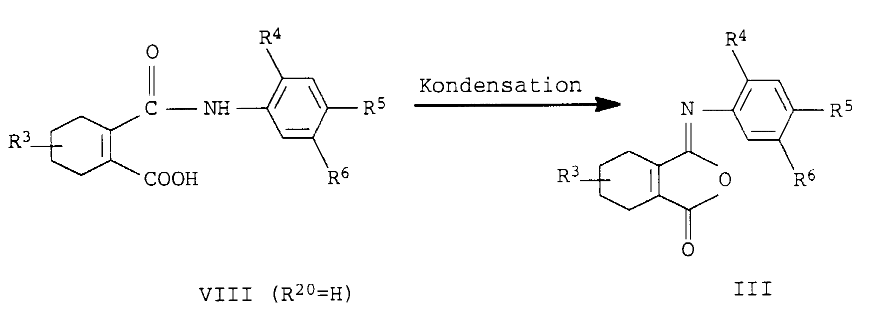

Die Kondensation erfolgt in einem inerten Lösungsmittel in Gegenwart eines Kondensationsmittels.

Als Lösungsmittel kommen beispielsweise aromatische Kohlenwasserstoffe wie Toluol und Xylol, aliphatische Kohlenwasserstoffe wie Hexan, halogenierte Kohlenwasserstoffe wie Dichlormethan oder Chlorbenzol, Ketone wie Aceton und Methylethylketon, Ether wie Diethylether, Dioxan und Tetrahydrofuran, Ester wie Ethylacetat, oder Nitrile wie Acetonnitril, in Betracht.

Als Kondensationsmittel eignen sich vornehmlich Carbodiimidderivate, z.B. Dicyclohexylcarbodiimide oder Diethylcarbodiimid, oder Kombinationen aus einer Base und einem Acylierungsmittel oder Säurechlorid.

Als Basen kommen hierfür beispielsweise aliphatische, aromatische oder heterocyclische Stickstoffverbindungen wie Triethylamin, Dimethylanilin und Pyridin, oder auch anorganische Basen wie Natriumcarbonat, Kaliumcarbonat und Natriumhydrogencarbonat in Betracht.

Unter den Säurechloriden sind z.B. Thionylchlorid und Phosphoroxychlorid geeignet.

Als Acylierungsmittel können beispielsweise Essigsaureanhydrid, Chlorameisensauremethylester, Trifluoressigsaureanhydrid oder Acetylchlorid verwendet werden.

Die Menge an Kondensationsmittel ist nicht kritisch. Um einen größtmöglichen Umsatz zu erzielen, empfiehlt sich die Verwendung der äquimolaren bis doppelten Menge an Kondensationsmittel, bezogen auf VIII.

Die Reaktionsführung erfolgt bei einer Temperatur von (-20) bis 100°C, bevorzugt von 0 bis 50°C.

Die Verbindungen VIII erhält man beispielsweise durch Umsetzung
- eines Anilins XI mit einem Tetrahydrophthalsäureanhydrid XII in einem inerten Lösungsmittel (vgl. hierzu die U.S. 4,472,190 sowie die dort zitierte Literatur) oder
- eines substituierten 3,4,5,6-Tetrahydrophthalimids II mit einem Hydroxid oder Alkoholat R²⁰-O- M⁺, wobei M⁺ das Äquivalent eines Metallions, insbesondere eines Alkalimetallions wie Lithium, Natrium und Kalium und R²⁰ Wasserstoff, C₁-C₆-Alkyl, C₃-C₆-Alkenyl oder Phenyl, das unsubstituiert sein oder ein bis drei Reste tragen kann, ausgewählt aus einer Gruppe bestehend aus Halogen, Cyano, Nitro, C₁-C₆-Alkyl, C₁-C₄-Halogenalkyl und C₁-C₆-Alkoxy, bedeuten.

Die Umsetzung eines Anilin-Derivats XI mit einem Tetrahydrophthalsäureanhydrid XII erfolgt in einem inerten Lösungs- oder Verdünnungsmittel, wobei hierfür insbesondere aromatische Kohlenwasserstoffe wie Toluol und Xylol, aliphatischen Kohlenwasserstoffe wie Hexan, halogenierte Kohlenwasserstoffe wie Dichlormethan und Chlorbenzol, Ether wie Diethylether, Dioxan und Tetrahydrofuran, Alkohole wie Methanol und Ethanol, oder organischen Säuren wie Essigsäure in Betracht kommmen.

Die Umsetzung eines substituierten 3,4,5,6-Tetrahydrophthalimids II mit einem Hydroxid oder Alkoholat wird ebenfalls in einem inerten Lösungs- oder Verdünnungsmittel vorgenommen, wobei sich hierfür beispielsweise aromatische Kohlenwasserstoffe wie Toluol und Xylol, aliphatischen Kohlenwasserstoffe wie Hexan, halogenierte Kohlenwasserstoffe wie Dichlormethan und Chlorbenzol, Ether wie Diethylether, Dioxan und Tetrahydrofuran, oder Alkohole wie Methanol und Ethanol, eignen.

Für beide Darstellungsvarianten werden die Reaktionspartner normalerweise in etwa äquimolaren Mengen eingesetzt, sofern sich nicht ein Überschuß der einen oder anderen Komponente, bis etwa 10 mol-%, empfiehlt.

Die Reaktionstemperatur liegt üblicherweise zwischen 0 und 100°C, bevorzugt zwischen 10 und 60°C.

Die Verbindungen X, XI und XII sind bekannt oder nach an sich bekannten Methoden, wie sie z.B. in den oben erwähnten Druckschriften beschrieben sind, herstellbar.

Die Tetrahydrophthalamidsäureester der Formel VIIIa, 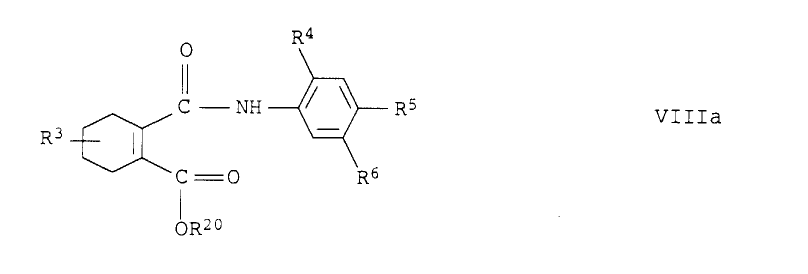 in der die Substituenten R³ bis R⁶ die in Anspruch 1 genannte Bedeutung besitzen, sind neu. (vgl. hierzu DE-A 27 33 115, EP-A 073 409, EP-A 271 170, EP-A 275 131, JO 59/070 682 und JO 59/204 181).

Sowohl die substituierten Cyclohexen-1,2-dicarbonsäurederivate Ia und Ib, als auch die Tetrahydrophthalisoimide III und die Tetrahydrophthalamidsäureester VIII können bei der Herstellung als Isomerengemische anfallen. Alle Isomerengemische lassen sich jedoch gewünschtenfalls nach den hierfür üblichen Methoden, z.B. durch Kristallisation oder Chromatographie, gegebenenfalls an einem optisch aktiven Absorbat, in die reinen Isomere trennen.

Mischungen aus den optisch aktiven Isomeren, die ein Isomeres im Überschuß enthalten, lassen sich beispielsweise auch unter Verwendung optisch aktiver Ausgangsprodukte herstellen.

Die erfindungsgemäßen substituierten Cyclohexen-1,2-dicarbonsäurederivate Ia und Ib, Tetrahydrophthalisoimide IIIa und Tetrahydrophthalamidsäureester VIIIa eignen sich, sowohl als Isomerengemische als auch in Form der reinen Isomeren, als Herbizide und als Defoliations-/Desikkationsmittel.

Die substituierten Cyclohexen-1,2-dicarbonsäurederivate Ia und Ib, Tetrahydrophthalisoimide IIIa und Tetrahydrophthalamidsäureester VIIIa eignen sich, sowohl als Isomerengemische als auch in Form der reinen Isomeren, als Herbizide, insbesondere zur Bekämpfung von dikotylen Unkräutern.

Insbesondere bei niedrigen Aufwandmengen sind sie verträglich und somit selektiv in Kulturen wie Weizen, Reis, Mais, Soja und Baumwolle.

Die substituierten Cyclohexen-1,2-dicarbonsäurederivate Ia und Ib, Tetrahydrophthalisoimide IIIa und Tetrahydrophthalamidsäureester VIIIa eignen sich außerdem als Desikkantien und Defoliantien, insbesondere zur Entlaubung von Baumwolle, und als Defoliantien zur Austrocknung der oberirdischen Pflanzenteile bei Kulturpflanzen, z.B. Kartoffel, Sonnenblume, Sojabohne und Raps. Damit wird ein vollständig mechanisiertes Beernten dieser wichtigen Kulturpflanzen ermöglicht.

Von wirtschaftlichem Interesse ist ferner die Ernteerleichterung,die durch das zeitlich konzentrierte Abfallen der oder Vermindern der Haftfestigkeit am Baum bei Zitrusfrüchten, Oliven oder bei anderen Arten und Sorten von Kern-, Stein- und Schalenobst ermöglicht wird. Außerdem führen sie zu einer gleichmäßigen Abreife der Erntefrüchte.

Derselbe Mechanismus, d.h. die Förderung der Ausbildung von Trenngewebe zwischen Frucht- bzw. Blatt- und Sproßteil der Pflanze ist auch für ein gut kontrollierbares Entblättern von Nutzpflanzen wie insbesondere Baumwolle wesentlich. Außerdem führt die Verkürzung des Zeitintervalls, in dem die einzelnen Baumwollpflanzen reif werden, zu einer erhöhten Faserqualität nach der Ernte.

Die Verbindungen Ia, Ib, IIIa und VIIIa bzw. die sie enthaltenden herbiziden oder Desikkations-/Defoliations-Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der Wirkstoffe gewährleisten.

Die Verbindungen Ia, Ib, IIIa und VIIIa eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isocctyl-, Octyl- cder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulosein Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Iziorägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:
I. eine Mischung aus 20 Gew.-Teilen der Verbindung Nr. Ia.12, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine wäßrige Dispersion, die 0,02 Gew.-% des Wirkstoffs enthält.
II. eine Dispersion von 20 Gew.-Teilen der Verbindung Nr. Ia.09 in einer Mischung aus 40 Gew.-Teilen Cyclohexanon, 30 Gew.-Teilen Isobutanol, 20 Gew.-Teilen des Anlagerungsproduktes von 7 mol Ethylenoxid an 1 mol Isooctylphenol und 10 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.
III. eine Dispersion von 20 Gew.-Teilen der Verbindung Nr. Ib.010 in einer Mischung aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;
IV. eine in einer Hammermühle vermahlene Mischung aus 20 Gew.-Teilen der Verbindung Nr. IIb.02* (N-[4-Chlor-5-(2-chlor-2-methoxycarbonylethyl-1)phenyl] -3,4,5,6-tetrahydrophthalimid), 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
V. eine Mischung aus 3 Gew.-Teilen der Verbindung Nr. IIb.04* (N-[4-Chlor-5-(2-chlor-2-ethoxycarbonylethyl-1)phenyl]-3,4,5,6-tetrahydrophthalimid und 97 Gew.-Teilen feinteiligem Kaolin. Dieses Stäubemittel enthält 3 Gew.-% Wirkstoff;
VI. eine stabile ölige Dispersion aus 20 Gew.-Teilen der Verbindung Nr. IIIa.010, 2 Gew.-Teilen des Calciumsalzes der Dodecylbenzolsulfonsäure, 8 Gew.-Teilen Fettalkohol-polyglykolether, 2 Gew.-Teilen des Natriumsalzes eines Phenolsulfonsäure-harnstoffformaldehyd Kondensates und 68 Gew.-Teilen eines paraffinischen Mineralöls;

* Die Tetrahydrophthalimide IIb.02 und IIb.04 sind nicht erfindungsgemäß und stehen nur beispielhaft für eine erfindungsgemäße Verbindung.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

In Anbetracht der Vielseitigkeit der Applikationsmethoden können die Verbindungen Ia, Ib, IIIa und VIIIa bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rüben |
| Camellia sinensis | Teestrauch |
| Carthamus tinctorius | Saflor-Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Verbindungen Ia, Ib, IIIa und VIIIa mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen Ia, Ib, IIIa oder VIIIa allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können aber auch nichtphytotoxische Öle und ölkonzentrate zugesetzt werden.

### Herstellungsbeispiele

### Beispiel 1

### N-n-Propyl-N'-[4-chlor-3-(2-n-propylcarbamoyl-prop-1-en-1-yl)-phenyl]-3,4,5,6-tetrahydrophthaldiamid (Verbindung Ia.01)

Zu einer Lösung von 5,9 g n-Propylamin in ca. 150 ml Ethylacetat wurde unter Eiskühlung eine Lösung von 10,9 g.
N-[4-chlor-3-(2-chlorcarbonyl-prop-1-en-1-yl)-phenyl]-3,4,5,6-tetrahydrophthalimid in 200 ml Ethylacetat getropft. Die Mischung wurde 3 Stunden bei etwa 20°C gerührt, wonach man den gebildeten Feststoffanteil abtrennte, zweimal mit 5 gew.-%iger wässriger Salzsäure und dann einmal mit Petrolether wusch und anschließend unter reduziertem Druck bei 40°C trocknete. Das Rohprodukt wurde aus Ethylacetat umkristallisiert. Smp.: 130 - 131°C.

### Beispiel 2

### N,N-Dimethyl-N'-[4-chlor-3-(2-chlor-2-ethoxycarbonylethenyl)-phenyl]-3,4,5,6-tetrahydrophthaldiamid (Verbindung Ia.09)

Zu einer Lösung von 3,9 g N-[4-Chlor-3-(2-chlor-2-ethoxycarbonylethenyl)-phenyl]-3,4,5,6-tetrahydrophthalimid in 100 ml Acetonitril wurden bei etwa 20°C 1,39 ml einer 40-gew.%igen wässrigen Dimethylamin-Lösung gegeben. Die erhaltene Mischung wurde noch 20 Stunden bei 20-25°C gerührt, wonach man den gebildeten Feststoffanteil abtrennte, mit Petrolether wusch und schließlich trocknete. Smp.: 161 - 164°C.

### Beispiel 3

### N-[4-Chlor-3-(2-chlor-2-ethoxycarbonyl-ethenyl)-phenyl]-3,4,5,6-tetrahydrophthaldiamid (Verbindung Ia.12)

In eine Lösung von 5,9 g N-[4-Chloro-3-(2-chlor-2- ethoxycarbonyl-ethenyl)-phenyl]-3,4,5,6-tetrahydrophthalimid in 150 ml Acetonitril wurde bis zur Sättigung Ammoniak eingegast. Das erhaltene Reaktionsgemisch wurde anschließend noch 19 Std. bei 20-25°C gerührt, wonach man den gebildeten Feststoffanteil abtrennte und danach mit Petrolether wusch. Smp.: 185 - 186°C.

### Beispiel 4

### N-[4-chlor-3-(2-chlor-2-ethoxycarbonyl-ethenyl)-phenyl]-3,4,5,6-tetrahydrophthalisoimid (Verbindung IIIa.02)

Zu einer Suspension von 3,3 g N-[4-Chlor-3-(2-chlor-2- ethoxycarbonyl-ethenyl)-phenyl]-3,4,5,6-tetrahydrophthalsaure-monoamid in 40 ml Toluol wurden bei etwa 20°C 1,85 g Dicyclohexyl-carbodiimid gegeben. Anschließend rührte man die erhaltene Reaktionsmischung 3 Stunden bei 20-25°C. Nach Abtrennung des Feststoffanteils wurde die erhaltene klare Lösung eingeengt. Die Reinigung des Rohrprodukts erfolgte chromatographisch an Kieselgel (Laufmittel: Toluol/Ethylacetat im Verhältnis 8:2). Ausbeute: 0,9 g;

200 MHz-¹H-NMR (in CDCl₃; TMS als interner Standard): d = 1.4 ppm (t,3H), 1.76-1.88 ppm (m,4H), 2,35-2,62 ppm (m,4H), 4.38 ppm (q,2H), 7,14-7.56 ppm (m,2H), 7.88 ppm (d,1H), 8,12 ppm (s,1H).

### Beispiel 5

### N-[3-(2-Brom-2-methoxycarbonyl-ethenyl)-4-chlor-phenyl]-2-Cyanocyclohexencarbonsäureamid (Verb. Ib.02)

Zu einer Lösung von 5 g N-[3-(2"Brom-2-methoxycarbonylethenyl)-4-chlor-phenyl]-cyclohexen-1,2-dicarbonsäureamid in 80 ml Dichlormethan wurden 2,6 g Pyridin und anschließend bei 0 bis 5°C 4,6 g Trifluoressigsäureanhydrid in 10 ml Dichlormethan getropft. Nach 1 Std. Rühren bei 5°C und 2 Std. bei 25°C wurden 100 ml Wasser zugegeben und nochmals 30 min gerührt. Anschließend wurde die organische Phase abgetrennt und getrocknet. Nach Entfernen des Lösungsmittels wurde das Rohprodukt in 50 ml Diethylether gelöst. Aus der abgekühlten Lösung trennte man das auskristallisierte Produkt ab, dessen Schmelzpunkt 123-125°C betrug.

### Beispiel 6

### N-[4-Chlor-3-(2-chlor-2-ethoxycarbonyl-ethenyl)-phenyl]-3,4,5,6-tetrahydrophthalsäuremonoamid (Verbindung VIIIa.01)

Zu einer Lösung von 1,2 g Natriumhydroxid in 200 ml Ethanol wurden bei etwa 20 °C 10,8 g N-[4-Chlor-3-(2-chlor-2- ethoxycarbonylethenyl)-phenyl]-3,4,5,6-tetrahydrophthalsäureimid gegeben. Anschließend rührte man die erhaltene Reaktionsmischung 17 Stunden bei 20-25 °C, wonach man das Lösungsmittels entfernte. Der Rückstand wurde in 100 ml Wasser gelöst. Nach Ansäuern der erhaltenen wässrigen Phase mit 10 mol-%iger wässriger Salzsäure bis zu einen pH-Wert von 3 trennte man den gebildeten Feststoff ab. Das erhaltene Rohprodukt wurde mit Wasser und Petrolether gewaschen und schließlich getrocknet. Smp.: 131-132°C.

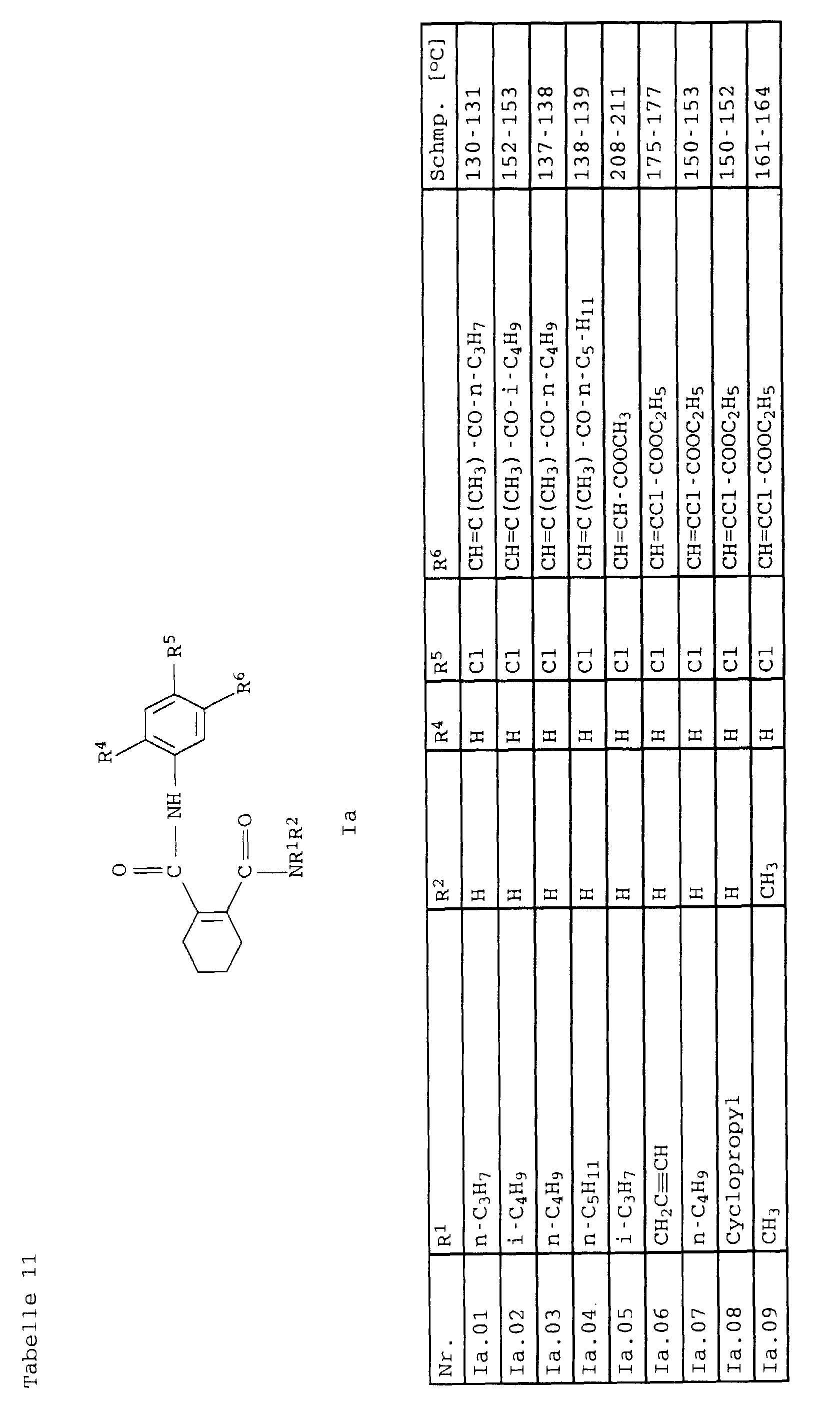

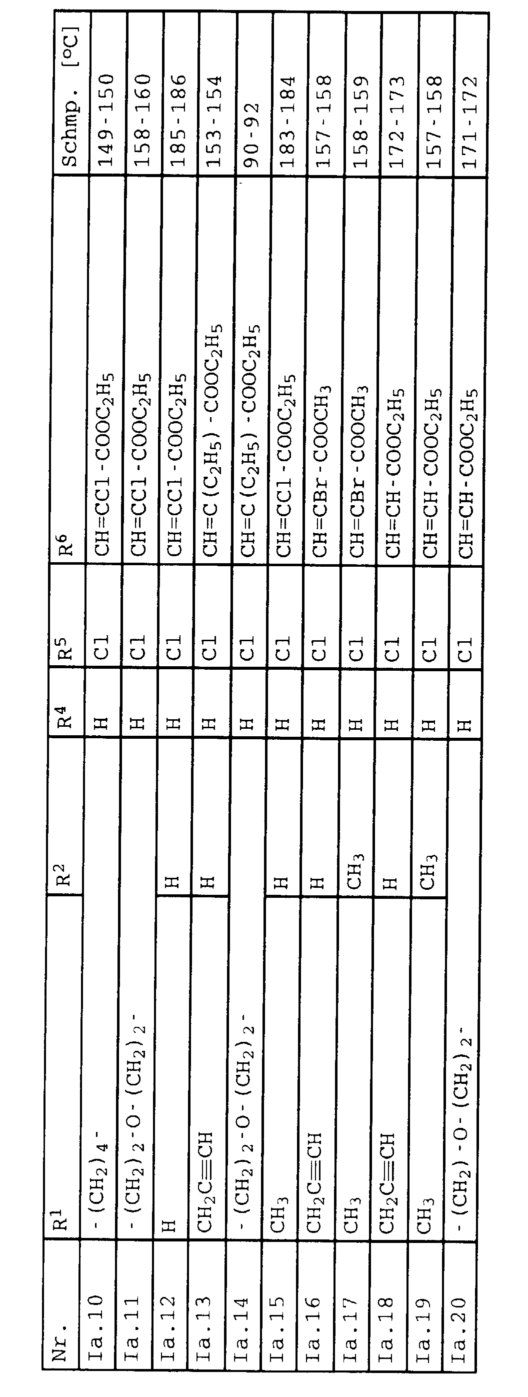

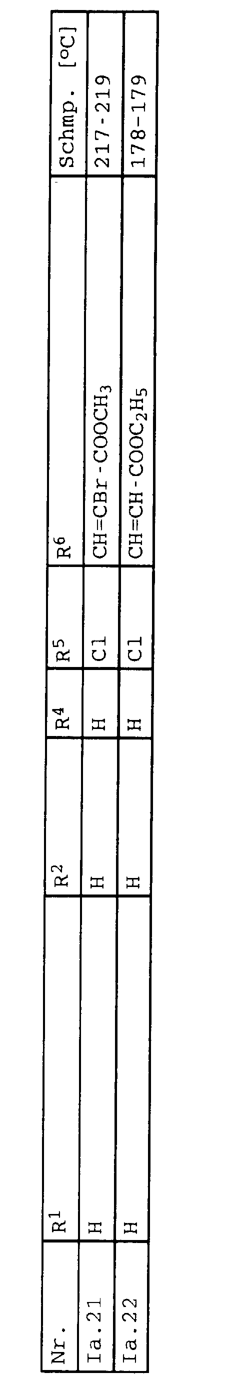

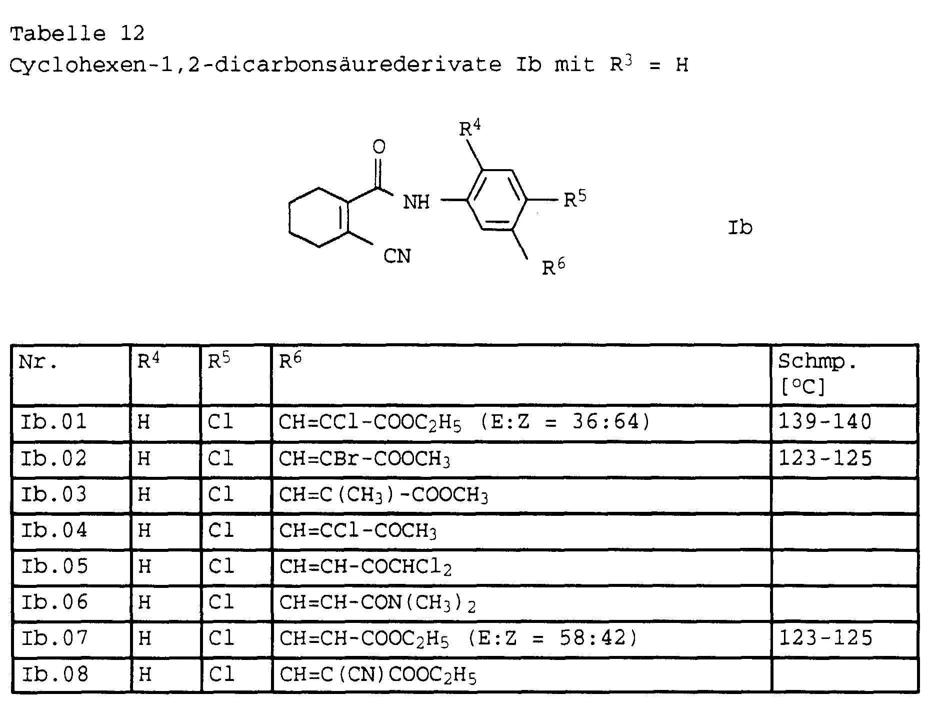

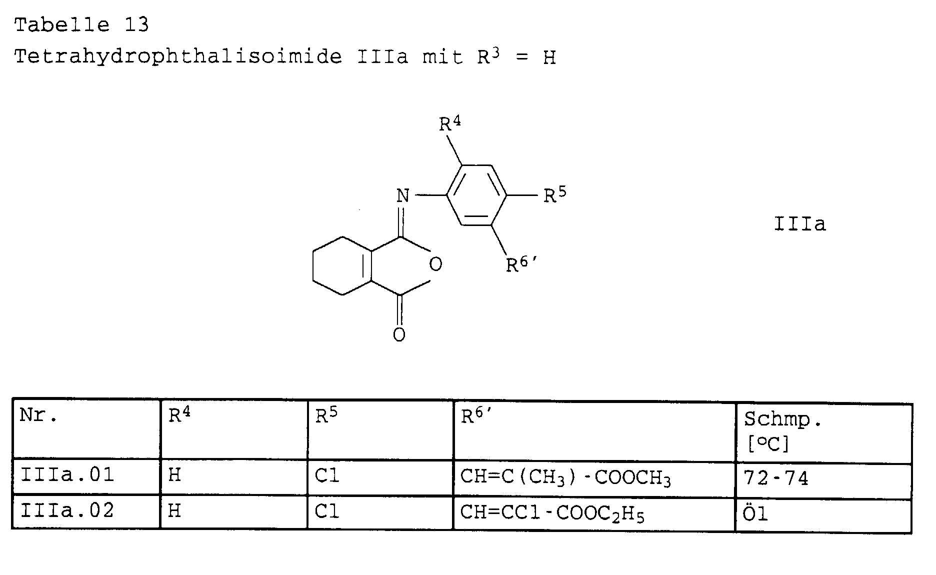

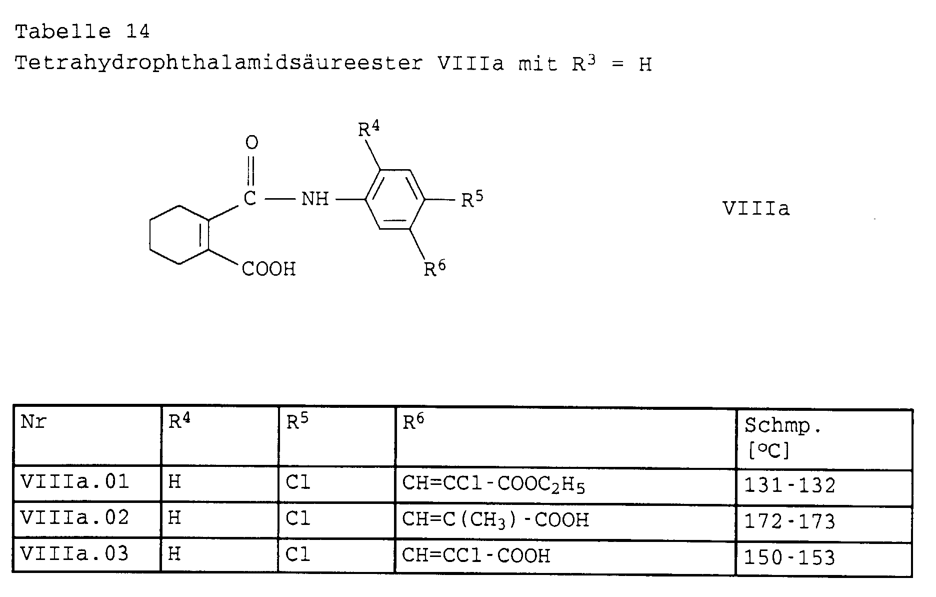

### Anwendungsbeispiele (herbizide Wirksamkeit)

Die herbizide Wirkung der substituierten Cyclohexen-1,2-dicarbonsäurederivate I, Tetrahydrophthalisoimide IIIa und Tetrahydrophthalamidsäureester VIIIa ließ sich durch Gewächshausversuche zeigen:

Als Kulturgefäße dienten Plastikblumentöpfe mit lehmigem Sand mit etwa 3,0 Gew.-% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkte ein gleichmäßigeres Keimen der Testpflanzen, sofern dies nicht durch die Wirkstoffe beeinträchtigt wurde.

Zum Zwecke der Nachauflaufbehandlung wurden die Testpflanzen je nach Wuchsform bis zu einer Wuchshöhe von 3 bis 15 cm angezogen und erst dann mit den in Wasser suspendierten oder emulgierten Wirkstoffen behandelt. Die Testpflanzen wurden entweder bereits in den Versuchsgefäßen gesät und aufgezogen, in denen sie behandelt wurden, oder sie wurden als Keimpflanzen getrennt angezogen und einige Tage vor der Behandlung mit den Wirkstoffaufbereitungen in die Versuchsgefäße verpflanzt.

Die Aufwandmenge für die Nachauflaufbehandlung betrug 0,5 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus | Zurückgekrümmter Fuchsschwanz | redroot pigweed |
| Centaurea cyanus | Kornblume | cornflower |
| Galium aparine | Klettenlabkraut | catchweed bedstraw |
| Ipomoea subspecies | Prunkwindearten | morningglory |

Das Ergebnis zeigte, daß mit den Verbindungen Nr. Ia.12, Ia.09, Ia.06, Ia.08, Ia.07, Ia.10, Ia.11, Ib.01 und IIIa.02 unerwünschte Unkräuter sehr gut bekämpft werden können.

## Patentansprüche

1. Substituierte Cyclohexen-1,2-dicarbonsaurederivate der allgemeinen Formeln Ia und Ib 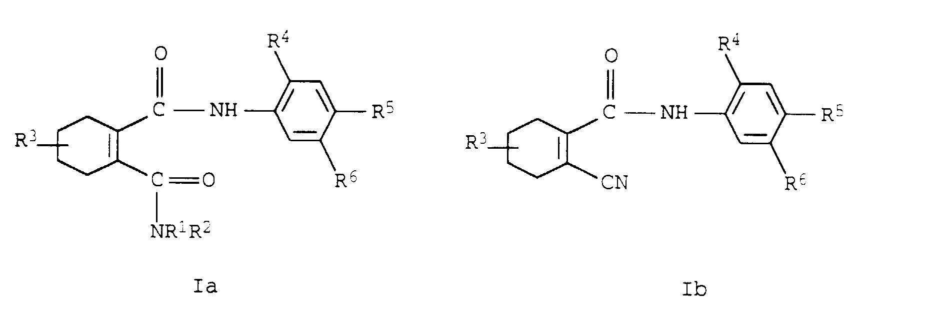 in der die Variablen folgende Bedeutung haben:
R¹, R²
- Wasserstoff,
- eine C₁-C₆-Alkyl-, C₃-C₆-Alkenyl- oder C₃-C₆-Alkinylgruppe, wobei jede dieser drei Gruppen noch einen bis drei Reste tragen kann, ausgewählt aus einer Gruppe bestehend aus Halogen, Cyano, Amino, Thio, Hydroxy, C₁-C₆-Alkoxy, C₁-C₆-Alkylsulfinyl, C₁-C₆-Alkylsulfonyl, C₁-C₆-Alkylcarbonyl, Carboxy, C₁-C₆-Alkoxycarbonyl, C₁-C₆-Alkylamino, C₁-C₆-Dialkylamino, C₁-C₆-Alkylaminocarbonyl, C₁-C₆-Dialkylaminocarbonyl, C₁-C₆-Alkylphosphono, C₁-C₆-Dialkylphosphono, Phenyl, 3- bis 8-gliedriges Heterocyclyl, das gesättigt oder partiell oder vollständig ungesättigt sein kann, wobei die Heterocyclen ein bis vier Heteroatome tragen können, ausgewählt aus einer Gruppe bestehend aus ein bis vier Stickstoffatomen, einem Sauerstoff- und einem Schwefelatom, und wobei die Phenyl- und heterocyclischen Reste ihrerseits an jedem substituierbaren Atom einen der folgenden Substituenten tragen können: Hydroxy, Halogen, Cyano, Nitro, Trifluormethyl, C₁-C₆-Alkyl oder C₁-C₆-Alkoxy,
- eine C₃-C₈-Cycloalkylgruppe,
- die Phenylgruppe oder eine gesättigte oder partiell oder vollständig ungesättigte 3- bis 8-gliedrige Heterocyclylgruppe, die jeweils ein bis vier Heteroatome tragen kann, ausgewählt aus einer Gruppe bestehend aus ein bis vier Stickstoffatomen, einem oder zwei Sauerstoff- und einem oder zwei Schwefelatomen, und wobei die Phenyl- und heterocyclischen Gruppen ihrerseits an jedem substituierbaren C-Atom einen der folgenden Reste tragen können: Hydroxy, Halogen, Cyano, Nitro, Trifluormethyl, Halogen oder C₁-C₆-Alkyl,
und, sofern R¹ für Wasserstoff oder eine C₁-C₆-Alkylgruppe steht,
R² zusätzlich
- Hydroxy,
- eine C₁-C₆-Alkoxy-, C₃-C₆-Alkenyloxy- oder C₃-C₆-Alkinyloxygruppe,
- eine C₃-C₇-Cycloalkoxy- oder C₅-C₇-Cycloalkenyloxygruppe,
- eine C₁-C₆-Halogenalkoxy- oder C₃-C₆-Halogenalkenyloxygruppe,
- eine C₃-C₇-cycloalkyl-C₁-C₆-alkoxygruppe,
- eine C₁-C₆-Alkylcarbonyloxygruppe,
- eine C₁-C₆-Cyanoalkoxygruppe,
- eine Hydroxy-C₁-C₆-alkoxy-, C₁-C₆-Alkoxy-C₁-C₆-alkoxy- oder C₁-C₆-Alkoxycarbonyl-C₁-C₆-alkoxygruppe,
- C₁-C₆-Alkylthio-C₁-C₆-alkoxygruppe,
- eine C₁-C₆-Alkylamino-C₁-C₆-alkoxy- oder C₁-C₆-Dialkylamino-C₁-C₆-alkoxygruppe,
- eine Phenyl-C₁-C₆-alkoxy-, Phenyl-C₃-C₆-alkenyloxy- oder Phenyl-C₃-C₆-alkinyloxygruppe, wobei jeweils eine oder zwei Methylengruppen der Alkoxy-, Alkenyloxy- und Alkinyloxy-Ketten durch Sauerstoff, Schwefel und/oder eine C₁-C₆-Alkylamino-Kette ersetzt sein können, und wobei der Phenylring jeweils unsubstituiert sein oder einen bis drei Reste tragen kann, ausgewählt aus der Gruppe bestehend aus C₁-C₆-Alkyl, C₂-C₆-Alkenyl, Halogen, Cyano, Nitro, C₁-C₆-Halogenalkyl, C₁-C₆-Alkoxy und C₁-C₆-Alkoxycarbonyl,
- eine Gruppe -N(R⁷)R⁸, wobei R⁷ und R⁸ unabhängig voneinander Wasserstoff, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkylcarbonyl, C₁-C₆-Alkoxycarbonyl, C₁-C₆-Alkoxy-C₁-C₆-alkyl oder Phenyl, das unsubstituiert sein oder ein bis drei Reste tragen kann, ausgewählt aus der Gruppe bestehend aus C₁-C₆-Alkyl, Halogen, Cyano, Nitro, C₁-C₆-Alkoxy und C₁-C₆-Alkoxycarbonyl, bedeuten,
oder wobei
R⁷ und R⁸ zusammen mit dem gemeinsamen Stickstoffatom einen gesättigten oder partiell oder vollständig ungesättigten 4- bis 7-gliedrigen Ring bilden, der noch ein weiteres Stickstoffatom oder ein Sauerstoff oder Schwefelatom als zweites Ringglied enthalten kann;
oder
R¹ und R² gemeinsam mit dem Stickstoff-Atom, an das sie gebunden sind,
einen drei- bis achtgliedrigen gesättigten oder ungesättigten, nicht aromatischen Heterocyclus, der ein bis drei Stickstoffatome und/oder ein Sauerstoff- oder Schwefelatom sowie ein oder zwei Carbonylgruppen tragen kann,
und wobei der Heterocyclus unsubstituiert sein oder ein bis drei C₁-C₆-Alkylreste tragen kann;
R³ Wasserstoff oder eine C₁-C₆-Alkylgruppe;
R⁴ Wasserstoff oder Halogen;
R⁵ Wasserstoff, Halogen, Nitro, Cyano oder Trifluormethyl;
R⁶ eine Gruppe -A-CO-B, wobei
A für
eine C₂-Alkenylenkette, die unsubstituiert sein oder ein oder zwei Reste tragen kann, ausgewählt aus der Gruppe bestehend aus Halogen, Cyano, C₁-C₆-Alkyl, C₁-C₆-Halogenalkyl, Hydroxy, C₁-C₆-Alkoxy, C₁-C₆-Alkylcarbonyloxy, C₁-C₆-Alkoxycarbonyl und C₁-C₆-Alkylcarbonyl,
B für
- Wasserstoff, C₁-C₆-Alkyl, C₃-C₆-Alkenyl, C₃-C₆-Alkinyl, C₁-C₆-Halogenalkyl, C₃-C₇-Cycloalkyl,
- OR¹⁷ oder -SR¹⁷, wobei R¹⁷
- Wasserstoff, C₁-C₆-Alkyl, C₃-C₆-Alkenyl, C₃-C₆-Alkinyl, C₃-C₆-Cycloalkyl, C₁-C₆-Halogenalkyl,
- Phenyl, das unsubstituiert sein oder ein bis drei Reste tragen kann, ausgewählt aus der Gruppe bestehend aus C₁-C₆-Alkyl, C₂-C₆-Alkenyl, Halogen, Cyano, Nitro, C₁-C₆-Halogenalkyl, C₁-C₆-Alkoxy und C₁-C₆-Alkoxycarbonyl,
- C₁-C₆-Cyanoalkyl, C₃-C₆-Halogenalkenyl, C₁-C₆-Alkoxycarbonyl, C₁-C₆-Alkoxy-C₁-C₆-alkyl, C₁-C₆-Alkoxy-carbonyl-C₁-C₆-alkyl oder C₁-C₆-Alkyloximino-C₁-C₆-alkyl,
bedeutet;
- Phenyl, das unsubstituiert sein oder ein bis drei Reste tragen kann, ausgewählt aus der Gruppe bestehend aus C₁-C₆-Alkyl, C₂-C₆-Alkenyl, Halogen, Cyano, Nitro, C₁-C₆-Halogenalkyl, C₁-C₆-Alkoxy und C₁-C₆-Alkoxycarbonyl,
- C₁-C₆-Alkoxy-C₁-C₆-alkyl, Di-(C₁-C₆-alkoxy)-C₁-C₆-alkyl C₁-C₆-Alkylthio-C₁-C₆-alkyl, oder
- -N(R¹⁸)R¹⁹, wobei R¹⁸ und R¹⁹ unabhängig voneinander Wasserstoff, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkylcarbonyl, C₁-C₆-Alkoxycarbonyl, C₁-C₆-Alkoxy-C₁-C₆-alkyl oder Phenyl, das unsubstituiert sein oder ein bis drei Reste tragen kann, ausgewählt aus der Gruppe bestehend aus C₁-C₆-Alkyl, C₃-C₆-Alkenyl, Halogen, Cyano, Nitro, C₁-C₆-Alkoxy und C₁-C₆-Alkoxycarbonyl, bedeuten,
oder wobei
R¹⁸ und R¹⁹ zusammen mit dem gemeinsamen Stickstoffatom einen gesättigten oder partiell oder vollständig ungesättigten 4- bis 7-gliedrigen Ring bilden, der noch ein weiteres Stickstoffatom oder ein Sauerstoff oder Schwefelatom als zweites Ringglied enthalten kann,
sowie die landwirtschaftlich brauchbaren Salze der Verbindungen Ia und Ib.

2. 3,4,5,6-Tetrahydrophthalisoimide der allgemeinen Formel IIIa 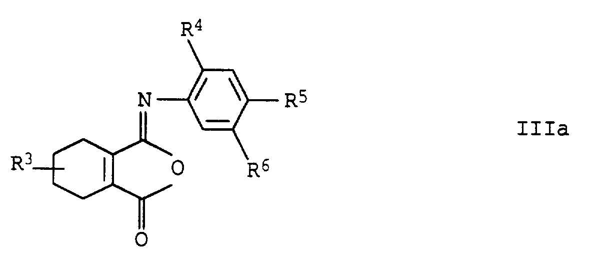 in der die Substituenten R³ bis R⁶ die in Anspruch 1 angegebenen Bedeutungen haben.

3. Tetrahydrophthalamidsäureester der allgemeinen Formel VIIIa 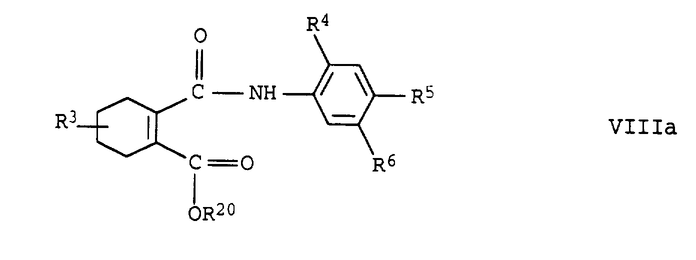 in der die Substituenten R³ bis R⁶ die in Anspruch 1 angegebenen Bedeutungen haben und
R²⁰ für Wasserstoff, eine C₁-C₆-Alkylgruppe, eine C₃-C₆-Alkenylgruppe oder eine Phenylgruppe, die unsubstituiert sein oder ein bis drei Reste tragen kann, ausgewählt aus der Gruppe bestehend aus Halogen, Cyano, Nitro, C₁-C₄-Alkyl, C₁-C₄-Halogenalkyl und C₁-C₆-Alkoxy,
steht.

4. Verfahren zur Herstellung von substituierten Cyclohexen-1,2-dicarbonsäurederivaten Ia nach Anspruch 1, dadurch gekennzeichnet, daß man ein substituiertes 3,4,5,6-Tetrahydrophthalimid der Formel II 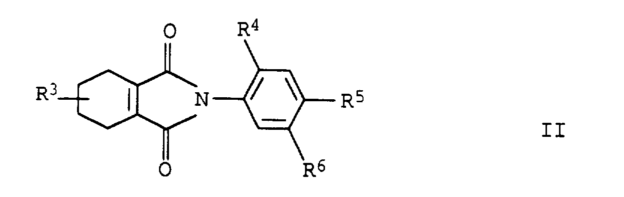 oder ein 3,4,5,6-Tetrahydrophthalisoimid der Formel III 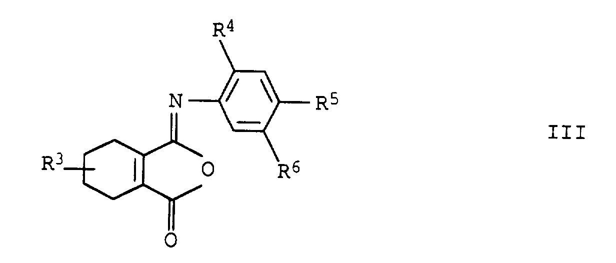 mit einem Amin, Hydroxylamin oder Hydrazin IV
HNR¹R² IV
umsetzt.

5. Verfahren zur Herstellung von substituierten Cyclohexen-1,2-dicarbonsäurederivate Ia nach Anspruch 1, wobei R¹ Wasserstoff bedeutet, dadurch gekennzeichnet, daß man ein substituiertes Anilin der Formel V 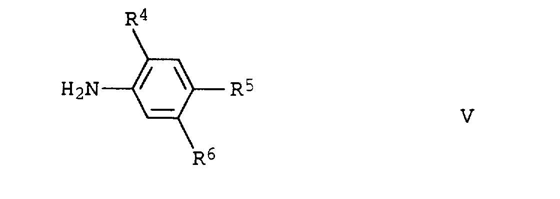 mit einem Tetrahydrophthalimid der Formel VI 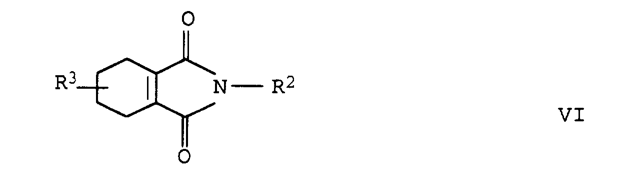 oder mit einem Tetrahydrophthalisoimid der Formel VII 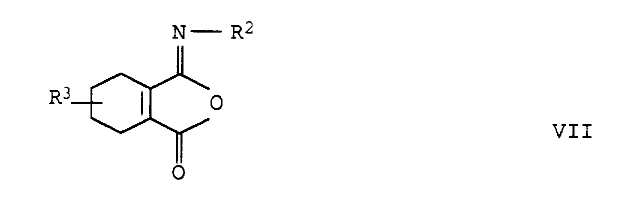 umsetzt.

6. Verfahren zur Herstellung von Cyclohexen-1,2-dicarbonsäurederivaten der Formel Ib nach Anspruch 1, dadurch gekennzeichnet, daß man die Cyclohexen-1,2-dicarbonsäurederivate der Formel Ia, wobei R¹ und R² Wasserstoff bedeuten, mit einem Dehydratisierungsmittel umsetzt.

7. Herbizides Mittel, enthaltend einen inerten flüssigen oder festen Trägerstoff und eine herbizid wirksame Menge mindestens eines substituierten Cyclohexen-1,2-dicarbonsäurederivates der Formeln Ia oder Ib oder ein landwirtschaftlich brauchbares Salz von Ia oder Ib gemäß Anspruch 1.

8. Verfahren zur Bekämpfung von unerwünschtem Pflanzenwuchs, dadurch gekennzeichnet, daß man eine herbizid wirksame Menge mindestens eines substituierten Cyclohexen-1,2-dicarbonsäurederivaten der Formeln Ia oder Ib oder ein landwirtschaftlich brauchbares Salz von Ia oder Ib gemäß Anspruch 1, auf Pflanzen, deren Lebensraum oder auf Saatgut einwirken läßt.

9. Mittel zur Desikkation und/oder Defoliation von Pflanzen, enthaltend neben üblichen Zusatzstoffen eine desikkant oder defoliant wirksame Menge mindestens eines substituierten Cyclohexen-1,2-dicarbonsäurederivaten der Formeln Ia oder Ib 5. oder ein landwirtschaftlich brauchbares Salz von Ia oder Ib gemäß Anspruch 1.

10. Verfahren zur Desikkation und/oder Defoliation von Pflanzen, dadurch gekennzeichnet, daß man eine desikkant oder defoliant wirksame Menge eines substituierten Cyclohexen-1,2-dicarbonsäurederivaten der Formeln Ia oder Ib oder ein landwirtschaftlich brauchbares Salz von Ia oder Ib gemäß Anspruch 1 auf die Pflanzen einwirken läßt.

11. Verfahren zur Bekämpfung von unerwünschtem Pflanzenwuchs, dadurch gekennzeichnet, daß man eine herbizid wirksame Menge mindestens eines 3,4,5,6-Tetrahydrophthalisoimids der Formel IIIa gemäß Anspruch 2, auf Pflanzen, deren Lebensraum oder auf Saatgut einwirken läßt.

12. Mittel zur Desikkation und/oder Defoliation von Pflanzen, enthaltend neben üblichen Zusatzstoffen eine desikkant oder defoliant wirksame Menge mindestens eines 3,4,5,6-Tetrahydrophthalisoimids der Formel IIIa gemäß Anspruch 2.

13. Verfahren zur Desikkation und/oder Defoliation von Pflanzen, dadurch gekennzeichnet, daß an eine desikkant oder defoliant wirksame Menge eines 3,4,5,6-Tetrahydrophthalisoimids der Formel IIIa gemäß Anspruch 2 auf die Pflanzen einwirken läßt.

14. Verfahren zur Bekämpfung von unerwünschtem Pflanzenwuchs, dadurch gekennzeichnet, daß man eine herbizid wirksame Menge mindestens eines Tetrahydrophthalamidsäureesters der Formel VIIIa gemäß Anspruch 3 auf Pflanzen, deren Lebensraum oder auf Saatgut einwirken läßt.

15. Mittel zur Desikkation und/oder Defoliation von Pflanzen, enthaltend neben üblichen Zusatzstoffen eine desikkant oder defoliant wirksame Menge mindestens eines Tetrahydrophthalamidsäureesters der Formel VIIIa gemäß Anspruch 3.

16. Verfahren zur Desikkation und/oder Defoliation von Pflanzen, dadurch gekennzeichnet, daß man eine desikkant oder defoliant wirksame Menge eines Tetrahydrophthalamidsäureesters der Formel VIIIa gemäß Anspruch 3 auf die Pflanzen einwirken läßt.

## Claims

1. A substituted cyclohexene-1,2-dicarboxylic acid derivative of the formula Ia or Ib 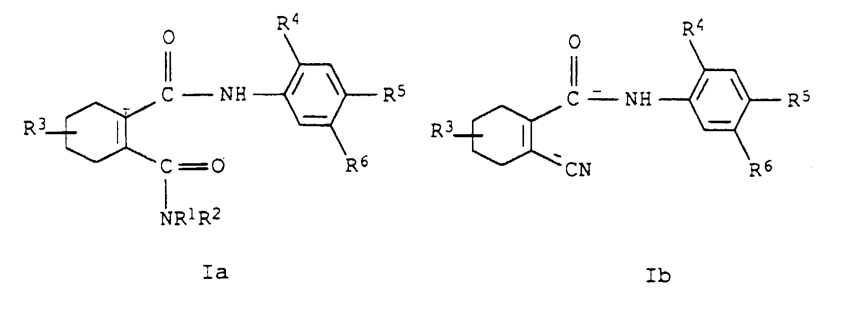 where
R¹ and R² are each
hydrogen,
C₁-C₆-alkyl, C₃-C₆-alkenyl or C₃-C₆-alkynyl, each of which may furthermore carry from one to three radicals selected from the group consisting of halogen, cyano, amino, thio, hydroxyl, C₁-C₆-alkoxy, C₁-C₆-alkylsulfinyl, C₁-C₆-alkylsulfonyl, C₁-C₆-alkylcarbonyl, carboxyl, C₁-C₆-alkoxycarbonyl, C₁-C₆-alkylamino, C₁-C₆-dialkylamino, C₁-C₆-alkylaminocarbonyl, C₁-C₆-dialkylaminocarbonyl, C₁-C₆-alkylphosphono, C₁-C₆-dialkylphosphono, phenyl, 3-membered to 8-membered heterocyclyl which may be saturated or partially or completely unsaturated, it being possible for the heterocycles to carry from one to four hetero atoms selected from the group consisting of from one to four nitrogen atoms, one oxygen atom and one sulfur atom, and it being possible for the phenyl and heterocyclic radicals in turn to carry one of the following substituents on each substitutable atom: hydroxyl, halogen, cyano, nitro, trifluoromethyl, C₁-C₆-alkyl or C₁-C₆-alkoxy, C₃-C₈-cycloalkyl,
phenyl or a saturated or partially or completely unsaturated 3-membered to 8-membered heterocyclyl group, each of which may carry from one to four hetero atoms selected from the group consisting of from one to four nitrogen atoms, one or two oxygen atoms and one or two sulfur atoms, it being possible for the phenyl and heterocyclic groups in turn to carry one of the following radicals on each substitutable carbon atom: hydroxyl, halogen, cyano, nitro, trifluoromethyl, halogen or C₁-C₆-alkyl,
and, if R¹ is hydrogen or C₁-C₆-alkyl, R² may additionally be
hydroxyl,
C₁-C₆-alkoxy, C₃-C₆-alkenyloxy or C₃-C₆-alkynyloxy,
C₃-C₇-cycloalkoxy or C₅-C₇-cycloalkenyloxy,
C₁-C₆-haloalkoxy or C₃-C₆-haloalkenyloxy,
C₃-C₇-cycloalkyl-C₁-C₆-alkoxy,
C₁-C₆-alkylcarbonyloxy,
C₁-C₆-cyanoalkoxy,
hydroxy-C₁-C₆-alkoxy, C₁-C₆-alkoxy-C₁-C₆-alkoxy or C₁-C₆-alkoxycarbonyl-C₁-C₆-alkoxy,
C₁-C₆-alkylthio-C₁-C₆-alkoxy,
C₁-C₆-alkylamino-C₁-C₆-alkoxy or C₁-C₆-dialkylamino-C₁-C₆-alkoxy,
phenyl-C₁-C₆-alkoxy, phenyl-C₃-C₆-alkenyloxy or phenyl-C₃-C₆-alkynyloxy, where in each case one or two methylene groups of the alkoxy, alkenyloxy or alkynyloxy chains may be replaced by oxygen, sulfur or a C₁-C₆-alkylamino chain, and each phenyl ring may be unsubstituted or may carry from one to three radicals selected from the group consisting of C₁-C₆-alkyl, C₂-C₆-alkenyl, halogen, cyano, nitro, C₁-C₆-haloalkyl, C₁-C₆-alkoxy and C₁-C₆-alkoxycarbonyl,
-N(R⁷)R⁸, where R⁷ and R⁸, independently of one another are each hydrogen, C₁-C₆-alkyl, C₃-C₆-alkenyl, C₃-C₆-alkynyl, C₃-C₆-cycloalkyl, C₁-C₆-haloalkyl, C₁-C₆-alkylcarbonyl, C₁-C₆-alkoxycarbonyl, C₁-C₆-alkoxy-C₁-C₆-alkyl or phenyl which may be unsubstituted or may carry from one to three radicals selected from the group consisting of C₁-C₆-alkyl, halogen, cyano, nitro, C₁-C₆-alkoxy and C₁-C₆-alkoxycarbonyl, or
R⁷ and R⁸ together with the common nitrogen atom may form a saturated or partially or completely unsaturated 4-membered to 7-membered ring which may also contain a further nitrogen atom or an oxygen or sulfur atom as a second ring member,
or
R¹ and R², together with the nitrogen atom to which they are bonded, may form
a three-membered to eight-membered saturated or unsaturated, nonaromatic heterocyclic structure which may carry from one to three nitrogen atoms or one oxygen or sulfur atom and one or two carbonyl groups, it being possible for the heterocyclic structure to be unsubstituted or to carry from one to three C₁-C₆-alkyl radicals;
R³ is hydrogen or C₁-C₆-alkyl;
R⁴ is hydrogen or halogen;
R⁵ is hydrogen, halogen, nitro, cyano or trifluoromethyl;
R⁶ is a group -A-CO-B, where A is a C₂-alkenylene chain, which may be unsubstituted or may carry one or two radicals selected from the group consisting of halogen, cyano, C₁-C₆-alkyl, C₁-C₆-haloalkyl, hydroxyl, C₁-C₆-alkoxy, C₁-C₆-alkylcarbonyloxy, C₁-C₆-alkoxycarbonyl and C₁-C₆-alkylcarbonyl,
B is
hydrogen, C₁-C₆-alkyl, C₃-C₆-alkenyl, C₃-C₆-alkynyl, C₁-C₆-haloalkyl or C₃-C₇-cycloalkyl,
-OR¹⁷ or -SR¹⁷, where R¹⁷ is
hydrogen, C₁-C₆-alkyl, C₃-C₆-alkenyl, C₃-C₆-alkynyl, C₃-C₆-cycloalkyl, C₁-C₆-haloalkyl,
phenyl which may be unsubstituted or may carry from one to three radicals selected from the group consisting of C₁-C₆-alkyl, C₂-C₆-alkenyl, halogen, cyano, nitro, C₁-C₆-haloalkyl, C₁-C₆-alkoxy and C₁-C₆-alkoxycarbonyl,
C₁-C₆-cyanoalkyl, C₃-C₆-haloalkenyl, C₁-C₆-alkoxycarbonyl, C₁-C₆-alkoxy-C₁-C₆-alkyl, C₁-C₆-alkoxycarbonyl-C₁-C₆-alkyl or C₁-C₆-alkoximino-C₁-C₆-alkyl;
phenyl which may be unsubstituted or may carry from one to three radicals selected from the group consisting of C₁-C₆-alkyl, C₂-C₆-alkenyl, halogen, cyano, nitro, C₁-C₆-haloalkyl, C₁-C₆-alkoxy and C₁-C₆-alkoxycarbonyl, C₁-C₆-alkoxy-C₁-C₆-alkyl, di-(C₁-C₆-alkoxy)-C₁-C₆-alkyl or C₁-C₆-alkylthio-C₁-C₆-alkyl, or
-N(R¹⁸)R¹⁹, where R¹⁸ and R¹⁹, independently of one another, are each hydrogen, C₁-C₆-alkyl, C₃-C₆-alkenyl, C₃-C₆-alkynyl, C₃-C₆-cycloalkyl, C₁-C₆-haloalkyl, C₁-C₆-alkylcarbonyl, C₁-C₆-alkoxycarbonyl, C₁-C₆-alkoxy-C₁-C₆-alkyl or phenyl which may be unsubstituted or may carry from one to three radicals selected from the group consisting of C₁-C₆-alkyl, C₃-C₆-alkenyl, halogen, cyano, nitro, C₁-C₆-alkoxy and C₁-C₆-alkoxycarbonyl, or
R¹⁸ and R¹⁹, together with the common nitrogen atom, form a saturated or partially or completely unsaturated 4-membered to 7-membered ring which may also contain a further nitrogen atom or an oxygen or sulfur atom as a second ring member, and the agriculturally useful salts of the compounds Ia and Ib.

2. A 3,4,5,6-tetrahydroisophthalimide of the formula IIIa 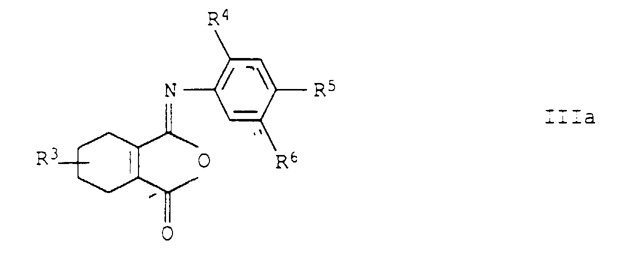 where the substituents R³ to R⁶ have the meanings given in claim 1.

3. A tetrahydrophthalamic ester of the formula VIIIa 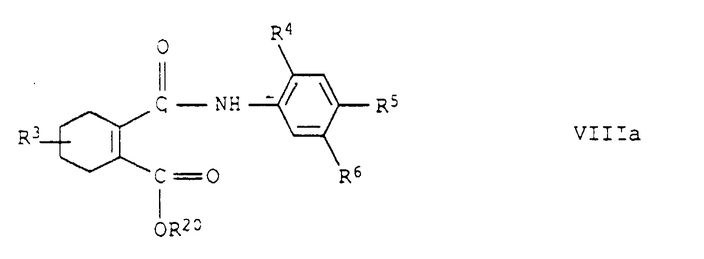 where the substituents R³ to R⁶ have the meanings given in claim 1 and
R²⁰ is hydrogen, C₁-C₆-alkyl, C₃-C₆-alkenyl or phenyl which may be unsubstituted or may carry from one to three radicals selected from the group consisting of halogen, cyano, nitro, C₁-C₄-alkyl, C₁-C₄-haloalkyl and C₁-C₆-alkoxy.

4. A process for the preparation of a substituted cyclohexene-1,2-dicarboxylic acid derivative Ia as claimed in claim 1, wherein a substituted 3,4,5,6-tetrahydrophthalimide of the formula II 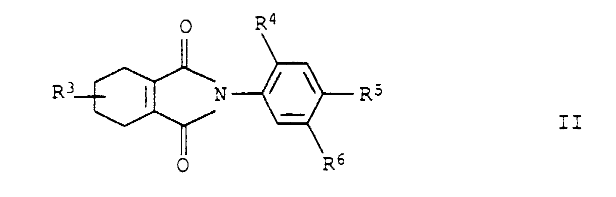 or a 3,4,5,6-tetrahydroisophthalimide of the formula III 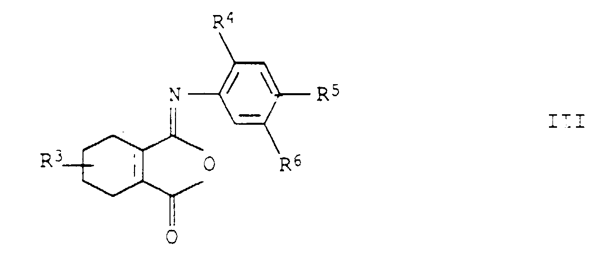 is reacted with an amine, hydroxylamine or hydrazine IV
HNR¹R² IV.

5. A process for the preparation of a substituted cyclohexene-1,2-dicarboxylic acid derivative Ia as claimed in claim 1, where R¹ is hydrogen, wherein a substituted aniline of the formula V 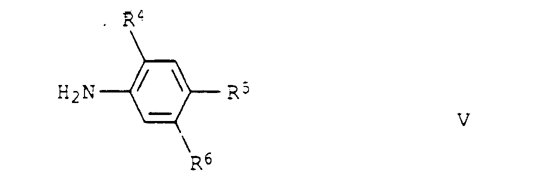 is reacted with a tetrahydrophthalimide of the formula VI 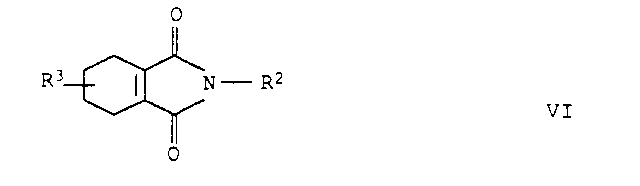 or with a tetrahydroisophthalimide of the formula VII

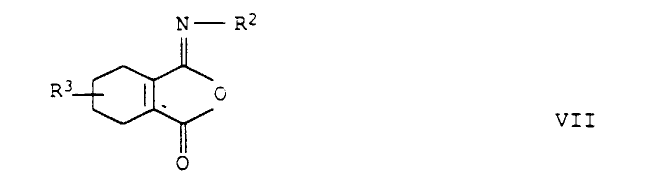

6. A process for the preparation of a cyclohexene-1,2-dicarboxylic acid derivative of the formula Ib as claimed in claim 1, wherein a cyclohexene-1,2-dicarboxylic acid derivative of the formula Ia, where R¹ and R² are each hydrogen, is reacted with a dehydrating agent.

7. A herbicide containing an inert liquid or solid carrier and a herbicidal amount of at least one substituted cyclohexene-1,2-dicarboxylic acid derivative of the formula Ia or Ib or an agriculturally useful salt of Ia or Ib as claimed in claim 1.

8. A method for controlling undesirable plant growth, wherein a herbicidal amount of at least one substituted cyclohexene-1,2-dicarboxylic derivative of the formula Ia or Ib or an agriculturally useful salt of Ia or Ib as claimed in claim 1 is allowed to act on plants or their habitat or on seed.

9. A desiccant or defoliant containing, in addition to conventional additives, an amount, having a dessicant or defoliant action, of at least one substituted cyclohexene-1,2-dicarboxylic acid derivative of the formula Ia or Ib or an agriculturally useful salt of Ia or Ib as claimed in claim 1.

10. A method for the desiccation or defoliation of plants, wherein an amount, having a desiccant or defoliant action, of a substituted cyclohexene-1,2-dicarboxylic acid derivative of the formula Ia or Ib or an agriculturally useful salt of Ia or Ib as claimed in claim 1 is allowed to act on the plants.

11. A method for controlling undesirable plant growth, wherein a herbicidal amount of at least one 3,4,5,6-tetrahydroisophthalimide of the formula IIIa as claimed in claim 2 is allowed to act on plants or their habitat or on seed.

12. A desiccant or defoliant containing, in addition to conventional additives, an amount, having a desiccant or defoliant action, of at least one 3,4,5,6-tetrahydroisophthalimide of the formula IIIa as claimed in claim 2.

13. A method for the desiccation or defoliation of plants, wherein an amount, having a desiccant or defoliant action, of a 3,4,5,6-tetrahydroisopthalimide of the formula IIIa as claimed in claim 2 is allowed to act on the plants.

14. A method for controlling undesirable plant growth, wherein a herbicidal amount of at least one tetrahydrophthalamic ester of the formula VIIIa as claimed in claim 3 is allowed to act on plants or their habitat or on seed.

15. A desiccant or defoliant containing, in addition to conventional additives, an amount, having a desiccant or defoliant action, of at least one tetrahydrophthalamic ester of the formula VIIIa as claimed in claim 3.

16. A method for the desiccation or defoliation of plants, wherein an amount, having a desiccant or defoliant action, of a tetrahydrophthalamic ester of the formula VIIIa as claimed in claim 3 is allowed to act on the plants.

## Revendications

1. Dérivés substitués de l'acide cyclohexène-1,2-dicarboxylique de formules générales Ia et Ib 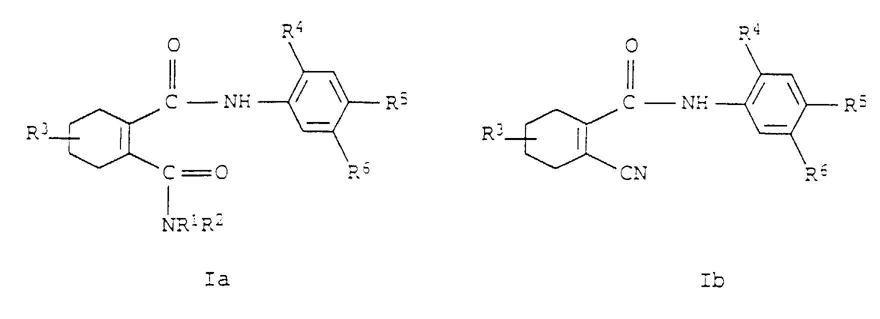 dans laquelle les symboles ont les significations suivantes :
R¹, R²
- l'hydrogène,
- un groupe alkyle en C1-C6, alcényle en C3-C6 ou alcynyle en C3-C6, chacun des trois groupes pouvant encore porter un à trois substituants choisis parmi les halogènes, les groupes cyano, amino, thio, hydroxy, alcoxy en C1-C6, alkylsulfinyle en C1-C6, alkylsulfonyle en C1-C6, alkylcarbonyle en C1-C6, carboxy, (alcoxy en C1-C6)carbonyle, alkylamino en C1-C6, di-(alkyle en C1-C6)amino, alkylamino en (C1-C6)carbonyle, di-(alkyle en C1-C6)aminocarbonyle, alkylphosphono en C1-C6, di-(alkyle en C1-C6)phosphono, phényle, hétérocyclyle de trois à huit chaînons qui peut être saturé ou partiellement ou totalement insaturé, les hétérocycles pouvant porter un à quatre hétaroatomes choisis dans un groupe consistant en un à quatre atomes d'azote, un atome d'oxygène et un atome de soufre, les radicaux phényle et les radicaux hétérocycliques pouvant eux-mêmes porter sur chaque atome substitable l'un des substituants suivants : hydroxy, halogéno, cyano, nitro, trifluorométhyle, alkyle en C1-C6 ou alcoxy en C1-C6,
- un groupe cycloalkyle en C3-C8,
- le groupe phényle ou un groupe hétérocyclyle de trois à huit chaînons, saturé ou partiellement ou totalement insaturé, qui peut porter un à quatre hétéroatomes choisis dans un groupe consistant en un à quatre atomes d'azote, un ou deux atomes d'oxygène et un ou deux atomes de soufre, le groupe phényle et les groupes hétérocycliques pouvant eux-mêmes porter sur chaque atome de carbone substituable l'un des substituants suivants : hydroxy, halogéno, cyano, nitro, trifluorométhyle, halogéno ou alkyle en C1-C6,
et, lorsque R¹ représente l'hydrogène ou un groupe alkyle en C1-C6,
R² peut en outre représenter :
- un groupe hydroxy,
- un groupe alcoxy en C1-C6, alcényloxy en C3-C6 ou alcynyloxy en C3-C6,
- un groupe cycloalcoxy en C3-C7 ou cycloalcényloxy en C5-C7,
- un groupe halogénoalcoxy en C1-C6 ou halogénoalcényloxy en C3-C6,
- un groupe (cycloalkyle en C3-C7)alcoxy en C1-C6,
- un groupe (alkyle en C1-C6)carbonyloxy,
- un groupe cyanoalcoxy en C1-C6,
- un groupe hydroxy-alcoxy en C1-C6, (alcoxy en C1-C6)alcoxy en C1-C6 ou (alcoxy en C1-C6)carbonyl-alcoxy en C1-C6,
- un groupe (alkylthio en C1-C6)alcoxy en C1-C6,
- un groupe (alkylamino en C1-C6)alcoxy en C1-C6 ou di-(alkyle en C1-C6)amino-alcoxy en C1-C6,
- un groupe phényl-alcoxy en C1-C6, phényl-alcényloxy en C3-C6 ou phényl-alcynyloxy en C3-C6, dans lesquels un ou deux groupes méthylène des chaînes alcoxy, alcényloxy et alcynyloxy peuvent être remplacées par l'oxygène, le soufre et/ou une chaîne alkylamino en C1-C6, le cycle phényle pouvant dans tous les cas être non substitué ou porter un à trois substituants choisis parmi les groupes alkyle en C1-C6, alcényle en C2-C6, les halogènes, les groupes cyano, nitro, halogénoalkyle en C1-C6, alcoxy en C1-C6 et (alcoxy en C1-C6)carbonyle,
- un groupe -N(R⁷)R⁸ dans lequel R⁷ et R⁸ représentent chacun, indépendamment l'un de l'autre, l'hydrogène, un groupe alkyle en C1-C6, alcényle en C3-C6, alcynyle en C3-C6, cycloalkyle en C3-C6, halogénoalkyle en C1-C6, (alkyle en C1-C6)carbonyle, (alcoxy en C1-C6)carbonyle, (alcoxy en C1-C6)alkyle en C1-C6 ou phényle, lequel est non substitué ou porte un à trois substituants choisis parmi les groupes alkyle en C1-C6, les halogènes, les groupes cyano, nitro, alcoxy en C1-C6 et (alcoxy en C1-C6)carbonyle,
ou bien
R⁷ et R⁸ forment ensemble et avec l'atome d'azote commun un cycle de quatre à sept chaînons, saturé ou partiellement ou totalement insaturé, qui peut encore contenir un autre atome d'azote ou un atome d'oxygène ou de soufre en tant que deuxième chaînon cyclique ;
ou bien
R¹ et R² forment ensemble, et avec l'atome d'azote auquel ils sont reliés, un hétérocycle non aromatique de trois à huit chaînons, saturé ou insaturé, qui peut contenir un à trois atomes d'azote et/ou un atome d'oxygène ou de soufre et un ou deux groupes carbonyle,
l'hétérocycle étant non substitué ou portant un à trois groupes alkyle en C1-C6 ;
R³ représente l'hydrogène ou un groupe alkyle en C1-C6 ;
R⁴ représente l'hydrogène ou un halogène ;
R⁵ représente l'hydrogène, un halogène, un groupe nitro, cyano ou trifluorométhyle ;
R⁶ représente un groupe -A-CO-B dans lequel
A représente
une chaîne alcénylène en C2 non substituée ou portant un ou deux substituants choisis parmi les halogènes, les groupes cyano, alkyle en C1-C6, halogénoalkyle en C1-C6, hydroxy, alcoxy en C1-C6, (alkyle en C1-C6)carbonyloxy, (alcoxy en C1-C6)carbonyle et (alkyle en C1-C6)carbonyle,
B représente
- l'hydrogène, un groupe alkyle en C1-C6, alcényle en C3-C6, alcynyle en C3-C6, halogénoalkyle en C1-C6, cycloalkyle en C3-C7,
- un groupe -OR¹⁷ ou SR¹⁷ dans lequel R¹⁷ représente
- l'hydrogène, un groupe alkyle en C1-C6, alcényle en C3-C6, alcynyle en C3-C6, cycloalkyle en C3-C6, halogénoalkyle en C1-C6,
- un groupe phényle non substitué ou portant un à trois substituants choisis parmi les groupes alkyle en C1-C6, alcényle en C2-C6, les halogènes, les groupes cyano, nitro, halogénoalkyle en C1-C6, alcoxy en C1-C6 et (alcoxy en C1-C6)carbonyle,
- un groupe cyanoalkyle en C1-C6, halogénoalcényle en C3-C6, (alcoxy en C1-C6)carbonyle, (alcoxy en C1-C6)alkyle en C1-C6, (alcoxy en C1-C6)carbonyl-alkyle en C1-C6 ou (alkyle en C1-C6)oximino-alkyle en C1-C6 ;
- un groupe phényle non substitué ou portant un à trois substituants choisis parmi les groupes alkyle en C1-C6, alcényle en C2-C6, les halogènes, les groupes cyano, nitro, halogénoalkyle en C1-C6, alcoxy en C1-C6 et (alcoxy en C1-C6)carbonyle,
- un groupe (alcoxy en C1-C6)alkyle en C1-C6, di-(alcoxy en C1-C6)alkyle en C1-C6, (alkylthio en C1-C6)alkyle en C1-C6, ou bien
- un groupe -N(R¹⁸)R¹⁹ dans lequel R¹⁸ et R¹⁹ représentent chacun, indépendamment l'un de l'autre,
l'hydrogène, un groupe alkyle en C1-C6, alcényle en C3-C6, alcynyle en C3-C6, cycloalkyle en C3-C6, halogénoalkyle en C1-C6, (alkyle en C1-C6)carbonyle, (alcoxy en C1-C6)carbonyle, (alcoxy en C1-C6)alkyle en C1-C6 ou phényle, lequel est non substitué ou porte un à trois substituants choisis parmi les groupes alkyle en C1-C6, alcényle en C3-C6, les halogènes, les groupes cyano, nitro, alcoxy en C1-C6 et (alcoxy en C1-C6)carbonyle,
ou bien
R¹⁸ et R¹⁹ forment ensemble et avec l'atome commun un cycle de quatre à sept chaînons, saturé ou partiellement ou totalement insaturé, qui peut encore contenir un autre atome d'azote ou un atome d'oxygène ou de soufre en tant que deuxième chaînon cyclique,
ainsi que les sels des composés la et Ib convenant pour les applications agricoles.

2. 3,4,5,6-tétrahydrophtalisoimides de formule générale IIIa 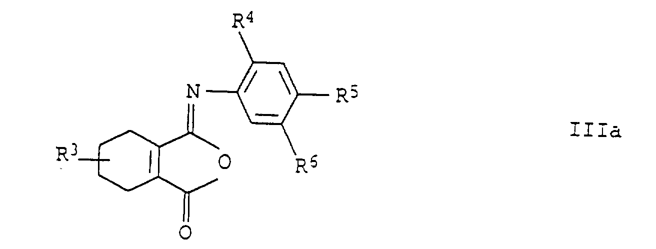 dans laquelle les symboles R³ à R⁶ ont le significations indiquées dans la revendication 1.

3. Esters tétrahydrophtalamidiques de formule générale VIIIa 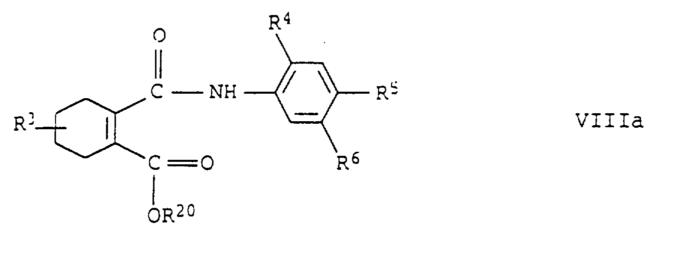 dans laquelle les symboles R³ à R⁶ ont les significations indiquées dans la revendication 1 et
R²⁰ représente l'hydrogène, un groupe alkyle en C1-C6, alcényle en C3-C6 ou un groupe phényle non substitué ou portant un à trois substituants choisis parmi les halogènes, les groupes cyano, nitro, alkyle en C1-C4, halogénoalkyle en C1-C4 et alcoxy en C1-C6.

4. Procédé de préparation des dérivés substitués de l'acide cyclohexène-1,2-dicarboxylique Ia de la revendication 1, caractérisé par le fait que l'on fait réagir un 3,4,5,6-tétrahydrophtalimide substitué de formule II 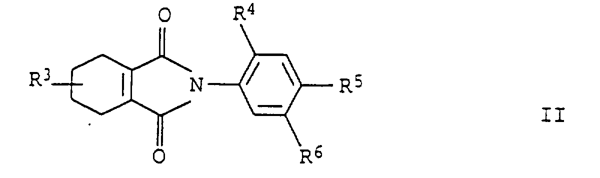 ou un 3,4,5,6-tétrahydrophtalisoimide de formule III 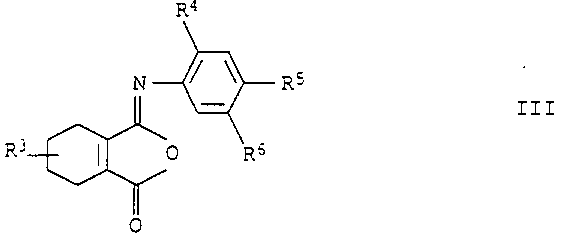 avec une amine, une hydroxylamine ou une hydrazine IV
HNR¹R² IV

5. Procédé de préparation des dérivés substitués de l'acide cyclohexène-1,2-dicarboxylique Ia de la revendication dans laquelle R¹ représente l'hydrogène, caractérisé par le fait que l'on fait réagir une aniline substituée de formule V 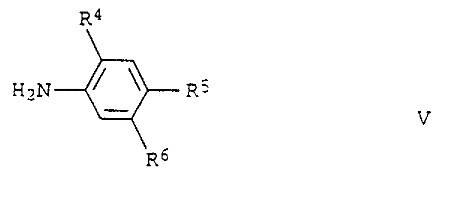 avec un tétrahydrophtalimide de formule VI 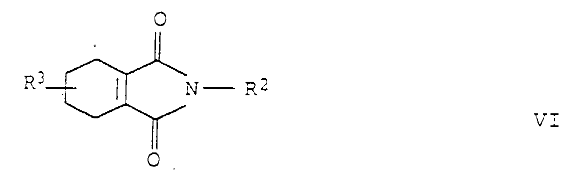 ou avec un tétrahydrophtalisoimide de formule VII

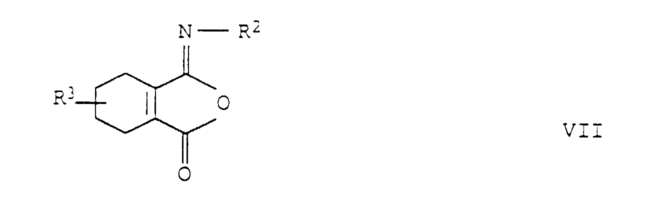

6. Procédé de préparation des dérivés substitués de l'acide cyclohexène-1,2-dicarboxylique de formule Ib de la revendication 1, caractérisé par le fait que l'on fait réagir les dérivés de l'acide cyclohexène-1,2-dicarboxylique de formule Ia dans laquelle R¹ et R² représentent l'hydrogène avec un agent déshydratant.

7. Produit herbicide contenant un véhicule solide ou liquide inerte et une quantité herbicide efficace d'au moins un dérivé substitué de l'acide cyclohexène-1,2-dicarboxylique de formule Ia ou Ib ou d'un sel acceptable pour les usages de Ia ou Ib selon la revendication 1.

8. Procédé pour combattre les croissances de végétaux indésirables, caractérisé par le fait que l'on fait agir sur les végétaux, leur habitat ou les semences, une quantité herbicide efficace d'au moins un dérivé substitué de l'acide cyclohexène-1,2-dicarboxylique de formule Ia ou Ib ou d'un sel acceptable pour les usages agricoles de Ia ou Ib selon la revendication 1.

9. Produit pour la dessiccation et/ou défoliation de végétaux, contenant, avec des additifs usuels, une quantité dessiccante ou défoliante efficace d'au moins un dérivé substitué de l'acide cyclohexène-1,2-dicarboxylique de formule Ia ou Ib ou d'un sel acceptable pour les usages agricoles Ia ou Ib selon la revendication 1.

10. Procédé pour la dessiccation et/ou la défoliation de végétaux, caractérisé par le fait que l'on fait agir sur les végétaux une quantité dessiccante ou défoliante efficace d'un dérivé substitué de l'acide cyclohexène-1,2-dicarboxylique de formule Ia ou Ib ou d'un sel acceptable pour les usages agricoles de Ia ou Ib selon la revendication 1.

11. Procédé pour combattre les croissances de végétaux indésirables, caractérisé par le fait que l'on fait agir sur les végétaux, leur habitat ou les semences, une quantité herbicide efficace d'au moins un 3,4,5,6-tétrahydrophtalisoimide de formule IIIa de la revendication 2.

12. Produit pour la dessiccation et/ou la défoliation de végétaux, contenant, avec des additifs usuels, une quantité dessiccante ou défoliante efficace d'au moins un 3,4,5,6-tétrahydrophtalisoimide de formule IIIa de la revendication 2.

13. Procédé pour la dessiccation et/ou la défoliation de végétaux, caractérisé par le fait que l'on fait agir sur les végétaux une quantité dessiccante ou défoliante efficace d'un 3,4,5,6-tétrahydrophtalisoimide de formule IIIa de la revendication 2.

14. Procédé pour combattre les croissances de végétaux indésirables, caractérisé par le fait que l'on fait agir sur les végétaux, leur habitat ou les semences, une quantité herbicide efficace d'au moins un ester tétrahydrophtalamidique de formule VIIIa de la revendication 3.

15. Produit pour la dessiccation et/ou la défoliation des végétaux contenant, avec des additifs usuels, une quantité dessiccante ou défoliante efficace d'au moins un ester tétrahydrophtalamidique de formule VIIIa de la revendication 3.

16. Procédé pour la dessiccation et/ou la défoliation de végétaux, caractérisé par le fait que l'on fait agir sur les végétaux une quantité dessiccante ou défoliante efficace d'un ester tétrahydrophtalamidique de formule VIIIa de la revendication 3.
